# EUROPEAN PATENT APPLICATION

(11) **EP 4 834 907 A1**
(43) Date of publication of application: **04.11.2026**
(21) Application number: 25751634.4
(22) Date of filing: 07.02.2025
(51) Int. Cl.: C07D 487/04, C07D 519/00, A61P 17/06, A61P 17/00, A61K 31/519, A61K 31/542, A61P 37/06

(54) **HETEROCYCLIC COMPOUND, AND PHARMACEUTICAL COMPOSITION THEREOF AND USE THEREOF**

(30) Priority: 07.02.2024 CN 202410176290
(71) Applicant: Shanghai Equvie Bioscience Co., Ltd., Shanghai 201306 (CN)
(72) Inventor: WEI, Changqing, Shanghai 201306 (CN); ZHANG, Wei, Shanghai 201306 (CN)
(74) Representative: Chung, Hoi Kan
(86) International application number: PCT/CN2025/076132
(87) International publication number: WO 2025/168035

(57) **Abstract**

Disclosed in the present invention are a heterocyclic compound, and a pharmaceutical composition thereof and the use thereof. Specifically, disclosed in the present invention are a compound as shown in formula (I), and a pharmaceutically acceptable salt thereof, a solvate thereof, or a solvate of the pharmaceutically acceptable salt thereof. The compound of the present invention has an excellent inhibitory activity on TYK2, has, in particular, an excellent inhibitory activity on TYK2-E957D and/or TYK2 JH2, and can significantly inhibit the proliferation of cells carrying TYK2-E957D mutations.

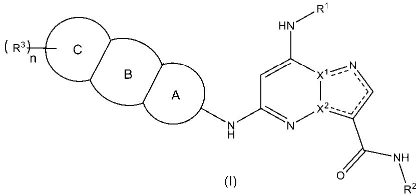

## Description

This application claims priority to Chinese Patent Application No. 202410176290.9, filed on February 7, 2024. The entire disclosure of the above-mentioned Chinese patent application is incorporated herein by reference.

### TECHNICAL FIELD OF THE INVENTION

The present invention relates to heterocyclic compound and pharmaceutical composition thereof and use thereof.

### BACKGROUND OF THE INVENTION

Tyrosine kinase 2 (TYK2) is a non-receptor tyrosine kinase member of the Janus kinase (JAK) protein family. JAK proteins, including TYK2, are essential components of cytokine signal transduction. TYK2 associates with the cytoplasmic domains of type I and type II cytokine receptors as well as type I and type III interferon receptors, and is activated by these receptors upon cytokine binding. Cytokines involved in TYK2 activation include interferons and interleukins. Upon cytokine activation, the TYK2 signaling pathway promotes the phosphorylation and activation of signal transducer and activator of transcription (STAT) proteins, which then translocate into the nucleus to participate in the production of specific proteins. This pathway is involved in the functions of TH17 cells, TH1 cells, B cells, and myeloid cells; these cells play key roles in the pathology of autoimmune diseases such as psoriasis, psoriatic arthritis, inflammatory bowel disease, lupus erythematosus, and atopic dermatitis.

Meanwhile, TYK2 has been demonstrated to play an important role in maintaining tumor surveillance. TYK2 knockout mice exhibit impaired cytotoxic T cell responses and accelerated tumor progression. Studies on T-cell acute lymphoblastic leukemia (T-ALL) have shown that T-ALL is highly dependent on IL-10 via TYK2, and maintains cancer cell survival through STAT-mediated signal transduction by upregulating the anti-apoptotic protein BCL2. Knockdown of TYK2 (rather than other JAK family members) reduces cell growth. TYK2-specific activating mutations that promote cancer cell survival include mutations in the FERM domain (G36D, S47N, and R425H), the JH2 domain (V73 11), and the kinase domain (E957D and R1027H). However, it has also been found that the kinase function of TYK2 is necessary for enhancing cancer cell survival, because the TYK2 enzyme, when carrying kinase-dead mutations (M978Y or M978F) in addition to an activating mutation (E957D), fails to transform.

Several TYK2 inhibitors including deucravacitinib and zasocitinib have been reported in the prior art, yet the variety remains relatively limited. Given that TYK2 plays a critical role in the pathological progression of numerous diseases, there is still a need to provide additional novel compounds with TYK2 inhibitory activity to meet the needs of disease treatment.

### SUMMARY OF THE INVENTION

The technical problem to be solved by the present invention is to address the deficiency of TYK2 inhibitor types in the prior art, and to provide a heterocyclic compound with a novel structure, a pharmaceutical composition thereof, and uses thereof. The compounds of the present invention exhibit excellent TYK2 inhibitory activity; in particular, they possess potent inhibitory activity against TYK2-E957D and/or TYK2-JH2, and can significantly inhibit the proliferation of cells carrying the TYK2-E957D mutation.

The present invention solves the above-mentioned technical problems through the following technical solutions.

The present invention provides a compound represented by Formula (I), a pharmaceutically acceptable salt thereof, a solvate thereof, or a solvate of the pharmaceutically acceptable salt thereof:

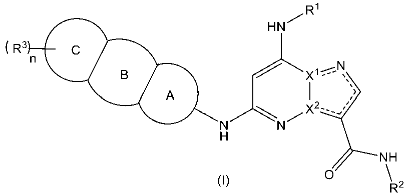

Wherein, 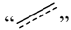 represents a single bond or a double bond;
X¹ is C or N;
X² is C or N;
R¹ is H, C₁-C₆ alkyl, or C₁-C₆ alkyl substituted with one or more deuterium atoms;
R² is C₁-C₆ alkyl, C₁-C₆ alkoxy, C₃-C₁₂ cycloalkyl, 3-12 membered heterocycloalkyl, C₁-C₆ alkyl substituted with one or more R²⁻¹, C₁-C₆ alkoxy substituted with one or more R²⁻², C₃-C₁₂ cycloalkyl substituted with one or more R²⁻³, or 3-12 membered heterocycloalkyl substituted with one or more R²⁻⁴;
R²⁻¹ and R²⁻² are each independently halogen, OH, C₃-C₁₂ cycloalkyl, or 3-12 membered heterocycloalkyl;

Or, two adjacent R²⁻¹ groups, together with the carbon atom to which they are attached, together form a C₃-C₇ monocyclic cycloalkyl, a C₅-C₁₂ fused cycloalkyl, a C₅-C₁₂ bridged cycloalkyl, a C₅-C₁₂ spiro cycloalkyl, a 3-7 membered monocyclic heterocycloalkyl, a 5-12 membered fused heterocycloalkyl, a 5-12 membered bridged heterocycloalkyl, or a 5-12 membered spiro heterocycloalkyl;

Or, two R²⁻¹ groups attached to the same carbon atom, together with the carbon atom to which they are attached, together form a C₃-C₇ monocyclic cycloalkyl, a C₅-C₁₂ fused cycloalkyl, a C₅-C₁₂ bridged cycloalkyl, a C₅-C₁₂ spiro cycloalkyl, a 3-7 membered monocyclic heterocycloalkyl, a 5-12 membered fused heterocycloalkyl, a 5-12 membered bridged heterocycloalkyl, or a 5-12 membered spiro heterocycloalkyl;

Or, two adjacent R²⁻² groups, together with the carbon atom to which they are attached, together form a C₃-C₇ monocyclic cycloalkyl, a C₅-C₁₂ fused cycloalkyl, a C₅-C₁₂ bridged cycloalkyl, a C₅-C₁₂ spiro cycloalkyl, a 3-7 membered monocyclic heterocycloalkyl, a 5-12 membered fused heterocycloalkyl, a 5-12 membered bridged heterocycloalkyl, or a 5-12 membered spiro heterocycloalkyl;

Or, two R²⁻² groups attached to the same carbon atom, together with the carbon atom to which they are attached, together form a C₃-C₇ monocyclic cycloalkyl, a C₅-C₁₂ fused cycloalkyl, a C₅-C₁₂ bridged cycloalkyl, a C₅-C₁₂ spiro cycloalkyl, a 3-7 membered monocyclic heterocycloalkyl, a 5-12 membered fused heterocycloalkyl, a 5-12 membered bridged heterocycloalkyl, or a 5-12 membered spiro heterocycloalkyl;
R²⁻³ and R²⁻⁴ are each independently halogen, OH, C₁-C₆ alkyl, or C₁-C₆ alkoxy;
Or, two adjacent R²⁻³ groups, together with the carbon atom to which they are attached, together form a C₃-C₇ cycloalkyl or a 3-7 membered heterocycloalkyl;
Or, two R²⁻³ groups attached to the same carbon atom, together with the carbon atom to which they are attached, together form a C₃-C₇ cycloalkyl or a 3-7 membered heterocycloalkyl;
Or, two adjacent R²⁻⁴ groups, together with the carbon atom to which they are attached, together form a C₃-C₇ cycloalkyl or a 3-7 membered heterocycloalkyl;
Or, two R²⁻⁵ groups attached to the same carbon atom, together with the carbon atom to which they are attached, together form a C₃-C₇ cycloalkyl or a 3-7 membered heterocycloalkyl;
Ring A, Ring B and Ring C are each independently a benzene ring, a 5- to 6-membered heteroaryl, a C₅-C₆ cycloalkene or a 5- to 6-membered heterocycloalkene;
Ring A and Ring B share a bond, and said bond is a single bond or a double bond;
Ring B and Ring C share a bond, and said bond is a single bond or a double bond;
R³ is a substituent on Ring A, Ring B or Ring C;
Each R³ is independently halogen, CN, C₁-C₆ alkyl, C₁-C₆ alkoxy, C₃-C₁₂ cycloalkyl, 3-12 membered heterocycloalkyl, C₁-C₆ alkyl substituted with one or more R³⁻¹, or C₁-C₆ alkoxy substituted with one or more R³⁻²;
R³⁻¹ and R³⁻² are each independently halogen;
n is 0, 1, 2 or 3;

In the "3-12 membered heterocycloalkyl", "3-12 membered heterocycloalkyl substituted with one or more R²⁻⁴", "5-6 membered heterocycloalkene", "3-8 membered heterocycloalkyl", "3-7 membered monocyclic heterocycloalkyl", "5-12 membered fused heterocycloalkyl", "5-12 membered bridged heterocycloalkyl", "5-12 membered spiro heterocycloalkyl" and "3-7 membered heterocycloalkyl", the types of heteroatoms or heteroatomic groups are each independently selected from one or more of N, O, S, C(=O), S(=O) and S(=O)₂, and the number of heteroatoms or heteroatomic groups is each independently one or more;

In the "5-6 membered heteroaryl", the types of heteroatoms are each independently selected from one or more of N, O and S, and the number of heteroatoms is each independently one or more.

In certain preferred embodiments of the present invention, certain groups of the compound represented by Formula (I), a pharmaceutically acceptable salt thereof, a solvate thereof, or a solvate of the pharmaceutically acceptable salt thereof are defined as follows, and groups not mentioned are the same as those defined in any embodiment of the present invention (referred to as "in one embodiment of the present invention" for short).

In one embodiment of the present invention, in the "3-12 membered heterocycloalkyl", "3-12 membered heterocycloalkyl substituted with one or more R²⁻⁴", "5-6 membered heterocycloalkene", "3-8 membered heterocycloalkyl", "3-7 membered monocyclic heterocycloalkyl", "5-12 membered fused heterocycloalkyl", "5-12 membered bridged heterocycloalkyl", "5-12 membered spiro heterocycloalkyl" and "3-7 membered heterocycloalkyl", the types of heteroatoms or heteroatomic groups are each independently selected from 1, 2 or 3 of N, O, S, C(=O), S(=O) and S(=O)₂, and the number of heteroatoms or heteroatomic groups is each independently 1, 2 or 3.

In one embodiment of the present invention, in the "5-6 membered heteroaryl", the types of heteroatoms are each independently selected from 1, 2 or 3 of N, O and S, and the number of heteroatoms is each independently 1, 2 or 3.

In one embodiment of the present invention, each "C₁-C₆ alkyl" is independently methyl, ethyl, n-propyl, isopropyl, n-butyl, isobutyl, sec-butyl, tert-butyl, n-pentyl, -CH₂CH₂CH₂CH₂CH₃, -CH(CH₃)CH₂CH₂CH₃, - CH₂CH(CH₃)CH₂CH₃, -CH₂CH₂CH(CH₃)₂, -CH(C₂H₅)CH₂CH₃, -C(CH₃)₂CH₂CH₃, -CH(CH₃)CH(CH₃)₂ or - CH₂C(CH₃)₃, such as methyl, isopropyl, isobutyl or -CH₂C(CH₃)₃.

In one embodiment of the present invention, each "C₁-C₆ alkoxy" is independently methoxy, ethoxy, n-propoxy, isopropoxy, n-butoxy, isobutoxy, sec-butoxy or tert-butoxy, such as methoxy.

In one embodiment of the present invention, each "C₃-C₁₂ cycloalkyl" is independently a C₃-C₆ cycloalkyl, such as cyclopropyl, cyclobutyl, cyclopentyl, cyclohexyl, spiro[2.2]pentyl or spiro[2.3]hexyl, further such as

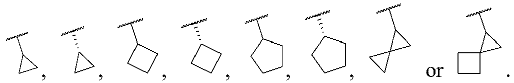

In one embodiment of the present invention, each "3-12 membered heterocycloalkyl" is independently a 3-6 membered heterocycloalkyl in which the heteroatoms are selected from one or more of N, O and S and the number of heteroatoms is 1 or 2, such as aziridinyl, oxiranyl, azetidinyl, oxetanyl, tetrahydrofuranyl, pyrrolidinyl, morpholinyl or piperidinyl, further such as

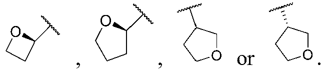

In one embodiment of the present invention, each "halogen" is independently F, Cl, Br or I, such as F.

In one embodiment of the present invention, each "5-6 membered heteroaryl" is a 5-6 membered heteroaryl in which the heteroatoms are selected from one or more of N, O and S and the number of heteroatoms is 1 or 2, such as furan ring, thiophene ring, pyrrole ring, pyrazole ring, imidazole ring, pyridine ring, pyrazine ring, pyridazine ring or pyrimidine ring.

In one embodiment of the present invention, each "5-6 membered heterocycloalkene" is 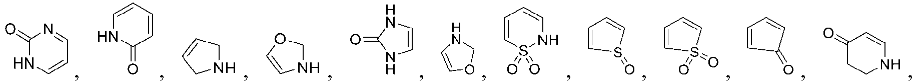 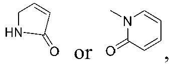 for example

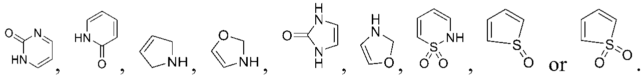

In one embodiment of the present invention, 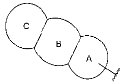 is 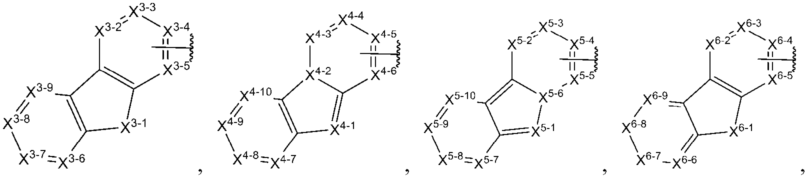 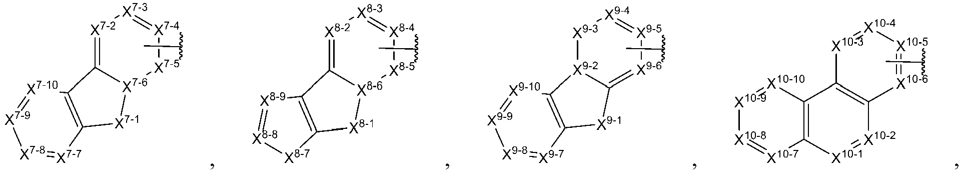 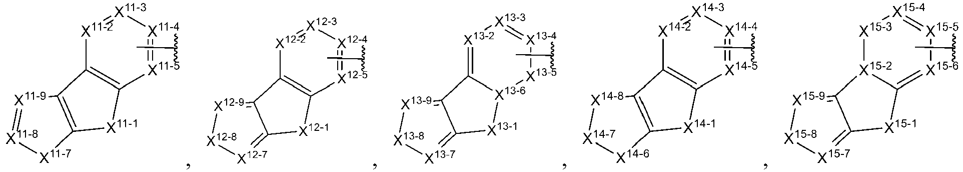 or 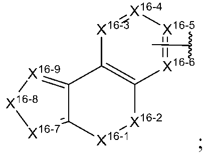 wherein,
X³⁻¹ is CH₂, NH, O, S, C(=O), S(=O) or S(=O)₂; X³⁻², X³⁻³, X³⁻⁴, X³⁻⁵, X³⁻⁶, X³⁻⁷, X³⁻⁸ and X³⁻⁹ are each independently CH or N;
X⁴⁻¹, X⁴⁻², X⁴⁻³, X⁴⁻⁴, X⁴⁻⁵, X⁴⁻⁶, X⁴⁻⁷, X⁴⁻⁸, X⁴⁻⁹ and X⁴⁻¹⁰ are each independently CH or N;
X⁵⁻¹, X⁵⁻², X⁵⁻³, X⁵⁻⁴, X⁵⁻⁵, X⁵⁻⁶, X⁵⁻⁷, X⁵⁻⁸, X⁵⁻⁹ and X⁵⁻¹⁰ are each independently CH or N;
X⁶⁻¹, X⁶⁻⁷ and X⁶⁻⁸ are each independently CH₂, NH, O, S, C(=O), S(=O) or S(=O)₂, X⁶⁻², X⁶⁻³, X⁶⁻⁴, X⁶⁻⁵, X⁶⁻⁶ and X⁶⁻⁹ are each independently CH or N;
X⁷⁻¹ 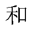 X⁷⁻⁵ are each independently CH₂, NH, O, S, C(=O), S(=O) or S(=O)₂, X⁷⁻², X⁷⁻³, X⁷⁻⁴, X⁷⁻⁶, X⁷⁻⁷, X⁷⁻⁸, X⁷⁻⁹ and X⁷⁻¹⁰ are each independently CH or N;
X⁸⁻¹, X⁸⁻⁵ and X⁸⁻⁷ are each independently CH₂, NH, O, S, C(=O), S(=O) or S(=O)₂, X⁸⁻², X⁸⁻³, X⁸⁻⁴, X⁸⁻⁶, X⁸⁻⁸ and X⁸⁻⁹ are each independently CH or N;
X⁹⁻¹ and X⁹⁻³ are each independently CH₂, NH, O, S, C(=O), S(=O) or S(=O)₂, X⁹⁻², X⁹⁻⁴, X⁹⁻⁵, X⁹⁻⁶, X⁹⁻⁷, X⁹⁻⁸, X⁹⁻⁹ and X⁹⁻¹⁰ are each independently CH or N;
X¹⁰⁻¹, X¹⁰⁻², X¹⁰⁻³, X¹⁰⁻⁴, X¹⁰⁻⁵, X¹⁰⁻⁶, X¹⁰⁻⁷, X¹⁰⁻⁸, X¹⁰⁻⁹ and X¹⁰⁻¹⁰ are each independently CH or N;
X¹¹⁻¹ and X¹¹⁻⁷ are each independently CH₂, NH, O, S, C(=O), S(=O) or S(=O)₂, X¹¹⁻², X¹¹⁻³, X¹¹⁻⁴, X¹¹⁻⁵, X¹¹⁻⁸ and X¹¹⁻⁹ are each independently CH or N;
X¹²⁻¹ and X¹²⁻⁸ are each independently CH₂, NH, O, S, C(=O), S(=O) or S(=O)₂, X¹²⁻², X¹²⁻³, X¹²⁻⁴, X¹²⁻⁵, X¹²⁻⁷ and X¹²⁻⁹ are each independently CH or N;
X¹³⁻¹, X¹³⁻⁵ and X¹³⁻⁸ are each independently CH₂, NH, O, S, C(=O), S(=O) or S(=O)₂, X¹³⁻², X¹³⁻³, X¹³⁻⁴, X¹³⁻⁶, X¹³⁻⁷ and X¹³⁻⁹ are each independently CH or N;
X¹⁴⁻¹, X¹⁴⁻⁶, X¹⁴⁻⁷ and X¹⁴⁻⁸ are each independentlyCH₂, NH, O, S, C(=O), S(=O) or S(=O)₂, X¹⁴⁻², X¹⁴⁻³, X¹⁴⁻⁴ and X¹⁴⁻⁵ are each independently CH or N;
X¹⁵⁻¹, X¹⁵⁻³ and X¹⁵⁻⁸ are each independently CH₂, NH, O, S, C(=O), S(=O) or S(=O)₂, X¹⁵⁻², X¹⁵⁻⁴, X¹⁵⁻⁵, X¹⁵⁻⁶, X¹⁵⁻⁷ and X¹⁵⁻⁹ are each independently CH or N;
X¹⁶⁻¹, X¹⁶⁻² and X¹⁶⁻⁸ are each independently CH₂, NH, O, S, C(=O), S(=O) or S(=O)₂, X¹⁶⁻³, X¹⁶⁻⁴, X¹⁶⁻⁵, X¹⁶⁻⁶, X¹⁶⁻⁷ and X¹⁶⁻⁹ are each independently CH or N.

In one embodiment of the present invention, 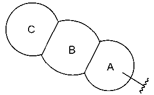 is

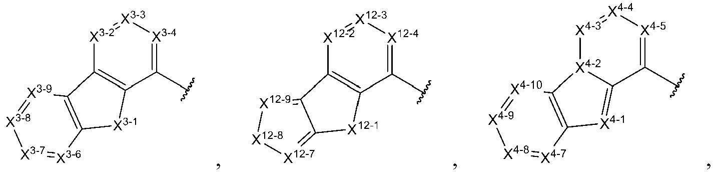

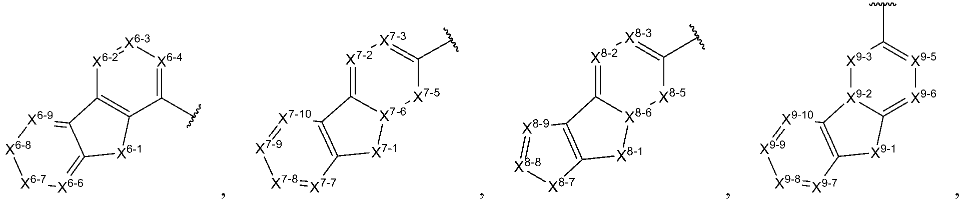

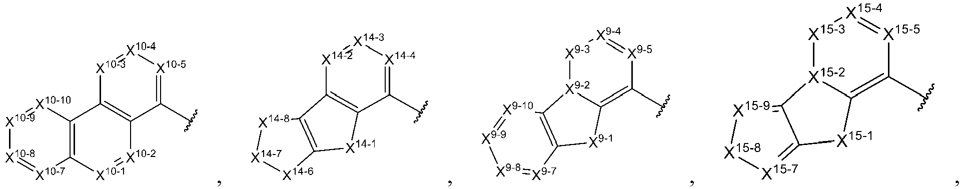

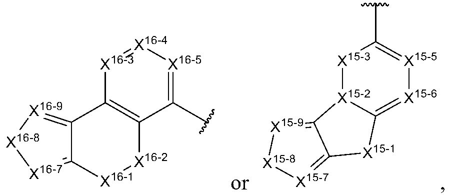

Wherein,
X³⁻¹ is CH₂, NH, O, S, C(=O), S(=O) or S(=O)₂, X³⁻², X³⁻³, X³⁻⁴, X³⁻⁶, X³⁻⁷, X³⁻⁸ and X³⁻⁹ are each independently CH or N;
X⁴⁻¹, X⁴⁻², X⁴⁻³, X⁴⁻⁴, X⁴⁻⁵, X⁴⁻⁷, X⁴⁻⁸, X⁴⁻⁹ and X⁴⁻¹⁰ are each independently CH or N;
X⁶⁻¹, X⁶⁻⁷ and X⁶⁻⁸ are each independently CH₂, NH, O, S, C(=O), S(=O) or S(=O)₂, X⁶⁻², X⁶⁻³, X⁶⁻⁴, X⁶⁻⁶ and X⁶⁻⁹ are each independently CH or N;
X⁷⁻¹ and X⁷⁻⁵ are each independently CH₂, NH, O, S, C(=O), S(=O) or S(=O)₂, X⁷⁻², X⁷⁻³, X⁷⁻⁶, X⁷⁻⁷, X⁷⁻⁸, X⁷⁻⁹ and X⁷⁻¹⁰ are each independently CH or N;
X⁸⁻¹, X⁸⁻⁵ and X⁸⁻⁷ are each independently CH₂, NH, O, S, C(=O), S(=O) or S(=O)₂, X⁸⁻², X⁸⁻³, X⁸⁻⁶, X⁸⁻⁸ and X⁸⁻⁹ are each independently CH or N;
X⁹⁻¹ and X⁹⁻³ are each independently CH₂, NH, O, S, C(=O), S(=O) or S(=O)₂, X⁹⁻², X⁹⁻⁴, X⁹⁻⁵, X⁹⁻⁶, X⁹⁻⁷, X⁹⁻⁸, X⁹⁻⁹ and X⁹⁻¹⁰ are each independently CH or N;
X¹⁰⁻¹, X¹⁰⁻², X¹⁰⁻³, X¹⁰⁻⁴, X¹⁰⁻⁵, X¹⁰⁻⁷, X¹⁰⁻⁸, X¹⁰⁻⁹ and X¹⁰⁻¹⁰ are each independently CH or N;
X¹²⁻¹ and X¹²⁻⁸ are each independently CH₂, NH, O, S, C(=O), S(=O) or S(=O)₂, X¹²⁻², X¹²⁻³, X¹²⁻⁴, X¹²⁻⁷ and X¹²⁻⁹ are each independently CH or N;
X¹⁴⁻¹, X¹⁴⁻⁶, X¹⁴⁻⁷ and X¹⁴⁻⁸ are each independentlyCH₂, NH, O, S, C(=O), S(=O) or S(=O)₂, X¹⁴⁻², X¹⁴⁻³ and X¹⁴⁻⁴ are each independently CH or N;
X¹⁵⁻¹, X¹⁵⁻³ and X¹⁵⁻⁸ are each independently CH₂, NH, O, S, C(=O), S(=O) or S(=O)₂, X¹⁵⁻², X¹⁵⁻⁴, X¹⁵⁻⁵, X¹⁵⁻⁶, X¹⁵⁻⁷ and X¹⁵⁻⁹ are each independently CH or N;
X¹⁶⁻¹, X¹⁶⁻² and X¹⁶⁻⁸ are each independently CH₂, NH, O, S, C(=O), S(=O) or S(=O)₂, X¹⁶⁻³, X¹⁶⁻⁴, X¹⁶⁻⁵, X¹⁶⁻⁷ and X¹⁶⁻⁹ are each independently CH or N.

In one embodiment of the present invention, 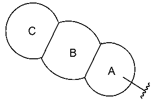 is any one of the following cases:
Case1: is 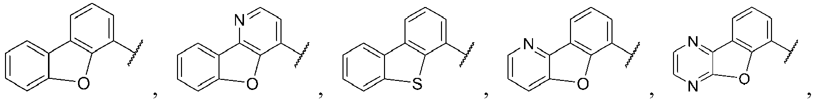 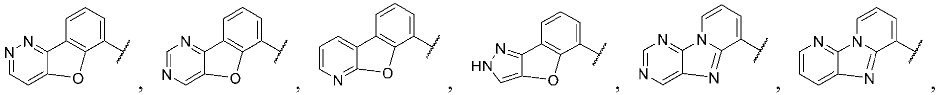 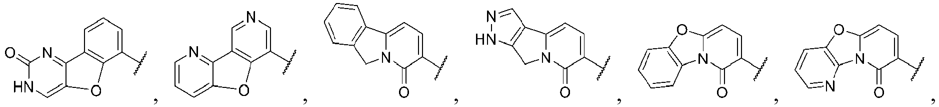 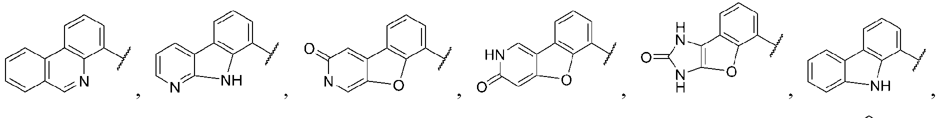 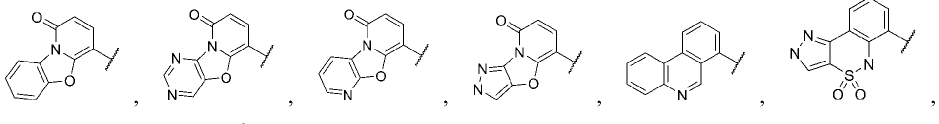 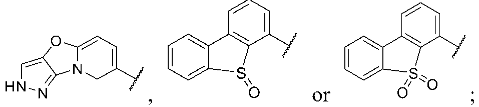
Case 2: is 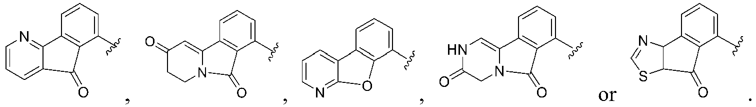 In one embodiment of the present invention, each R³ is independently F, CN, C₁-C₃ alkyl, C₁-C₃ alkoxy, C₃-C₆ cycloalkyl, or C₁-C₃ alkyl substituted with one or more F atoms, such as F, CN, CH₃, CF₃, OCH₃ or

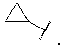

In one embodiment of the present invention, n is 0, 1 or 2.

In one embodiment of the present invention, 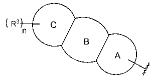 is any one of the following cases:
Case 1: is 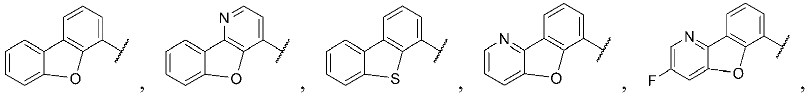 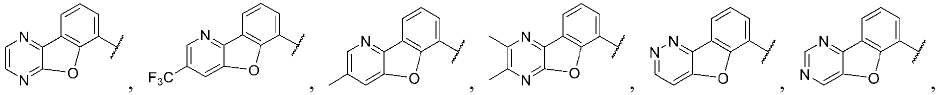 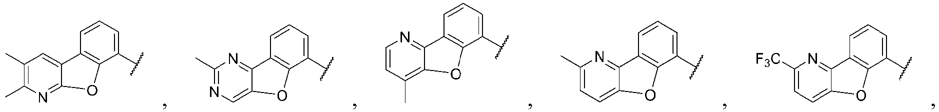 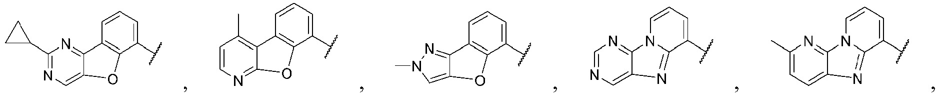 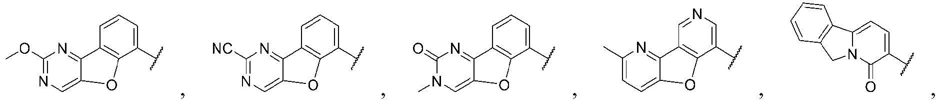 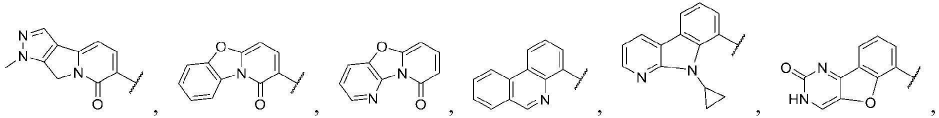 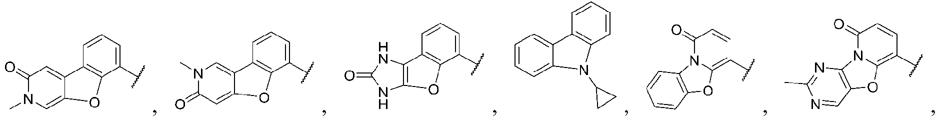 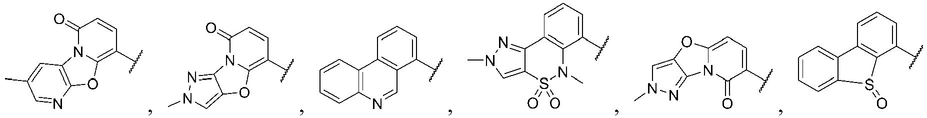 or 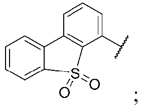
Case 2: is

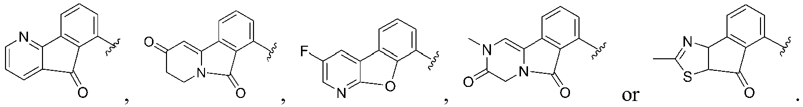

In one embodiment of the present invention, R¹ is C₁-C₃ alkyl, such as methyl.

In one embodiment of the present invention, R² is C₁-C₅ alkyl, C₃-C₆ cycloalkyl, 3-6 membered heterocycloalkyl, C₁-C₅ alkyl substituted with one or more R²⁻¹, C₃-C₆ cycloalkyl substituted with one or more R²⁻³, or 3-6 membered heterocycloalkyl substituted with one or more R²⁻⁴;

R²⁻¹ is independently OH or 3-6 membered heterocycloalkyl;

R²⁻³ is independently F, OH or OCH₃;

R²⁻⁴ is independently OH or OCH₃.

In one embodiment of the present invention, R² is

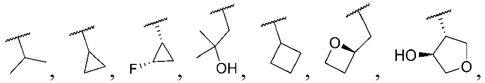

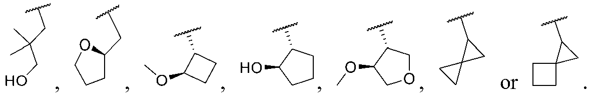

In one embodiment of the present invention, the compound represented by Formula (I) is a compound represented by Formula (I-1) or (I-2):

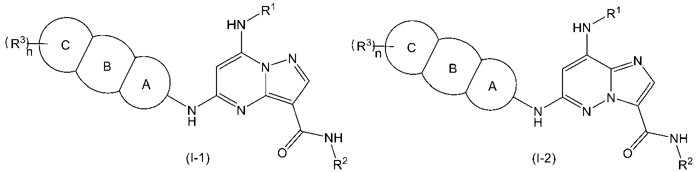

The definitions of R¹, R², R³, n, Ring A, Ring B and Ring C are the same as those defined in any embodiment of the present invention.

In one embodiment of the present invention, the compound represented by Formula (I) is a compound represented by Formula (I-3) or (I-4):

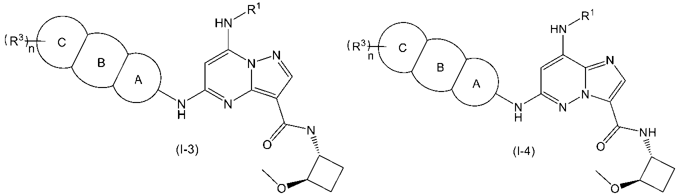

The definitions of R¹, R³, n, Ring A, Ring B and Ring C are the same as those defined in any embodiment of the present invention.

In one embodiment of the present invention, the compound represented by Formula (I) is any one of the following compounds:

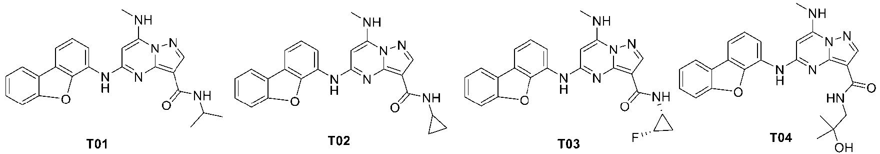

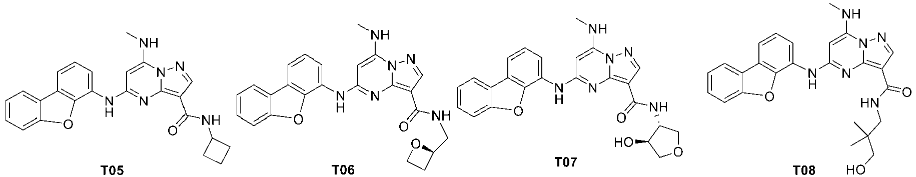

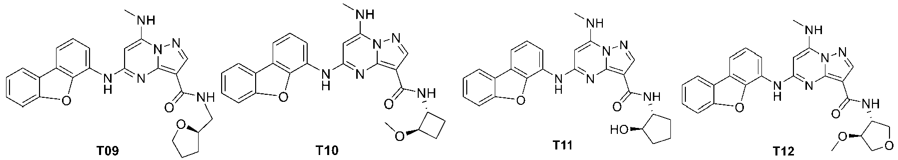

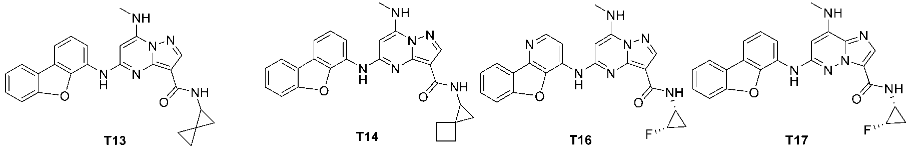

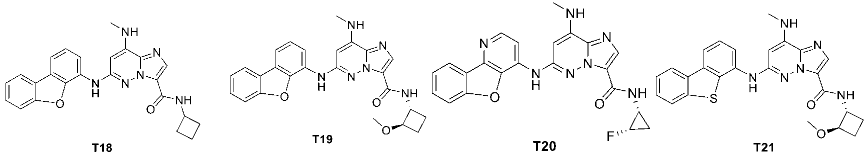

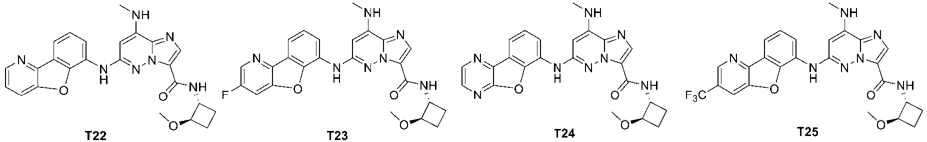

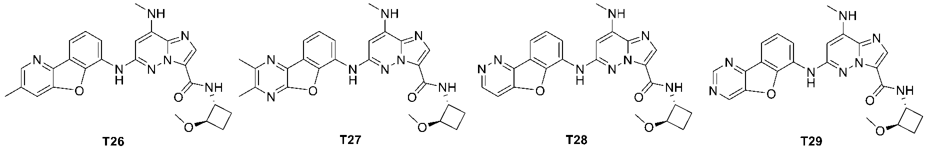

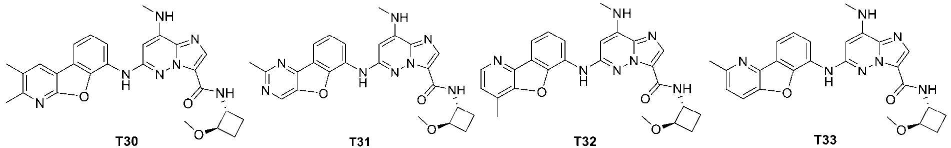

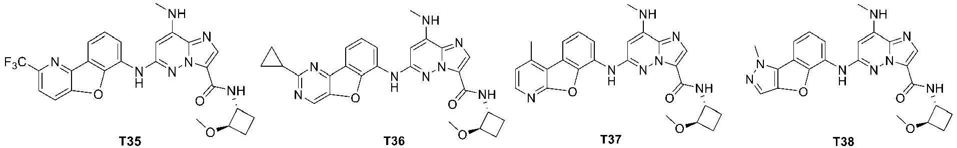

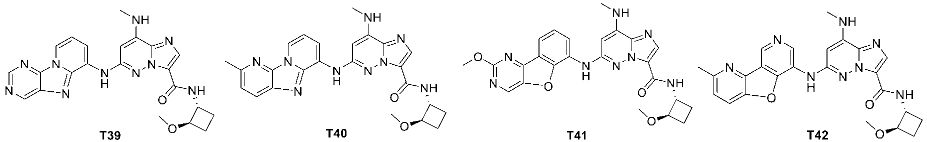

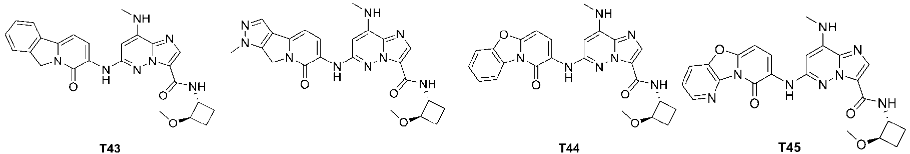

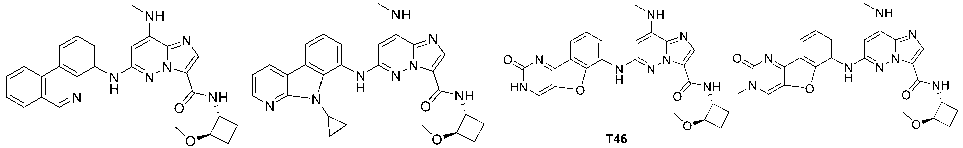

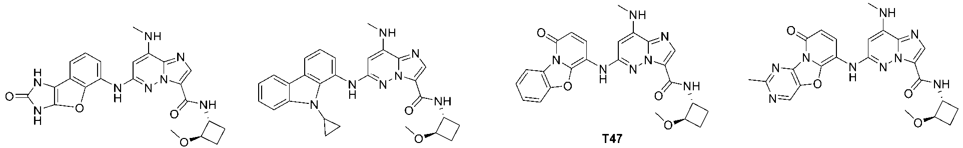

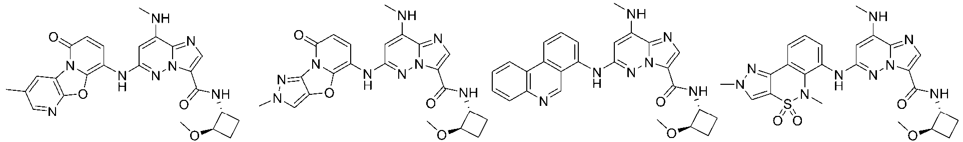

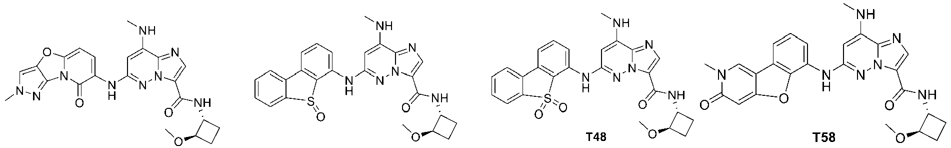

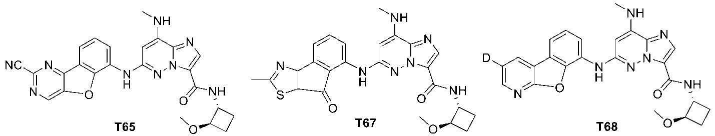

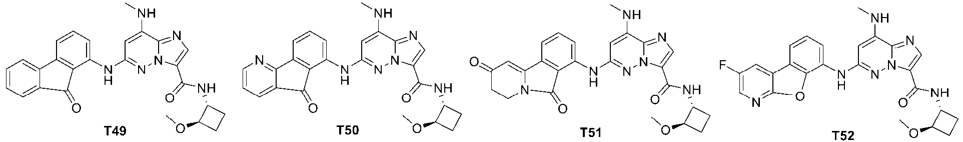

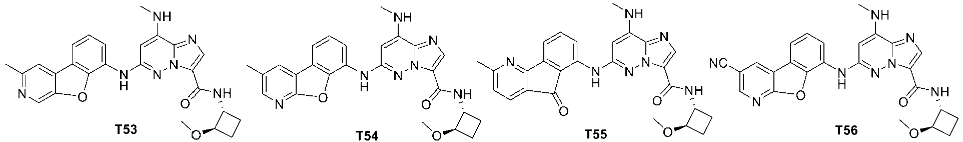

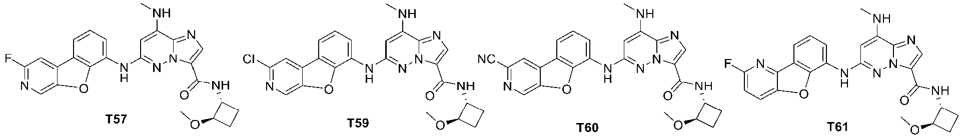

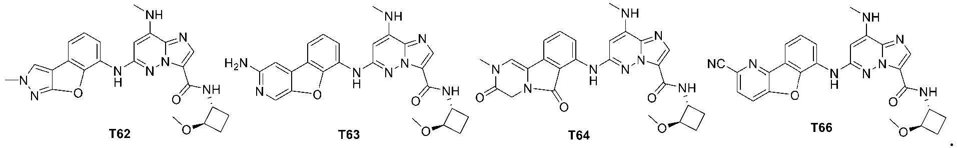

The present invention further provides a pharmaceutical composition, which comprises the compound represented by Formula (I), a pharmaceutically acceptable salt thereof, a solvate thereof, or a solvate of the pharmaceutically acceptable salt thereof according to any one of the above embodiments, together with pharmaceutically acceptable excipients.

The present invention further provides use of the compound represented by Formula (I), a pharmaceutically acceptable salt thereof, a solvate thereof, or a solvate of the pharmaceutically acceptable salt thereof according to any one of the above embodiments, or the aforementioned pharmaceutical composition, in the preparation of a TYK2 inhibitor; preferably, the TYK2 is TYK2-E957D.

The present invention further provides use of the compound represented by Formula (I), a pharmaceutically acceptable salt thereof, a solvate thereof, or a solvate of the pharmaceutically acceptable salt thereof according to any one of the above embodiments, or the aforementioned pharmaceutical composition, in the manufacture of a medicament for preventing and/or treating TYK2-related diseases; preferably, the TYK2-related diseases are diseases associated with TYK2-E957D and/or TYK2 JH2.

In one embodiment of the present invention, the TYK2-related diseases are autoimmune diseases, such as psoriasis, psoriatic arthritis, inflammatory bowel disease, lupus erythematosus or allergic dermatitis.

The present invention further provides use of the compound represented by Formula (I), a pharmaceutically acceptable salt thereof, a solvate thereof, or a solvate of the pharmaceutically acceptable salt thereof according to any one of the above embodiments, or the aforementioned pharmaceutical composition, in the manufacture of a medicament for preventing and/or treating autoimmune diseases, wherein the autoimmune diseases include psoriasis, psoriatic arthritis, inflammatory bowel disease, lupus erythematosus or allergic dermatitis.

Unless otherwise stated, each group in the present invention may be interpreted as follows.

Those skilled technicians in the field may understand that, in accordance with the conventions adopted in the field, the bond line 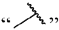 drawn in the structural formula for describing a group in the present invention indicates that the corresponding group is linked to other fragments or groups in the compound via this site.

In the present invention, the structural skeleton 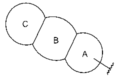 only represents that Ring A and Ring B form a fused ring, and Ring B and Ring C form a fused ring. Ring A and Ring B share a bond designated as bond i, which may be a single bond or a double bond; Ring B and Ring C share a bond designated as bond ii, which may be a single bond or a double bond. The relative positions of bond i and bond ii are not limited by the structural skeleton

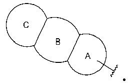

When any variable (e.g., R³) appears more than once in the composition or structure of a compound, its definition is independent for each occurrence. For example, if a group is substituted with 0 to 3 R³ groups, the group may be optionally substituted with up to three R³ groups, and each R³ independently has its own selection scope. Furthermore, combinations of substituents and/or variants thereof are permitted only when such combinations can form stable compounds.

In the present invention, each recitation of "C₁-C₆ alkyl" refers not only to a standalone C₁-C₆ alkyl group but also to the alkyl moiety within a substituted C₁-C₆ alkyl group; similarly, each recitation of "3-12 membered heterocycloalkyl" covers both an independent 3-12 membered heterocycloalkyl group and the heterocycloalkyl fragment in a substituted 3-12 membered heterocycloalkyl group. All other expressions introduced by "each" shall be construed in the same way.

The term "alkyl" means a saturated linear or branched monovalent hydrocarbon group containing a specified number of carbon atoms. For instance, C₁-C₆ alkyl denotes an alkyl group having 1 to 6 carbon atoms. Examples of alkyl groups include, but are not limited to, methyl (Me), ethyl (Et), propyl (e.g., n-propyl, isopropyl), butyl (e.g., n-butyl, isobutyl, sec-butyl, tert-butyl) and pentyl (e.g., n-pentyl, isopentyl, neopentyl).

The term "alkoxy" refers to the group -O-Rₓ, wherein Rₓ is an alkyl group as defined above.

The term "cycloalkyl" means a saturated monocyclic, fused, bridged or spiro cyclic group with a specified number of ring carbon atoms (e.g., C₃-C₁₂), wherein all ring atoms are carbon atoms.

The term "heterocycloalkyl" refers to a cyclic group with a specified number of ring atoms (e.g., 3-12 membered), a specified number of heteroatoms (e.g., 1, 2 or 3), and heteroatom types selected from 1, 2 or 3 of N, O, S, C(=O), S(=O) and S(=O)₂. Such groups are monocyclic, bridged or spiro, and every ring thereof is saturated.

The term "halogen" refers to F, Cl, Br and I.

The term "heteroaryl" means a cyclic unsaturated aromatic ring with a specified number of ring atoms (e.g., 5-6 membered), a specified number of heteroatoms (e.g., 1, 2 or 3), and heteroatom types selected from 1, 2 or 3 of N, O and S. A heteroaryl is attached to the rest of the molecule via a carbon atom or a heteroatom.

The term "cycloalkene" means a partially unsaturated cyclic ring with a specified number of carbon atoms (e.g., C₅-C₆), which contains one or more (e.g., 1 or 2) carbon-carbon sp² double bonds and is non-aromatic.

The term "heterocycloalkene" means a partially unsaturated cyclic ring with a specified number of ring atoms (e.g., 5-6 membered), a specified number of heteroatoms (e.g., 1, 2 or 3), and heteroatom types selected from 1, 2 or 3 of N, O, S, C(=O), S(=O) and S(=O)₂. It contains one or more (e.g., 1 or 2) carbon-carbon sp² double bonds and is non-aromatic.

The term "pharmaceutically acceptable salt" refers to the salts of the compounds of the present invention, which are prepared from the herein-disclosed compounds bearing specific substituents and relatively non-toxic acids or bases. When a compound of the present invention bears a relatively acidic functional group, base addition salts can be obtained by contacting the neutral form of such compound with a sufficient amount of a base in neat solution or a suitable inert solvent. When a compound of the present invention bears a relatively basic functional group, acid addition salts can be obtained by contacting the neutral form of such compound with a sufficient amount of an acid in neat solution or a suitable inert solvent.

The term "pharmaceutically acceptable excipients" refers to excipients and adjuvants used in pharmaceutical production and prescription compounding, covering all substances contained in a pharmaceutical formulation other than the active ingredient. Reference may be made to Volume IV of the Pharmacopoeia of the People's Republic of China (2020 Edition) or Handbook of Pharmaceutical Excipients (Raymond C Rowe, 6th Edition, 2009).

The term "solvate" refers to a substance formed by the association of a compound with a solvent. Solvates include stoichiometric solvates and non-stoichiometric solvates.

The term "solvate of a pharmaceutically acceptable salt" refers to a substance formed by combining a compound with a pharmaceutically acceptable acid or base and a solvent, wherein the amount of solvent may be stoichiometric or non-stoichiometric.

In the aforementioned uses, the TYK2 inhibitor can be applied in vivo in mammals; it may also be used in vitro mainly for experimental purposes, such as serving as a standard sample or control sample for comparison, or being formulated into a kit according to conventional methods in the field to enable rapid detection of TYK2 inhibitory activity.

All the above preferred conditions can be arbitrarily combined without conflicting with common general knowledge in the technical field to obtain various preferred embodiments of the present invention.

All reagents and raw materials adopted in the present invention are commercially available.

The beneficial technical effects of the present invention are as follows: the compounds of the present invention exhibit excellent inhibitory activity against TYK2, particularly potent inhibitory activity against TYK2-E957D and/or TYK2 JH2, and can significantly inhibit the proliferation of cells carrying the TYK2-E957D mutation.

### DETAILED DESCRIPTION OF THE PREFERRED EMBODIMENT

The present disclosure is described in detail by the embodiments below, but it does not mean that there are any adverse restrictions on the present disclosure. The present disclosure has been described in detail herein, and its specific embodiments have also been disclosed; for one skilled in the art, it is obvious to make various modifications and improvements to the embodiments of the present disclosure without departing from the spirit and scope of the present disclosure.

### Example T01

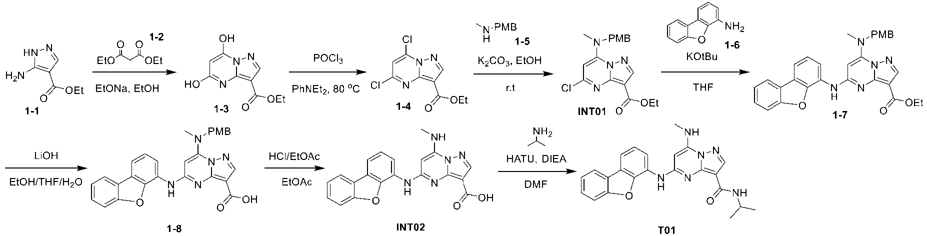

Step1: Dissolve **1-1** (50 g, 322 mmol) and diethyl malonate (103 g, 645 mmol) in 1000 mL of ethanol, and cool the mixture to 0 °C using an ice-water bath. Add sodium ethoxide (65.8 g, 0.97 mol) in batches, while keeping the system temperature below 10 °C. Heat the reaction mixture to 80 °C and stir overnight. After the reaction is complete, concentrate the reaction mixture to obtain a yellow solid suspended in 2 L of water. Adjust the pH to 4 using 6M HCl (77 g). Filter the mixture, wash the filter cake with 150 mL of water, and dry the filter cake at 50 °C overnight to obtain **1-3** (68 g, yield 94.6%). MS m/z: 224 [M+H]⁺.

Step2: Add **1-3** (10 g, 44.8 mmol) and N,N-diethylaniline (10 mL, 62.7 mmol) to a 250 mL three-neck flask, and slowly add POCl₃ (45 mL, 479.4 mmol) under an ice-water bath. After the addition is complete, heat the reaction system to 80 °C and stir for 3 hours. After the reaction is complete, allow the reaction system to cool to room temperature and slowly quench it by adding ice water. Adjust the pH to 6.4 with saturated NaHCO₃. Extract with EtOAc (200 mL × 3). Combine the organic phases, dry over Na₂SO₄, and then concentrate to obtain the crude product. Purify by column chromatography (PE:EtOAc = 10:1~5:1) to obtain product **1-4** (10 g, yield 86%). MS m/z: 260 [M+H]⁺.

Step3: Dissolve **1-3** (1.0 g, 3.84 mmol) in 20 mL of ethanol, transfer the solution to a 250 mL three-neck flask, add **1-5** (639 mg, 4.2 mmol) and K₂CO₃ (585 mg, 4.2 mmol). Stir the reaction system at room temperature overnight. Filter the reaction system, wash the filter cake with EtOAc, and concentrate the filtrate. Dissolve the obtained concentrate in EtOAc (50 mL) and wash with water (30 mL). Wash the organic phase with 10% citric acid, saturated NaHCO₃ solution, and saturated saline solution, respectively. Dry the organic phase over Na₂SO₄ and then evaporate to dryness to obtain the crude product. Purify the product with prep-TLC (PE:EtOAc = 5:1) to obtain **INT01** (1.2 g, yield 83%). MS m/z: 375 [M+H]⁺.

Step4: **INT01** (1.19 g, 3.2 mmol) was dissolved in 15 mL of THF, followed by the addition of 1-6 (611 mg, 3.3 mmol) and potassium tert-butoxide (713 mg, 6.4 mmol) at 0 °C. The reaction mixture was stirred at room temperature overnight. After the reaction was complete, the mixture was slowly added to a 10% aqueous solution of citric acid, and extracted with EtOAc (20 mL*3). The organic layers were combined, dried over Na₂SO₄, and then evaporated to dryness to obtain the crude product. Column chromatography (PE:EtOAc = 2:1) to obtain **1-7** (1 g, yield 60%). MS m/z: 522 [M+H]⁺. ¹H NMR (400 MHz, CDCl₃) δ 8.55 (d, *J* = 8.0 Hz, 1H), 8.39 (s, 1H), 7.96 (dt, *J* = 7.6, 1.0 Hz, 1H), 7.66 (dd, *J* = 7.8, 1.1 Hz, 1H), 7.59 - 7.52 (m, 1H), 7.51 - 7.42 (m, 1H), 7.47 - 7.30 (m, 3H), 7.16 - 7.09 (m, 2H), 6.86 - 6.79 (m, 2H), 5.66 (s, 1H), 5.08 (s, 2H), 4.45 (q, *J* = 7.1 Hz, 2H), 3.77 (s, 3H), 2.99 (s, 3H), 1.51 (t, *J* = 7.1 Hz, 3H).

Step5: Dissolve **1-7** (611.8 mg, 1.17 mmol) in 10 mL of THF, 10 mL of ethanol, and 5 mL of water, and add LiOH (281 mg, 11.7 mmol). Stir the reaction system at room temperature overnight. Concentrate the reaction system under reduced pressure, suspend the resulting concentrate in 20 mL of water, and adjust the pH to 4-5 with 0.5 N HCl. Filter, transfer the filter cake to a one-neck flask with EtOAc, dry and concentrate to obtain product **1-8** (542 mg, yield 94%), which is directly used for the next reaction step. MS m/z: 494 [M+H]⁺.

Step6: **1-8** (542 mg, 1.1 mmol) was suspended in 5 mL of EtOAc, followed by the addition of 5 mL of a 4 M HCl solution in EtOAc. The reaction mixture was stirred at room temperature overnight. The mixture was concentrated under reduced pressure to yield crude product **INT02** (411 mg, yield 100%). This crude product was directly used for the subsequent reaction step. MS m/z: 374 [M+H]⁺.

Step7: **INT02** (20 mg, 53.57 µmol) was dissolved in 3 mL of DMF, followed by the addition of HATU (40.74 mg, 107.13 µmol). The mixture was stirred at room temperature for 15 minutes, after which DIEA (34.62 mg, 267.83 µmol) and isopropylamine (6.33 mg, 107.13 µmol) were introduced. The reaction mixture was stirred overnight at room temperature. The mixture was partitioned into EtOAc, washed with 0.5 M HCl and water, respectively. The organic layer was dried over Na₂SO₄ and then evaporated to dryness to yield a crude product. Prep-TLC (PE:EtOAc = 2:1) to obtain **T01** (18 mg, yield 82%). MS m/z: 415 [M+H]⁺. ¹H NMR (400 MHz, DMSO-d6) δ 9.66 (s, 1H), 8.17 (d, *J* = 7.4 Hz, 1H), 8.11 (s, 1H), 7.97 - 7.87 (m, 2H), 7.84 (dd, *J* = 7.8, 1.1 Hz, 1H), 7.71 (d, *J* = 8.2 Hz, 1H), 7.58 - 7.36 (m, 4H), 5.82 (s, 1H), 3.93 - 3.75 (m, 1H), 2.94 (d, *J* = 4.8 Hz, 3H), 0.75 (d, *J* = 6.6 Hz, 6H).

The synthesis methods for **T02-T14** are similar to that of **T01,** involving the condensation of **INT02** with different amines or amine salts under the same conditions. The identification data for **T02-T14** are shown in Table 1.

**Table 1. Identification data for T02-T14**

| Example | Structure | MS & NMR |
|---|---|---|
| **T02** | 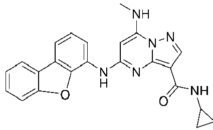 | MS m/z: 413 [M+H]⁺. ¹H NMR (400 MHz, DMSO-d6) δ 9.69 (s, 1H), 8.23 - 8.11 (m, 2H), 7.99 - 7.89 (m, 2H), 7.80 (d, *J* = 7.7 Hz, 1H), 7.74 - 7.66 (m, 2H), 7.58 - 7.49 (m, 1H), 7.43 (q, *J* = 7.4 Hz, 2H), 5.78 (d, *J* = 14.8 Hz, 1H), 2.94 (d, *J* = 4.8 Hz, 3H), 2.56 (dt, *J* = 7.5, 3.6 Hz, 1H), 0.50 - 0.40 (m, 2H), -0.06 (d, *J* = 5.0 Hz, 2H). |
| **T03** | 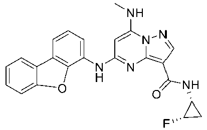 | MS m/z: 431 [M+H]⁺. ¹H NMR (400 MHz, DMSO-d6) δ 9.29 (s, 1H), 8.60 (d, *J* = 4.1 Hz, 1H), 8.18 (d, *J* = 7.2 Hz, 1H), 7.92 (dd, *J* = 7.7, 1.2 Hz, 1H), 7.85 (s, 1H), 7.70 (t, *J* = 7.4 Hz, 2H), 7.58 - 7.48 (m, 2H), 7.47 - 7.34 (m, 2H), 6.05 (s, 1H), 4.63-4.40 (m, 1H), 2.91 (d, *J* = 4.9 Hz, 3H), 2.05 - 1.95 (m, 1H), 0.84 (dq, *J* = 14.8, 8.2, 7.2 Hz, 1H), 0.35 - 0.22 (m, 1H). |
| **T04** | 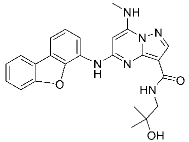 | MS m/z: 445 [M+H]⁺. ¹H NMR (400 MHz, DMSO-d6) δ 9.63 (s, 1H), 8.26 - 8.15 (m, 2H), 8.03 (d, *J* = 8.1 Hz, 1H), 7.94 - 7.80 (m, 3H), 7.71 (d, *J* = 8.3 Hz, 1H), 7.58 - 7.50 (m, 1H), 7.43 (t, *J* = 8.0 Hz, 2H), 5.90 (s, 1H), 4.41 (s, 1H), 3.08 (s, 2H), 2.97 (d, *J* = 4.8 Hz, 3H), 0.86 (s, 6H). |
| **T05** | 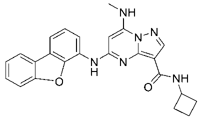 | MS m/z: 427 [M+H]⁺. ¹H NMR (400 MHz, DMSO-d6) δ 9.68 (s, 1H), 8.19 (dd, *J* = 7.6, 1.3 Hz, 1H), 8.09 (s, 1H), 7.99 (dd, *J* = 7.7, 1.1 Hz, 1H), 7.95 - 7.88 (m, 1H), 7.84 - 7.76 (m, 2H), 7.71 (d, *J* = 8.2 Hz, 1H), 7.53 (td, *J* = 8.3, 7.8, 1.4 Hz, 1H), 7.43 (td, *J* = 7.5, 5.3 Hz, 2H), 5.79 (s, 1H), 4.22 - 4.15 (m, 1H), 2.95 (d, *J* = 4.8 Hz, 3H), 2.00 - 1.85 (m, 2H), 1.25 - 1.00 (m, 3H), 0.85 (t, *J* = 6.5 Hz, 1H). |
| **T06** | 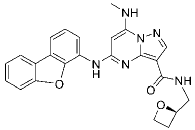 | MS m/z: 443 [M+H]⁺. ¹H NMR (400 MHz, DMSO-d6) δ 9.69 (s, 1H), 8.22 - 8.15 (m, 3H), 7.97 (t, *J* = 6.2 Hz, 2H), 7.97 - 7.86 (m, 2H), 7.72 (d, *J* = 8.2 Hz, 1H), 7.48 - 7.36 (m, 2H), 5.87 (s, 1H), 4.51 (p, *J* = 5.9 Hz, 1H), 4.35 - 4.28 (m, 1H), 4.26 (dt, *J* = 8.9, 6.0 Hz, 1H), 2.98 - 2.92 (m, 3H), 2.40 (dt, *J* = 16.5, 8.1 Hz, 1H), 2.14 - 1.92 (m, 1H). |
| **T07** | 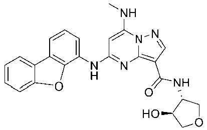 | MS m/z: 459 [M+H]⁺. ¹H NMR (400 MHz, DMSO-d6) δ 9.66 (s, 1H), 8.18 (d, *J* = 7.8 Hz, 2H), 7.93 (dd, *J* = 8.8, 6.4 Hz, 3H), 7.70 (dd, *J* = 17.6, 7.6 Hz, 2H), 7.59 - 7.50 (m, 1H), 7.44 (td, *J* = 7.7, 2.3 Hz, 2H), 5.87 (s, 1H), 5.41 (s, 1H), 5.22 (s, 1H), 4.01 - 3.92 (m, 1H), 3.90 - 3.74 (m, 1H), 3.73 - 3.56 (m, 1H), 3.25 - 3.18 (m, 1H), 2.94 (d, *J* = 4.8 Hz, 3H). |
| **T08** | 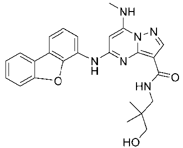 | MS m/z: 459 [M+H]⁺. ¹H NMR (400 MHz, DMSO-d6) δ 9.67 (s, 1H), 8.22 - 8.13 (m, 2H), 7.97 - 7.89 (m, 2H), 7.82 - 7.66 (m, 3H), 7.53 (ddd, *J* = 8.3, 7.2, 1.4 Hz, 1H), 7.48 - 7.36 (m, 2H), 5.80 (s, 1H), 4.48 (t, *J* = 6.3 Hz, 1H), 2.95 (d, *J* = 4.8 Hz, 3H), 2.83 (d, *J* = 6.3 Hz, 2H), 2.67 (d, *J* = 6.6 Hz, 2H), 0.43 (s, 6H). |
| **T09** | 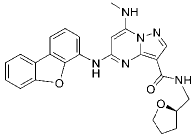 | MS m/z: 457 [M+H]⁺. ¹H NMR (400 MHz, DMSO-d6) δ 9.68 (s, 1H), 8.21 - 8.12 (m, 2H), 7.92 (dd, *J* = 7.6, 3.1 Hz, 3H), 7.83 (t, J = 6.0 Hz, 1H), 7.71 (d, *J* = 8.1 Hz, 1H), 7.54 (td, *J* = 8.2, 7.8, 1.3 Hz, 1H), 7.42 (q, *J* = 8.0 Hz, 2H), 5.85 (s, 1H), 3.56 (q, *J* = 6.6 Hz, 2H), 3.44 (q, *J* = 7.3 Hz, 1H), 3.12 - 2.97 (m, 1H), 2.95 (d, *J* = 4.8 Hz, 4H), 1.71-1.60 (m, 2H), 1.40 - 1.25 (m, 1H), 1.23 -1.17 (m, 1H). |
| **T10** | 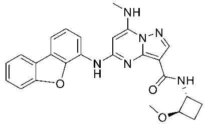 | MS m/z: 457 [M+H]⁺. ¹H NMR (400 MHz, DMSO-d6) δ 9.70 (s, 1H), 8.21 (d, *J* = 7.6 Hz, 1H), 8.20- 8.08 (m, 3H), 7.98 - 7.90 (m, 1H), 7.83 (d, *J* = 9.2 Hz, 1H), 7.70 (dd, *J* = 11.9, 8.0 Hz, 2H), 7.58 - 7.39 (m, 3H), 5.75 (s, 1H), 3.99 - 3.90 (m, 1H), 2.96 (d, *J* = 4.9 Hz, 3H), 2.86 (s, 3H), 2.60 - 2.52 (m, 1H), 1.64 - 1.51 (m, 2H), 0.35 (t, *J* = 10.2 Hz, 1H). |
| **T11** | 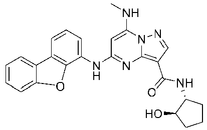 | MS m/z: 457 [M+H]⁺. ¹H NMR (400 MHz, DMSO-d6) δ 9.64 (s, 1H), 8.21 - 8.11 (m, 2H), 7.98 - 7.88 (m, 2H), 7.83 (d, *J* = 7.8 Hz, 1H), 7.71 (d, *J* = 8.3 Hz, 1H), 7.60 - 7.49 (m, 2H), 7.41 (dt, *J* = 13.2, 7.6 Hz, 2H), 5.81 (s, 1H), 4.71 (d, *J* = 4.2 Hz, 1H), 3.79 (s, 1H), 3.51 - 3.39 (m, 1H), 2.95 (d, *J* = 4.8 Hz, 3H), 2.00 (q, *J* = 7.1 Hz, 1H), 1.81 - 1.65 (m, 1H), 1.51 - 1.45 (m, 1H), 0.95 - 0.61 (m, 3H). |
| **T12** | 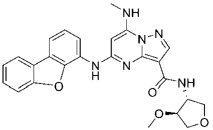 | MS m/z: 473 [M+H]⁺. ¹H NMR (400 MHz, DMSO-d6) δ 9.66 (s, 1H), 8.22 - 8.13 (m, 2H), 7.9 - 7.94 (m, 2H), 7.83 (d, *J* = 7.9 Hz, 1H), 7.70 (dd, *J* = 13.3, 7.7 Hz, 2H), 7.54 (dd, *J* = 8.2, 6.9 Hz, 1H), 7.44 (td, *J* = 7.6, 5.0 Hz, 2H), 5.83 (s, 1H), 4.20 - 4.12 (m, 1H), 3.82 - 3.75 (m, 2H), 3.51 - 3.42 (m, 1H), 3.31 - 3.21 (m, 2H), 3.13 (s, 3H), 2.95 (d, J = 4.9 Hz, 3H). |
| **T13** | 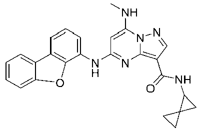 | MS m/z: 439 [M+H]⁺. ¹H NMR (400 MHz, DMSO-d6) δ 9.66 (s, 1H), 8.21 - 8.10 (m, 2H), 7.93 (t, *J* = 7.3 Hz, 2H), 7.80 - 7.66 (m, 3H), 7.54 (t, *J* = 7.8 Hz, 1H), 7.47 - 7.35 (m, 2H), 5.78 (s, 1H), 2.94 (d, *J* = 4.8 Hz, 3H), 2.07 - 1.91 (m, 1H), 1.51 - 1.41 (m, 1H), 1.10 - 0.98 (m, 1H), 0.90 - 0.80 (m, 1H), 0.74 - 0.67 (m, 1H), 0.38 - 0.21 (m, 2H). |
| **T14** | 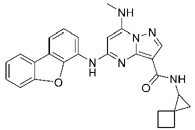 | MS m/z: 453 [M+H]⁺. ¹H NMR (400 MHz, DMSO-d6) δ 9.67 (s, 1H), 8.22 - 8.12 (m, 2H), 8.00 - 7.87 (m, 2H), 7.70 (dd, *J* = 13.2, 8.0 Hz, 2H), 7.57 - 7.47 (m, 2H), 7.42 (dt, *J* = 12.8, 7.6 Hz, 2H), 5.78 (s, 1H), 2.94 (d, *J* = 4.9 Hz, 3H), 2.20 - 1.80 (m, 3H), 1.51 - 0.41 (m, 1H), 0.88 - 0.75 (m, 1H), 0.54 (dd, *J* = 8.0, 5.9 Hz, 1H), - 0.18 (t, *J* = 5.2 Hz, 1H). |

### Example T16

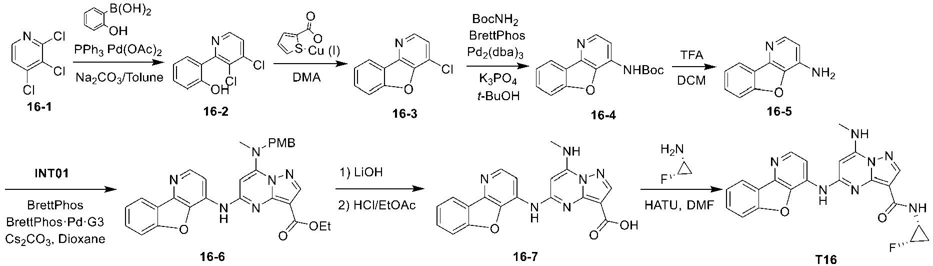

Step1: Mix **16-1** (4.0 g, 21.9 mmol), o-hydroxyphenylboronic acid (3.33 g, 24.1 mmol), Na₂CO₃ (4.65 g, 43.8 mmol), and PPh₃ (2.3 g, 0.4 mmol) in toluene (64 mL) and water (16 mL). Replace the atmosphere with nitrogen five times. Then, under nitrogen protection, add palladium acetate (492 mg, 2.19 mmol). Replace the atmosphere with nitrogen three times, stir at 80 °C overnight. Cool the reaction mixture, add water, and extract with EtOAc until the aqueous phase is free of product. Concentrate the organic phase under reduced pressure. Purify the crude product via silica gel chromatography (PE:EtOAc = 10:1) to obtain **16-2** (1.8 g, yield 62%). MS m/z: 240 [M+H]⁺.

Step2: To a 50 mL one-neck flask, **16-2** (127 mg, 0.53 mmol) and DMA (7 mL) were added. The flask was purged with nitrogen three times, and under nitrogen protection, copper(I) thieno[2,3-d]pyrimidine-2-carboxylate (121 mg, 0.63 mmol) was added. The flask was purged with nitrogen three times, and the reaction was stirred at 120 °C for 30 h. The reaction solution was concentrated under reduced pressure to obtain a crude product, which was purified by prep-TLC (PE:EtOAc = 10:1) to yield **16-3** (50 mg, yield 46%). MS m/z: 204 [M+H]⁺.

Step3: To a 25 mL Schlenk flask, add **16-3** (61 mg), *tert*-butyl carbamate (61 mg), K₃PO₄ (80 mg), BrretPhos (12 mg), and *tert*-butanol (10 mL). Replace the atmosphere with nitrogen three times, and under nitrogen protection, add Pd₂(dba)₃ (10 mg) to the reaction flask. Replace the atmosphere with nitrogen three times, and stir the reaction at 90 °C for 4 hours. Concentrate the reaction solution under reduced pressure to obtain a crude product, which is then purified by prep-TLC (PE:EtOAc = 6:1) to yield **16-4** (67 mg). MS m/z: 285 [M+H]⁺.

Step4: To a solution of **16-4** (193 mg, 0.068 mmol) in DCM (2 mL), TFA (2 mL) was added, and the reaction was stirred at room temperature for 2 hours. The reaction mixture was concentrated under reduced pressure to obtain **16-5** (257 mg, yield 100%), and the crude product was directly used for the next step. MS m/z: 185 [M+H]⁺.

Step5: To a 50 mL single-neck reaction flask, add **16-5** (41.28 mg, 0.224 mmol), **INT01** (70 mg, 0.187 mmol), Cs₂CO₃ (304 mg, 0.994 mmol), BrettPhos (20 mg, 0.037 mmol), and 1,4-dioxane (5 mL). Replace the atmosphere with nitrogen three times, and under nitrogen protection, add BrettPhos Pd G3 (17 mg, 0.019 mmol) to the reaction flask. Replace the atmosphere with nitrogen three times, and stir the reaction at 100 °C for 3 hours. Concentrate the reaction solution under reduced pressure to obtain a crude product, which is then purified by prep-TLC (PE:EtOAc = 2:1) to yield **16-6** (80.2 mg, yield 82%). MS m/z: 523 [M+H]⁺.

Step6 & Step7: Similar protocol as that for **T01,** using **16-6** to remove ethyl ester and PMB under the conditions of LiOH and HCl/EtOAc, respectively. MS m/z: 375 [M+H]⁺.

Step8: Similar protocol as that for **T01,** using **16-7.** MS m/z: 432 [M+H]⁺. ¹H NMR (400 MHz, DMSO-d6) δ10.24 (s, 1H), 8.47 (d, *J* = 5.4 Hz, 1H), 8.29 (s, 1H), 8.21 - 8.10 (m, 2H), 7.86 - 7.75 (m, 1H), 7.71 - 7.64 (m, 1H), 7.56 - 7.48 (m, 1H), 7.47 - 7.40 (m, 1H), 7.34 - 7.24 (m, 1H), 6.12 (s, 1H), 4.79 - 4.53 (m, 1H), 3.25 (d, *J* = 4.9 Hz, 3H), 2.62 (dqd, *J* = 9.1, 5.1, 4.3, 1.7 Hz, 1H), 1.13 - 0.94 (m, 1H), 0.90 - 0.75 (m, 1H).

### Example T17

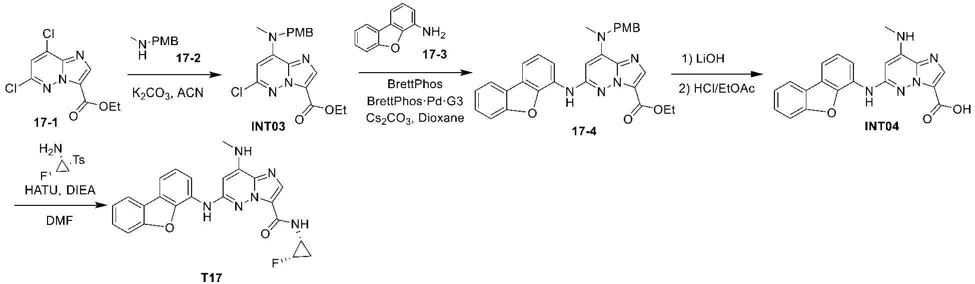

Step1: Dissolve **17-1** (250 mg, 0.96 mmol) in 5 mL of acetonitrile, add **17-2** (160 mg, 1.06 mmol) and K₂CO₃ (146 mg, 1.06 mmol). Stir the reaction system at room temperature overnight. Filter and concentrate the reaction system. Slurry the obtained product with 5 mL of PE:EtOAc = 2:1, filter to obtain pure **INT03** (300 mg, yield 83%). MS m/z: 375 [M+H]⁺.

Step2: Similar protocol as that for **T16,** using **INT03** and **17-3.** MS m/z: 522 [M+H]⁺.

Step3 & Step4: Similar protocol as that for **T01,** using **17-4** to remove ethyl ester and PMB under the conditions of LiOH and HCl/EtOAc, respectively. MS m/z: 374 [M+H]⁺.

Step5: Similar protocol as that for **T01,** using **INT04.** MS m/z: 431 [M+H]⁺. ¹H NMR (400 MHz, DMSO-d6) δ 9.29 (s, 1H), 8.60 (d, *J* = 4.1 Hz, 1H), 8.18 (d, *J* = 7.2 Hz, 1H), 7.92 (dd, *J* = 7.7, 1.2 Hz, 1H), 7.85 (s, 1H), 7.70 (t, *J* = 7.4 Hz, 2H), 7.58 - 7.48 (m, 2H), 7.47 - 7.34 (m, 2H), 6.05 (s, 1H), 4.64 -4.45 (m, 1H), 2.91 (d, *J* = 4.9 Hz, 3H), 2.05 - 1.95 (m, 1H), 0.84 (dq, *J* = 14.8, 8.2, 7.2 Hz, 1H), 0.35 - 0.22 (m, 1H).

The synthesis methods for **T18-T19** are similar to that of **T01,** involving the condensation of **INT04** with different amines or amine salts under the same conditions. The identification data for **T18-T19** are shown in Table 2.

**Table 2. Identification data for T18-T19**

| Example | Structure | MS & NMR |
|---|---|---|
| **T18** | 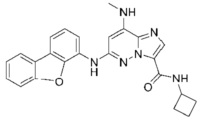 | MS m/z: 427 [M+H]⁺. ¹H NMR (400 MHz, DMSO-d6) δ 9.25 (s, 1H), 8.56 (d, *J* = 8.3 Hz, 1H), 8.19 (dd, *J* = 7.6, 1.2 Hz, 1H), 7.99 (dd, *J* = 7.7, 1.2 Hz, 1H), 7.77 (s, 1H), 7.74 - 7.64 (m, 2H), 7.57 - 7.34 (m, 4H), 5.99 (s, 1H), 4.16 (q, *J* = 8.3 Hz, 1H), 2.92 (d, *J= 4.9* Hz, 3H), 1.84 (q, *J* = 8.1, 7.6 Hz, 2H), 1.36 (s, 1H), 1.31 (d, *J* = 10.0 Hz, 1H), 1.01 (q, *J* = 10.2 Hz, 2H). |
| **T19** | 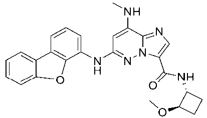 | MS m/z: 457 [M+H]⁺. ¹H NMR (400 MHz, DMSO-d6) δ 9.24 (s, 1H), 8.61 (d, *J* = 9.1 Hz, 1H), 8.22 (d, *J* = 7.5 Hz, 1H), 8.04 (d, *J* = 7.7 Hz, 1H), 7.78 (s, 1H), 7.71 (d, *J* = 8.2 Hz, 1H), 7.63 - 7.39 (m, 6H), 5.96 (s, 1H), 3.96 - 3.87 (m, 1H), 2.93 (d, *J*= 4.9 Hz, 3H), 2.78 (s, 3H), 2.60 - 2.52 (m, 1H), 1.68 - 1.60 (m, 2H), 0.26 - 0.16 (m, 1H). |

### Example T20

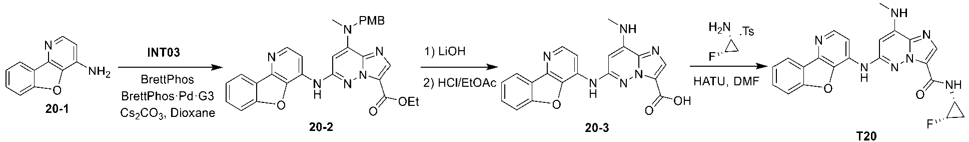

Step1: Similar protocol as that for **T16,** using **INT03** and **20-1** (same as **16-5).** MS m/z: 523 [M+H]⁺.

Step2 & Step3: Similar protocol as that for **T01,** using **20-2** to remove ethyl ester and PMB under the conditions of LiOH and HCl/EtOAc, respectively. MS m/z: 375 [M+H]⁺.

Step4: Similar protocol as that for **T16,** using **20-3.** MS m/z: 432 [M+H]⁺. ¹H NMR (400 MHz, DMSO-d6) δ 9.95 (s, 1H), 8.47 (d, *J* = 5.5 Hz, 1H), 7.95 (s, 1H), 7.89 (d, *J* = 5.5 Hz, 1H), 7.79 (d, J = 8.7 Hz, 1H), 7.68 (s, 1H), 7.45 - 7.33 (m, 1H), 7.26 (d, *J* = 8.7 Hz, 1H), 6.87 (s, 1H), 6.64 (s, 1H), 6.25 (s, 1H), 4.63 - 4.44 (m, 1H), 2.91 (d, *J* = 4.9 Hz, 3H), 2.05 - 1.95 (m, 1H), 0.84 (dq, *J* = 14.8, 8.2, 7.2 Hz, 1H), 0.35 - 0.22 (m, 1H).

### Example T21

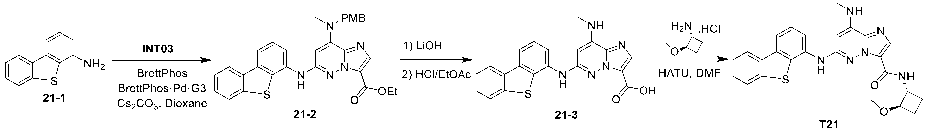

Step1: Similar protocol as that for **T16,** using **INT03** and **21-1.** MS m/z: 538 [M+H]⁺.

Step2 & Step3: Similar protocol as that for **T01,** using **21-2** to remove ethyl ester and PMB under the conditions of LiOH and HCl/EtOAc, respectively. MS m/z: 390 [M+H]⁺.

Step4: Similar protocol as that for **T01,** using **21-3.** MS m/z: 473 [M+H]⁺. ¹H NMR (400 MHz, DMSO-d6) δ 9.34 (s, 1H), 8.52 (d, *J* = 9.4 Hz, 1H), 8.45 (s, 1H), 8.34 (d, *J* = 7.8 Hz, 1H), 8.01 (d, *J* = 6.2 Hz, 1H), 7.76 (s, 1H), 7.66 - 7.44 (m, 6H), 5.89 (s, 1H), 3.86 - 3.77 (m, 1H), 2.94 (d, *J* = 4.8 Hz, 3H), 2.66 (s, 3H), 2.02 (dd, *J* = 15.5, 7.7 Hz, 1H), 1.44 (d, *J* = 9.9 Hz, 1H), 1.05 - 0.95 (m, 1H), -0.05 - -0.15 (m, 1H).

### Example T22

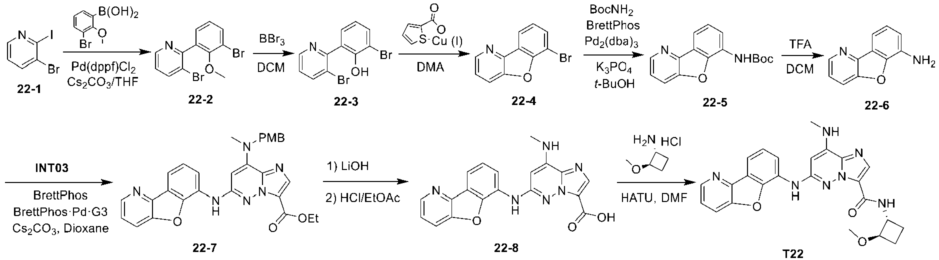

Step1: Dissolve **22-1** (1.0 g, 3.52 mmol) in THF (80 mL), add 3-bromo-2-methoxyphenylboronic acid (813.2 mg, 3.52 mmol), and replace with N₂ five times. Then, under nitrogen protection, add Cs₂CO₃ (2.3 g, 7.04 mmol) and Pd(dppf)Cl₂ (285.3 mg, 0.352 mmol). Replace the mixture with nitrogen three times, stir in an oil bath at 65 °C for 3 hours. Cool the reaction mixture, add water, and extract with DCM until the aqueous phase is free of product. Concentrate the organic phase under reduced pressure, and purify the crude product via silica gel chromatography (PE:EtOAc = 5:1) to obtain **22-2** (855 mg, yield 70%). MS m/z: 344 [M+H]⁺.

Step2: Dissolve **22-2** (850 mg, 2.48 mmol) in DCM (30 mL), add BBr₃ (3.7 g, 14.87 mmol), and stir at room temperature for 1 hour. Quench the reaction solution with saturated NaHCO₃ solution, add EtOAc for extraction until the aqueous phase is free of product. Concentrate the organic phase under reduced pressure, and purify the crude product via silica gel chromatography (PE:EtOAc = 5:1) to obtain **22-3** (610 mg, yield 75%). MS m/z: 330 [M+H]⁺.

Step3: Similar protocol as that for **T16,** using **22-3.** MS m/z: 248, 250 [M+H]⁺.

Step4: Similar protocol as that for **T16,** using **22-4.** MS m/z: 285 [M+H]⁺.

Step5: Similar protocol as that for **T16,** using **22-5.** MS m/z: 185 [M+H]⁺.

Step6: Similar protocol as that for **T16,** using **INT03** and **22-6.** MS m/z: 523 [M+H]⁺.

Step7 & Step8: Similar protocol as that for **T01,** using 22-7 to remove ethyl ester and PMB under the conditions of LiOH and HCl/EtOAc, respectively. MS m/z: 375 [M+H]⁺.

Step9: Similar protocol as that for **T01,** using **22-8.** MS m/z: 458 [M+H]⁺. ¹H NMR (400 MHz, DMSO-d6) δ 9.33 (s, 1H), 8.68 (dd, *J* = 4.8, 1.3 Hz, 1H), 8.58 (d, *J* = 9.0 Hz, 1H), 8.23 - 8.16 (m, 2H), 8.06 (d,*J* = 7.6 Hz, 1H), 7.81 - 7.71 (m, 2H), 7.57 (dd, *J* = 8.1, 5.3 Hz, 3H), 5.97 (s, 1H), 3.99 - 3.88 (m, 1H), 2.90 (d,*J* = 4.8 Hz, 3H), 2.81 (s, 3H), 2.44 - 2.22 (m, 1H), 1.63 (dd, *J* = 16.6, 9.2 Hz, 2H), 0.29 - 0.06 (m, 1H).

### Example T23

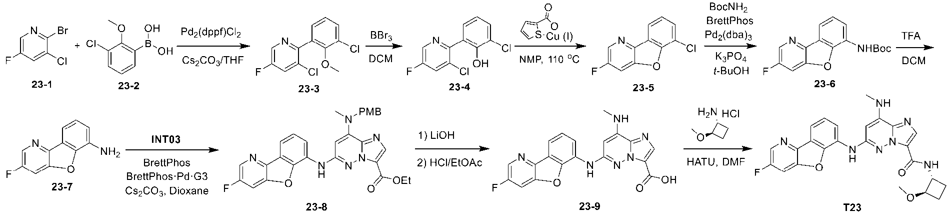

Step1: Similar protocol as that for **T22,** using **23-1** and **23-2.** MS m/z: 272 [M+H]⁺.

Step2: Similar protocol as that for **T22,** using **23-3.** MS m/z: 258 [M+H]⁺.

Step3: Similar protocol as that for **T16,** using **23-4** in NMP and temperature is 110 °C. MS m/z: 222 [M+H]⁺.

Step4: Similar protocol as that for **T16,** using **23-5.** MS m/z: 303 [M+H]⁺.

Step5: Similar protocol as that for **T16,** using **23-6.** MS m/z: 203 [M+H]⁺.

Step6: Similar protocol as that for **T16,** using **INT03** and **23-7.** MS m/z: 541 [M+H]⁺.

Step7 & Step8: Similar protocol as that for **T01,** using **23-8** to remove ethyl ester and PMB under the conditions of LiOH and HCl/EtOAc, respectively. MS m/z: 393 [M+H]⁺.

Step9: Similar protocol as that for **T01,** using **23-9.** MS m/z: 476 [M+H]⁺. ¹H NMR (400 MHz, DMSO-d6) δ 9.36 (s, 1H), 8.74 (dd, *J* = 2.5, 1.3 Hz, 1H), 8.58 (d, *J* = 9.0 Hz, 1H), 8.35 (dd, *J* = 9.2, 2.5 Hz, 1H), 8.30 - 8.20 (bs, 1H), 8.03 (dd, *J* = 7.8, 1.2 Hz, 1H), 7.79 (s, 1H), 7.74 (d, *J* = 7.8 Hz, 1H), 7.58 (t, *J* = 7.7 Hz, 2H), 5.96 (s, 1H), 3.94 (p, *J* = 8.9 Hz, 1H), 2.93 (d, *J* = 4.9 Hz, 3H), 2.84 (s, 3H), 2.47 - 2.38 (m, 1H), 1.65 (t, *J* = 9.1 Hz, 2H), 0.19 (q, *J* = 9.9 Hz, 1H).

### Example T24

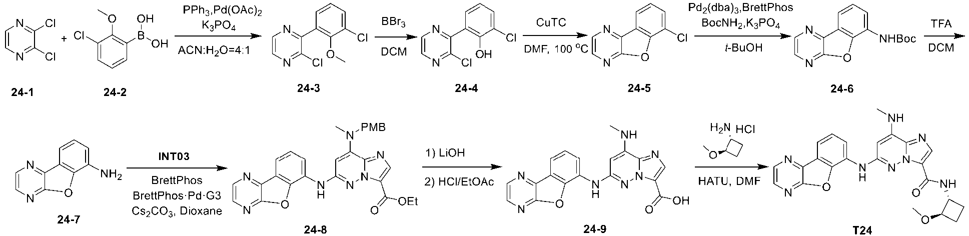

Step1: To a 50 mL three-neck flask, add **24-1** (1.0 g, 6.71 mmol), **24-2** (1.26 g, 6.76 mmol), PPh₃ (265.0 mg, 1.01 mmol), K₃PO₄ (4.28 g, 20.18 mmol), acetonitrile (20 mL), and water (5 mL). Replace the atmosphere with nitrogen three times. Under nitrogen protection, add palladium acetate (126.0 mg, 0.56 mmol) to the above reaction flask. Replace the atmosphere with nitrogen three times, stir and react at 60 °C for 3 hours. After the reaction is complete, add water to quench the reaction, and then extract with DCM. Combine the organic phases, wash with saturated saline solution, dry over anhydrous Na₂SO₄, and concentrate under reduced pressure to obtain a crude product. Purify the crude product by column chromatography (PE:EtOAc = 5:1) to obtain **24-3** (1.53 g, yield 87.7%). MS m/z: 255 [M+H]⁺.

Step2: Similar protocol as that for **T22,** using **24-3.** MS m/z: 241 [M+H]⁺.

Step3: Similar protocol as that for **T16,** using **24-4** in DMF and temperature is 100 °C. MS m/z: 205 [M+H]⁺.

Step4: Similar protocol as that for **T16,** using **24-5.** MS m/z: 286 [M+H]⁺.

Step5: Similar protocol as that for **T16,** using **24-6.** MS m/z: 186 [M+H]⁺.

Step6: Similar protocol as that for **T16,** using **INT03** and **24-7.** MS m/z: 524 [M+H]⁺.

Step7 & Step8: Similar protocol as that for **T01,** using **24-8** to remove ethyl ester and PMB under the conditions of LiOH and HCl/EtOAc, respectively. MS m/z: 376 [M+H]⁺.

Step9: Similar protocol as that for **T01,** using **24-9.** MS m/z: 459 [M+H]⁺. ¹H NMR (400 MHz, DMSO-d6) δ 9.47 (s, 1H), 8.81 (d, *J* = 2.8 Hz, 1H), 8.65 (d, *J* = 9.1 Hz, 1H), 8.56 (d, *J* = 2.7 Hz, 1H), 8.45 - 8.25 (bs, 1H), 8.08 (dd, *J* = 7.8, 1.2 Hz, 1H), 7.95 (dd, *J* = 7.9, 1.1 Hz, 1H), 7.82 (s, 1H), 7.67 - 7.58 (m, 2H), 6.05 (s, 1H), 4.01 (p, *J* = 8.8 Hz, 1H), 2.97 - 2.86 (m, 6H), 2.65 (q, *J* = 7.5 Hz, 1H), 1.71 (t, *J* = 9.3 Hz, 2H), 0.37 (p, *J* = 10.0 Hz, 1H).

### Example T25

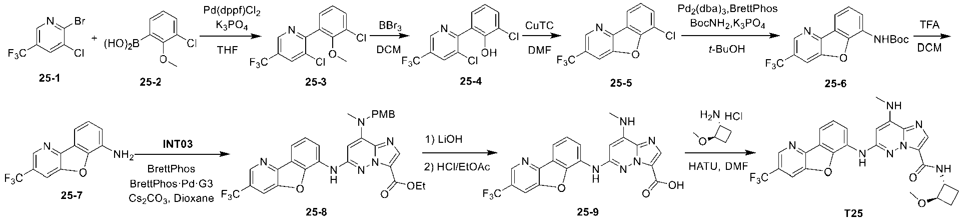

Step1: Dissolve **25-1** (500 mg, 1.91 mmol) in anhydrous THF (20 mL), add K₃PO₄ (815 mg, 3.82 mmol) and **25-2** (357 mg, 1.91 mmol), replace nitrogen three times, then add Pd(dppf)Cl₂ (140 mg, 0.19 mmol), replace nitrogen three times, and react the mixture at 60 °C for 3 hours. The reaction solution is concentrated under reduced pressure, and the crude product is purified by prep-TLC (PE:EtOAc = 10:1) to obtain **25-3** (384 mg, yield 62%). MS m/z: 322 [M+H]⁺.

Step2: Similar protocol as that for **T22,** using **25-3.** MS m/z: 308 [M+H]⁺.

Step3: Similar protocol as that for **T24,** using **25-4.** MS m/z: 272 [M+H]⁺.

Step4: Similar protocol as that for **T16,** using **25-5.** MS m/z: 353 [M+H]⁺.

Step5: Similar protocol as that for **T16,** using **25-6.** MS m/z: 253 [M+H]⁺.

Step6: Similar protocol as that for **T16,** using **INT03** and **25-7.** MS m/z: 591 [M+H]⁺.

Step7 & Step8: Similar protocol as that for **T01,** using **25-8** to remove ethyl ester and PMB under the conditions of LiOH and HCl/EtOAc, respectively. MS m/z: 443 [M+H]⁺.

Step9: Similar protocol as that for **T01,** using **25-9.** MS m/z: 526 [M+H]⁺. ¹H NMR (400 MHz, DMSO-d6) δ 9.43 (s, 1H), 9.09 (dd, *J* = 2.0, 0.9 Hz, 1H), 8.78 (d, *J* = 1.9 Hz, 1H), 8.52 (d, *J* = 9.0 Hz, 1H), 8.32 - 8.20 (bs, 1H), 8.23 (s, 1H), 7.84 (dd, *J* = 7.8, 1.2 Hz, 1H), 7.80 (s, 1H), 7.69 - 7.57 (m, 2H), 5.99 (s, 1H), 3.91 (p, *J* = 8.7 Hz, 1H), 2.94 (d, *J* = 4.9 Hz, 3H), 2.79 (s, 3H), 2.37 - 2.27 (m, 1H), 1.61 (t, *J* = 9.9 Hz, 2H), 0.12 (p, *J* = 9.9 Hz, 1H).

### Example T26

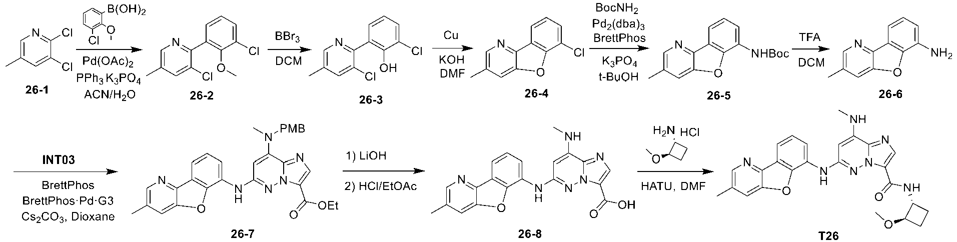

Step1: Similar protocol as that for **T24,** using **26-1.** MS m/z: 268 [M+H]⁺.

Step2: Similar protocol as that for **T22,** using **26-2.** MS m/z: 254 [M+H]⁺.

Step3: Dissolve **26-3** (100 mg, 0.40 mmol) in DMF (5 mL), then add KOH (44.35 mg, 0.79 mmol) and Cu (30 mg, 0.47 mmol), stir and reflux overnight in an oil bath at 85 °C. Cool down, add EDTA for complexation, and extract with DCM. Concentrate the organic phase under reduced pressure to obtain **26-4** (30 mg, yield 35%). MS m/z: 218 [M+H]⁺.

Step4: Similar protocol as that for **T16,** using **26-4.** MS m/z: 299 [M+H]⁺.

Step5: Similar protocol as that for **T16,** using **26-5.** MS m/z: 199 [M+H]⁺.

Step6: Similar protocol as that for **T16,** using **INT03** and **26-6.** MS m/z: 537 [M+H]⁺.

Step7 & Step8: Similar protocol as that for **T01,** using 26-7 to remove ethyl ester and PMB under the conditions of LiOH and HCl/EtOAc, respectively. MS m/z: 389 [M+H]⁺.

Step9: Similar protocol as that for **T01,** using 26-8. MS m/z: 472 [M+H]⁺. ¹H NMR (400 MHz, DMSO-d6) δ 9.31 (s, 1H), 8.63 - 8.53 (m, 2H), 8.27 - 8.10 (bs, 1H), 8.05 - 7.97 (m, 1H), 7.82 (d, *J* = 7.2 Hz, 1H), 7.78 (s, 1H), 7.69 (d, *J* = 7.7 Hz, 1H), 7.53 (p, *J* = 7.3 Hz, 2H), 5.96 (s, 1H), 3.94 (p, *J* = 8.1 Hz, 1H), 2.93 (d, *J* = 4.8 Hz, 3H), 2.81 (s, 3H), 2.48 (s, 3H), 2.08 - 1.90 (m, 1H), 1.63 (q, *J* = 9.8 Hz, 2H), 0.17 (t, *J =* 10.3 Hz, 1H).

### Example T27

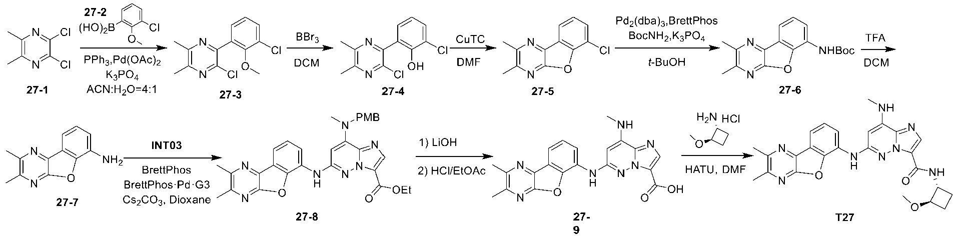

Step1: Similar protocol as that for **T24,** using **27-1.** MS m/z: 283 [M+H]⁺.

Step2: Similar protocol as that for **T22,** using **27-3.** MS m/z: 269 [M+H]⁺.

Step3: Similar protocol as that for **T24,** using **27-4.** MS m/z: 233 [M+H]⁺.

Step4: Similar protocol as that for **T16,** using **27-5.** MS m/z: 314 [M+H]⁺.

Step5: Similar protocol as that for **T16,** using **27-6.** MS m/z: 214 [M+H]⁺.

Step6: Similar protocol as that for **T16,** using **INT03** and **27-7.** MS m/z: 552 [M+H]⁺.

Step7 & Step8: Similar protocol as that for **T01,** using **27-8** to remove ethyl ester and PMB under the conditions of LiOH and HCl/EtOAc, respectively. MS m/z: 404 [M+H]⁺.

Step9: Similar protocol as that for **T01,** using **27-9.** MS m/z: 487 [M+H]⁺. ¹H NMR (400 MHz, DMSO-d6) δ 9.39 (s, 1H), 8.59 (dd, *J* = 19.7, 8.4 Hz, 2H), 8.04 - 7.97 (m, 1H), 7.84 - 7.77 (m, 2H), 7.63 - 7.51 (m, 2H), 6.01 (s, 1H), 4.02 - 3.93 (m, 1H), 2.96 - 2.83 (m, 6H), 2.70 (s, 3H), 2.55 (s, 3H), 2.65 - 2.51 (m, 1H), 1.68 (q, *J =* 9.6 Hz, 2H), 0.31 (t, *J* = 10.1 Hz, 1H).

### Example T28

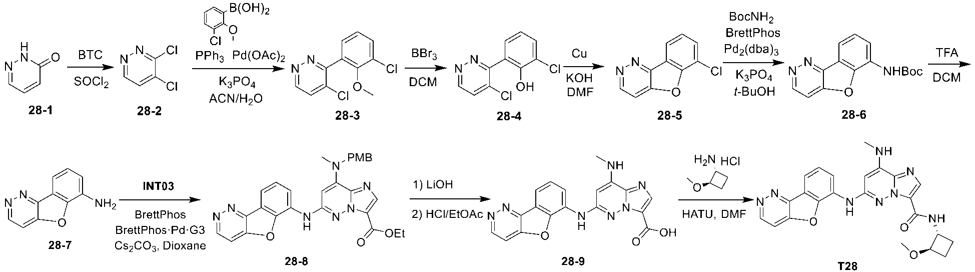

Step1: Dissolve **28-1** (1.0 g, 10.41 mmol) in DMAP (100 mL), add thionyl chloride (4 mL), and then dissolve BTC (6.17 g, 20.82 mmol) in another 8 mL of thionyl chloride. Slowly add the above mixture dropwise. Stir in an oil bath at 80 °C for 12 hours. Cool down, quench with water, and adjust the pH to 8 with a saturated Na₂CO₃ solution. Extract with DCM until the aqueous phase is free of product, then concentrate under reduced pressure. Purify the crude product via silica gel chromatography (PE:EtOAc = 10:1= 1:1) to obtain **28-2** (46 mg). MS m/z: 149 [M+H]⁺.

Step2: Similar protocol as that for **T24,** using **28-2.** MS m/z: 255 [M+H]⁺.

Step3: Similar protocol as that for **T22,** using **28-3.** MS m/z: 241 [M+H]⁺.

Step4: Similar protocol as that for **T26,** using **28-4.** MS m/z: 205 [M+H]⁺.

Step5: Similar protocol as that for **T16,** using **28-5.** MS m/z: 286 [M+H]⁺.

Step6: Similar protocol as that for **T16,** using **28-6.** MS m/z: 186 [M+H]⁺.

Step7: Similar protocol as that for **T16,** using **INT03** and **28-7.** MS m/z: 524 [M+H]⁺.

Step8 & Step9: Similar protocol as that for **T01,** using **28-8** to remove ethyl ester and PMB under the conditions of LiOH and HCl/EtOAc, respectively. MS m/z: 376 [M+H]⁺.

Step 10: Similar protocol as that for **T01,** using **28-9.** MS m/z: 459 [M+H]⁺. ¹H NMR (400 MHz, DMSO-d6) δ 9.47 (s, 1H), 9.37 (d, *J* = 6.0 Hz, 1H), 8.78 - 8.70 (bs, 1H), 8.57 (d, *J* = 8.9 Hz, 1H), 8.26 (dd, *J* = 7.8, 1.2 Hz, 1H), 8.13 (d, *J* = 5.9 Hz, 1H), 7.92 (dd, *J* = 7.9, 1.2 Hz, 1H), 7.81 (s, 1H), 7.68 (t, *J* = 7.8 Hz, 2H), 5.99 (s, 1H), 3.96 (p, *J* = 8.9 Hz, 1H), 2.93 (d, *J* = 4.8 Hz, 3H), 2.84 (s, 3H), 2.60 - 2.52 (m, 1H), 1.67 (t, *J* = 10.8 Hz, 2H), 0.27 (t, *J* = 10.1 Hz, 1H).

### Example T29

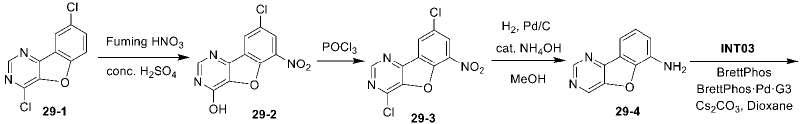

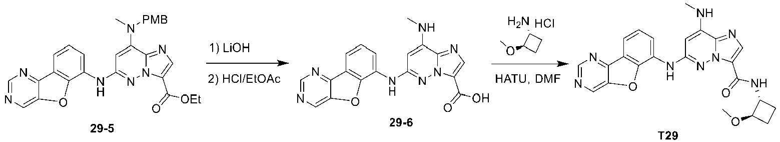

Step1: Dissolve **29-1** (50 mg, 209 µmol) in 1 mL of concentrated sulfuric acid, then add fuming nitric acid (130 mg, 2.09 mmol). Stir the mixture at room temperature for 40 minutes. LC-MS analysis reveals complete conversion of the starting material, with a hydrolysis side reaction observed in the product. Slowly add the system to a saturated Na₂CO₃ solution, and extract with EtOAc. Concentrate the organic phase under reduced pressure, and purify the crude product via silica gel column chromatography (PE:EtOAc = 5:1) to obtain **29-2** (30 mg). MS m/z: 266 [M+H]⁺.

Step2: Dissolve **29-2** (814 mg, 3.06 mmol) in POCl₃ (8 mL), allow the reaction system to react at 120 °C for 5 hours, then gradually add the reaction mixture to ice-cold water. Extract with EtOAc. Concentrate the organic phase under reduced pressure, and purify the crude product via silica gel chromatography (PE:EtOAc = 3:1) to obtain **29-3** (572 mg). MS m/z: 284 [M+H]⁺.

Step3: Dissolve **29-3** (385 mg, 1.35 mmol) in MeOH (5 mL), add ammonia water (5 mL) and Pd/C (35 mg), and react the system under a hydrogen pressure of 5 MPa for 15 hours. Filter and concentrate the reaction solution. Purify the crude product via silica gel column chromatography (DCM:MeOH = 10:1) to obtain **29-4** (54 mg). MS m/z: 186 [M+H]⁺.

Step4: Similar protocol as that for **T16,** using **INT03** and **29-4.** MS m/z: 524 [M+H]⁺.

Step5 & Step6: Similar protocol as that for **T01,** using **29-5** to remove ethyl ester and PMB under the conditions of LiOH and HCl/EtOAc, respectively. MS m/z: 376 [M+H]⁺.

Step7: Similar protocol as that for **T01,** using 29-6. MS m/z: 459 [M+H]⁺. ¹H NMR (400 MHz, DMSO-d6) δ 9.35 (s, 1H), 9.30 (s, 1H), 9.11 (s, 1H), 8.61 (d, *J* = 8.8 Hz, 1H), 8.25 (bs, 1H), 7.89 - 7.74 (m, 3H), 7.64 (t, *J* = 6.0 Hz, 2H), 6.11 (s, 1H), 4.04 (p, *J* = 8.8 Hz, 1H), 3.23 - 2.93 (m, 6H), 1.95 - 1.79 (m, 2H), 1.41 (dt, *J* = 19.2, 9.5 Hz, 1H), 0.70 (t, *J* = 10.1 Hz, 1H).

### Example T30

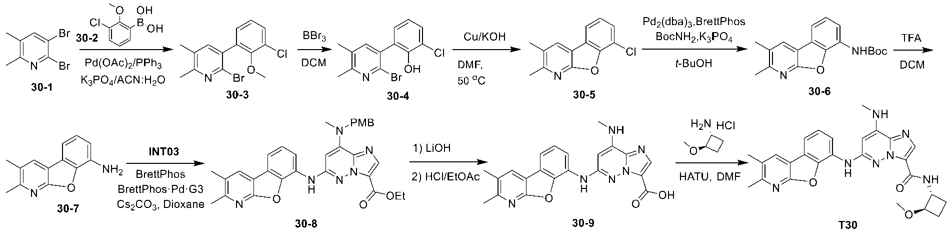

Step1: Similar protocol as that for **T24,** using **30-1.** MS m/z: 326 [M+H]⁺.

Step2: Similar protocol as that for **T22,** using **30-3.** MS m/z: 312 [M+H]⁺.

Step3: Similar protocol as that for **T26,** using **30-4.** MS m/z: 232 [M+H]⁺.

Step4: Similar protocol as that for **T16,** using **30-5.** MS m/z: 313 [M+H]⁺.

Step5: Similar protocol as that for **T16,** using **30-6.** MS m/z: 213 [M+H]⁺.

Step6: Similar protocol as that for **T16,** using **INT03** and **30-7.** MS m/z: 551 [M+H]⁺.

Step7 & Step8: Similar protocol as that for **T01,** using **30-8** to remove ethyl ester and PMB under the conditions of LiOH and HCl/EtOAc, respectively. MS m/z: 403 [M+H]⁺.

Step9: Similar protocol as that for **T01,** using **30-9.** MS m/z: 486 [M+H]⁺. ¹H NMR (400 MHz, DMSO-d6) δ 9.28 (s, 1H), 8.58 (d, *J* = 9.1 Hz, 1H), 8.25 (bs, 1H), 7.97 (dd, *J* = 7.7, 1.1 Hz, 2H), 7.66 (d, *J* = 7.7 Hz, 1H), 8.80 (s, 1H), 7.59 - 7.45 (m, 2H), 5.96 (s, 1H), 3.93 (q, *J* = 8.7 Hz, 1H), 2.93 (d, *J* = 4.9 Hz, 3H), 2.82 (s, 3H), 2.59 (s, 3H), 2.55 - 2.50 (m, 1H), 2.41 (s, 3H), 1.64 (dq, *J* = 19.2, 9.3 Hz, 2H), 0.25 (q, *J* = 10.1, 9.7 Hz, 1H).

### Example T31

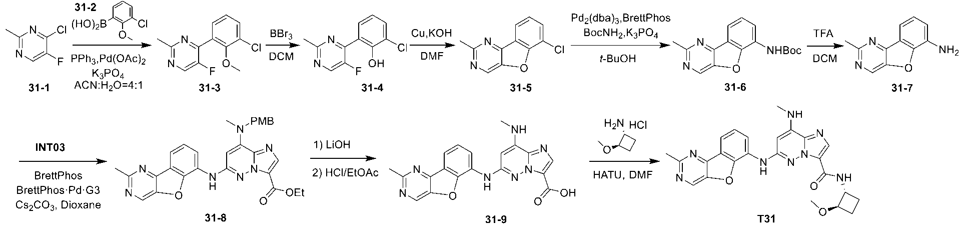

Step1: Similar protocol as that for **T24,** using **31-1.** MS m/z: 253 [M+H]⁺.

Step2: Similar protocol as that for **T22,** using **31-3.** MS m/z: 239 [M+H]⁺.

Step3: Similar protocol as that for **T26,** using **31-4.** MS m/z: 219 [M+H]⁺.

Step4: Similar protocol as that for **T16,** using **31-5.** MS m/z: 300 [M+H]⁺.

Step5: Similar protocol as that for **T16,** using **31-6.** MS m/z: 200 [M+H]⁺.

Step6: Similar protocol as that for **T16,** using **INT03** and **31-7.** MS m/z: 538 [M+H]⁺.

Step7 & Step8: Similar protocol as that for **T01,** using **31-8** to remove ethyl ester and PMB under the conditions of LiOH and HCl/EtOAc, respectively. MS m/z: 390 [M+H]⁺.

Step9: Similar protocol as that for **T01,** using **31-9.** MS m/z: 473 [M+H]⁺. ¹H NMR (400 MHz, DMSO-d6) δ 9.43 (s, 1H), 9.21 (s, 1H), 8.57 (d, *J* = 9.0 Hz, 1H), 8.25 (bs, 1H), 8.09 (dd, *J* = 7.9, 1.2 Hz, 1H), 7.94 (dd, *J* =7.8, 1.2 Hz, 1H), 7.81 (s, 1H), 7.66 - 7.57 (m, 2H), 6.00 (s, 1H), 4.05 - 3.92 (m, 1H), 2.93 (d, *J* = 4.9 Hz, 3H), 2.87 (s, 3H), 2.80 (s, 3H), 2.61 (q, *J* = 7.6 Hz, 1H), 1.69 (q, *J* = 9.8 Hz, 2H), 0.34 (t, *J* = 10.0 Hz, 1H).

### Example T32

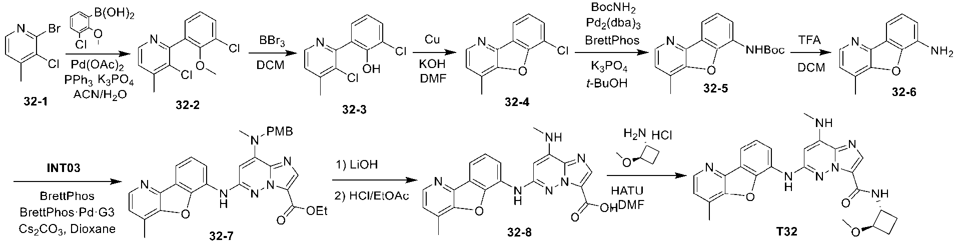

Step1: Similar protocol as that for **T24,** using **32-1.** MS m/z: 268 [M+H]⁺.

Step2: Similar protocol as that for **T22,** using **32-2.** MS m/z: 254 [M+H]⁺.

Step3: Similar protocol as that for **T26,** using **32-3.** MS m/z: 218 [M+H]⁺.

Step4: Similar protocol as that for **T16,** using **32-4.** MS m/z: 299 [M+H]⁺.

Step5: Similar protocol as that for **T16,** using **32-5.** MS m/z: 199 [M+H]⁺.

Step6: Similar protocol as that for **T16,** using **INT03** and **32-6.** MS m/z: 537 [M+H]⁺.

Step7 & Step8: Similar protocol as that for **T01,** using **32-7** to remove ethyl ester and PMB under the conditions of LiOH and HCl/EtOAc, respectively. MS m/z: 389 [M+H]⁺.

Step9: Similar protocol as that for **T01,** using **32-8.** MS m/z: 472 [M+H]⁺. ¹H NMR (400 MHz, DMSO-d6) δ 9.34 (s, 1H), 8.66 (d, *J* = 8.2 Hz, 1H), 8.54 (d, *J* = 4.8 Hz, 1H), 8.18 (d, *J* = 8.4 Hz, 1H), 8.01 (d, *J* = 7.5 Hz, 1H), 7.81 (s, 1H), 7.76 (d, *J* = 7.9 Hz, 1H), 7.54 (s, 1H), 7.43 (s, 1H), 7.22 (s, 1H), 6.00 (s, 1H), 4.05 - 3.92 (m, 1H), 2.94 (d, *J* = 4.6 Hz, 3H), 2.85 (s, 3H), 2.72 (s, 3H), 2.67 (q, *J* = 7.6 Hz, 1H), 1.62 (q, *J=* 9.8 Hz, 2H), 0.85 (d, *J* = 7.0 Hz, 1H).

### Example T33

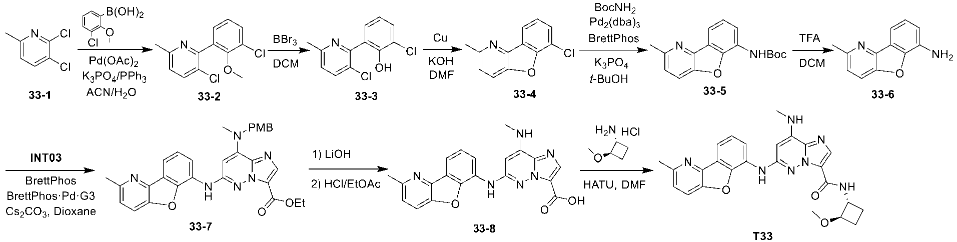

Step1: Similar protocol as that for **T24,** using **33-1.** MS m/z: 268 [M+H]⁺.

Step2: Similar protocol as that for **T22,** using **33-2.** MS m/z: 254 [M+H]⁺.

Step3: Similar protocol as that for **T26,** using **33-3.** MS m/z: 218 [M+H]⁺.

Step4: Similar protocol as that for **T16,** using **33-4.** MS m/z: 299 [M+H]⁺.

Step5: Similar protocol as that for **T16,** using **33-5.** MS m/z: 199 [M+H]⁺.

Step6: Similar protocol as that for **T16,** using **INT03** and 33-6. MS m/z: 537 [M+H]⁺.

Step7 & Step8: Similar protocol as that for **T01,** using **33-7** to remove ethyl ester and PMB under the conditions of LiOH and HCl/EtOAc, respectively. MS m/z: 389 [M+H]⁺.

Step9: Similar protocol as that for **T01,** using **33-8.** MS m/z: 472 [M+H]⁺. ¹H NMR (400 MHz, DMSO-d6) δ 9.30 (s, 1H), 8.58 (d, *J* = 9.0 Hz, 1H), 8.25 (bs, 1H), 8.10 - 7.98 (m, 2H), 7.79 (s, 1H), 7.72 (dd, *J* = 7.7, 1.2 Hz, 1H), 7.60 - 7.48 (m, 2H), 7.42 (d, *J* = 8.7 Hz, 1H), 5.97 (s, 1H), 3.95 (p, *J* = 8.9 Hz, 1H), 2.93 (d, *J* = 4.9 Hz, 3H), 2.83 (s, 3H), 2.65 (s, 3H), 1.65 (dd, *J* = 15.2, 9.2 Hz, 2H), 0.26 (q, *J* = 10.0 Hz, 1H).

### Example T35

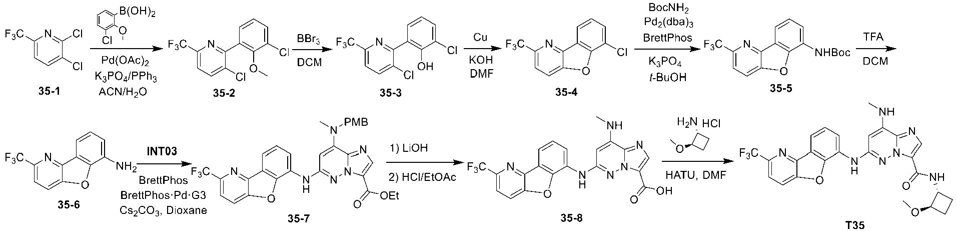

Step1: Similar protocol as that for **T24,** using **35-1.** MS m/z: 322 [M+H]⁺.

Step2: Similar protocol as that for **T22,** using **35-2.** MS m/z: 308 [M+H]⁺.

Step3: Similar protocol as that for **T26,** using **35-3.** MS m/z: 272 [M+H]⁺.

Step4: Similar protocol as that for **T16,** using **35-4.** MS m/z: 353 [M+H]⁺.

Step5: Similar protocol as that for **T16,** using **35-5.** MS m/z: 253 [M+H]⁺.

Step6: Similar protocol as that for **T16,** using **INT03** and **35-6.** MS m/z: 591 [M+H]⁺.

Step7 & Step8: Similar protocol as that for **T01,** using **35-7** to remove ethyl ester and PMB under the conditions of LiOH and HCl/EtOAc, respectively. MS m/z: 443 [M+H]⁺.

Step9: Similar protocol as that for **T01,** using **35-8.** MS m/z: 526 [M+H]⁺. ¹H NMR (400 MHz, DMSO-d6) δ 9.26 (s, 1H), 8.31 (dd, *J* = 20.2, 8.8 Hz, 2H), 8.01 (s, 1H), 7.96 (d, *J* = 7.8 Hz, 1H), 7.91 (d, *J* = 8.7 Hz, 1H), 7.67 (d, *J* = 7.7 Hz, 1H), 7.62 (s, 1H), 7.52 - 7.37 (m, 2H), 5.80 (s, 1H), 3.75 (p, *J* = 8.8 Hz, 1H), 2.75 (d, *J* = 4.8 Hz, 3H), 2.63 (s, 3H), 2.25 (q, *J* = 7.8 Hz, 1H), 1.44 (d, *J* = 9.6 Hz, 2H), -0.00 (p, *J* = 9.8 Hz, 1H).

### Example T36

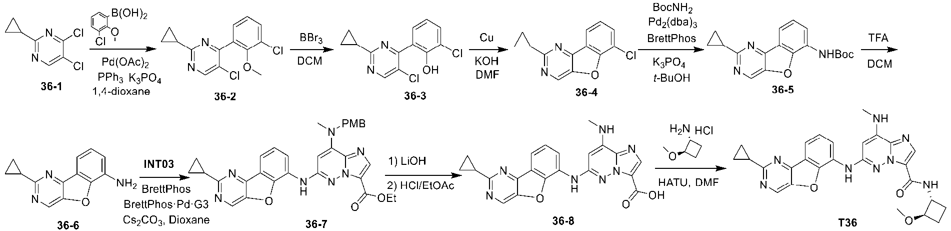

Step1: Dissolve **36-1** (274 mg, 1.45 mmol) in 1,4-dioxane (8 mL), then add 3-chloro-2-methoxyphenylboronic acid (270.17 mg, 1.45 mmol) and replace with N₂ five times. Under nitrogen protection, add K₃PO₄ (923.01 mg, 4.34 mmol), Pd(OAc)₂ (24.41 mg, 0.11 mmol), and PPh₃ (57.026 mg, 0.22 mmol). Replace the mixture with nitrogen three times, stir at 70°C overnight. Cool the reaction mixture, add water, and extract with DCM until the aqueous phase is free of product. Concentrate the organic phase under reduced pressure. Purify the crude product via silica gel chromatography (PE:EtOAc = 5:1) to obtain 36-2 (174 mg, yield 65.8%). MS m/z: 295 [M+H]⁺.

Step2: Similar protocol as that for **T22,** using **36-2.** MS m/z: 281 [M+H]⁺.

Step3: Similar protocol as that for **T26,** using **36-3.** MS m/z: 245 [M+H]⁺.

Step4: Similar protocol as that for **T16,** using **36-4.** MS m/z: 326 [M+H]⁺.

Step5: Similar protocol as that for **T16,** using **36-5.** MS m/z: 226 [M+H]⁺.

Step6: Similar protocol as that for **T16,** using **INT03** and **36-6.** MS m/z: 564 [M+H]⁺.

Step7 & Step8: Similar protocol as that for **T01,** using **36-7** to remove ethyl ester and PMB under the conditions of LiOH and HCl/EtOAc, respectively. MS m/z: 416 [M+H]⁺.

Step9: Similar protocol as that for **T01,** using **36-8.** MS m/z: 499 [M+H]⁺. ¹H NMR (400 MHz, DMSO-d6) δ 9.19 (s, 1H), 8.92 (s, 1H), 8.30 (d, *J* = 8.9 Hz, 1H), 7.84 (d, *J* = 7.8 Hz, 1H), 7.67 (d, *J* = 7.9 Hz, 1H), 7.57 (s, 1H), 7.37 (dd, *J* = 10.0, 6.1 Hz, 2H), 5.75 (s, 1H), 3.74 (q, *J* = 8.6 Hz, 1H), 2.69 (d, *J* = 4.9 Hz, 3H), 2.61 (s, 3H), 2.22 - 2.13 (m, 1H), 1.84 - 1.70 (m, 1H), 1.45 (p, *J* = 10.0 Hz, 2H), 1.20 (d, *J* = 15.9 Hz, 1H), 0.88 - 0.80 (m, 2H), 0.62 (t, *J* = 6.9 Hz, 1H), 0.02 (t, *J* = 9.9 Hz, 1H).

### Example T37

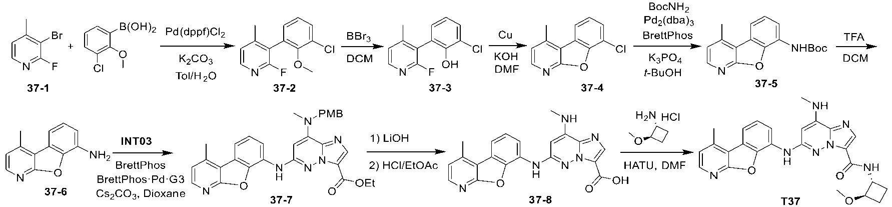

Step1: Dissolve **37-1** (200 mg, 1.05 mmol) in toluene (6 mL) and water (1 mL), then add 3-chloro-2-methoxyphenylboronic acid (215.82 mg, 1.16 mmol) and replace with N₂ five times. Under nitrogen protection, add K₂CO₃ (436.4 mg, 3.16 mmol) and Pd(dppf)Cl₂ (38.51 mg, 0.05 mmol). Replace the mixture with nitrogen three times, stir at 110 °C overnight. Cool the reaction mixture, concentrate under reduced pressure, and purify the crude product via silica gel chromatography (PE:EtOAc = 5:1) to obtain **37-2** (12.7 mg, yield 5%). MS m/z: 252 [M+H]⁺.

Step2: Similar protocol as that for **T22,** using **37-2.** MS m/z: 238 [M+H]⁺.

Step3: Similar protocol as that for **T26,** using **37-3.** MS m/z: 218 [M+H]⁺.

Step4: Similar protocol as that for **T16,** using **37-4.** MS m/z: 299 [M+H]⁺.

Step5: Similar protocol as that for **T16,** using **37-5.** MS m/z: 199 [M+H]⁺.

Step6: Similar protocol as that for **T16,** using **INT03** and **37-6.** MS m/z: 537 [M+H]⁺.

Step7 & Step8: Similar protocol as that for **T01,** using **37-7** to remove ethyl ester and PMB under the conditions of LiOH and HCl/EtOAc, respectively. MS m/z: 389 [M+H]⁺.

Step9: Similar protocol as that for **T01,** using **37-8.** MS m/z: 472 [M+H]⁺. ¹H NMR (400 MHz, DMSO-d6) δ 9.36 (s, 1H), 8.73 - 8.56 (m, 1H), 8.34 (d, *J* = 4.9 Hz, 1H), 8.02 (d, *J* = 7.7 Hz, 1H), 7.81 (s, 1H), 7.73 (d, *J* = 7.9 Hz, 1H), 7.65 - 7.47 (m, 2H), 7.37 (s, 1H), 6.01 (s, 1H), 4.09 - 3.89 (m, 1H), 2.93 (d, *J* = 4.6 Hz, 3H), 2.85 (s, 3H), 2.84 (s, 3H), 2.57 - 2.55 (m, **1H),** 1.66 **(d,** *J* = 9.5 Hz, 2H), 0.40 - 0.22 (m, 1H).

### Example T38

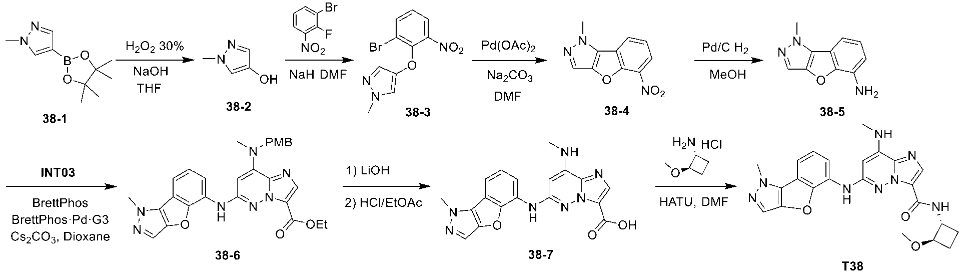

Step1: Dissolve **38-1** (1.0 g, 4.81 mmol) in THF and stir in an ice-water bath for 0.5 h. Add NaOH solution (3.2 mL, 3M), H₂O₂ (1.09 g, 9.6 mmol, 30%), and stir at 25 °C for 3 h. LCMS confirms complete reaction, and the reaction mixture is cooled to room temperature and then to 0 °C. Adjust the pH to 7~8 with concentrated HCl. Extract with a mixed solvent of DCM:MeOH (10:1), and concentrate the organic phase under reduced pressure to obtain **38-2** (382.3 mg, yield 81.1%). MS m/z: 99 [M+H]⁺.

Step2: Dissolve **38-2** (382.3 mg, 2.41 mmol) in DMF (5.0 mL) and stir in an ice-water bath for 20 minutes. Under nitrogen protection, add NaH (192.2 mg, 4.81 mmol) and stir in an ice-water bath for 0.5 hours. Dissolve 2-fluoro-3-bromonitrobenzene (793.0 mg, 3.61 mmol) in DMF and slowly add it dropwise, stirring at 25 °C for 20 minutes. Quench the reaction solution with aqueous solution, add EtOAc for extraction until no product is present in the aqueous phase. Concentrate the organic phase under reduced pressure, dissolve in DCM, and purify the crude product via silica gel chromatography (PE:EtOAc = 3:1) to obtain **38-3** (280.1 mg, yield 24.1%). MS m/z: 298 [M+H]⁺.

Step3: Dissolve **38-3** (100.0 mg, 0.22 mmol) and Na₂CO₃ (34.7 mg, 0.33 mmol) in DMF (2.5 mL), and replace the nitrogen atmosphere three times. Then, under nitrogen protection, add palladium acetate (4.9 mg, 0.022 mmol). Replace the nitrogen atmosphere with the mixture six times, and stir in an oil bath at 135 °C for 3 hours. LCMS confirms the reaction is complete. After the reaction mixture cools to room temperature, concentrate it under reduced pressure and dissolve it in DCM. Purify the crude product via silica gel chromatography (DCM) to obtain **38-4** (51.0 mg, yield 70%). MS m/z: 218 [M+H]⁺.

Step4: Dissolve **38-4** (51.0 mg, 0.23 mmol) in MeOH (4.0 mL), and then add palladium on carbon (5 mg). Replace the hydrogen in the mixture three times, and stir in an oil bath at 25 °C for 1.5 h. LCMS confirms the reaction is complete. Allow the reaction solution to cool to room temperature, filter, and then concentrate under reduced pressure to obtain **38-5** (40.0 mg, yield 91.1%). MS m/z: 188 [M+H]⁺.

Step5: Similar protocol as that for **T16,** using **INT03** and **38-5.** MS m/z: 526 [M+H]⁺.

Step6 & Step7: Similar protocol as that for **T01,** using **38-6** to remove ethyl ester and PMB under the conditions of LiOH and HCl/EtOAc, respectively. MS m/z: 378 [M+H]⁺.

Step8: Similar protocol as that for **T01,** using **38-7.** MS m/z: 461 [M+H]⁺. ¹H NMR (400 MHz, DMSO-d6) δ 9.22 (s, 1H), 8.65 (d, *J* = 9.1 Hz, 1H), 8.58 (s, 1H), 7.89 (d, *J* = 7.7 Hz, 1H), 7.79 (s, 1H), 7.59 - 7.52 (m, 2H), 7.43 (t, *J* = 7.9 Hz, 1H), 5.94 (s, 1H), 4.17 (s, 3H), 4.08 - 3.94 (m, 1H), 2.92 (d, *J* = 4.6 Hz, 3H), 2.90 (s, 3H), 2.63 (q, *J* = 7.7 Hz, 1H), 1.73 (dq, *J* = 19.6, 9.4 Hz, 2H), 0.42 - 0.32 (m, 1H).

### Example T39

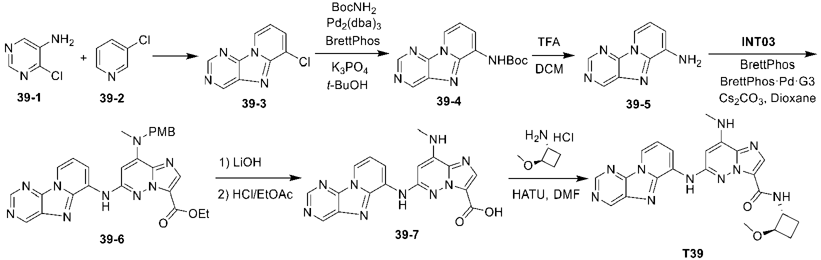

Step1: To a 15 mL Schlenk flask, **39-1** (100 mg, 0.77 mmol) was added, followed by three nitrogen purges. Under nitrogen protection, **39-2** (1 mL) was introduced into the reaction flask, and nitrogen purges were conducted three times. The mixture was stirred at 50 °C for 2 hours, sampled, and analyzed by LCMS. About 42% of the starting material remained unreacted. The temperature was raised to 80 °C, and the reaction was stirred for 5 hours. After sampling and LCMS analysis, it was found that about 6% of the starting material remained unreacted. The reaction mixture was concentrated under reduced pressure, and the obtained crude product was purified by prep-TLC (DCM:MeOH = 20:1) to yield **39-3** (21.5 mg, yield 13.6%). MS m/z: 205 [M+H]⁺.

Step2: Similar protocol as that for **T16,** using **39-3.** MS m/z: 286 [M+H]⁺.

Step3: Similar protocol as that for **T16,** using **39-4. MS** m/z: 186 [M+H]⁺.

Step4: Similar protocol as that for **T16,** using **INT03** and **39-5.** MS m/z: 524 [M+H]⁺.

Step5 & Step6: Similar protocol as that for **T01,** using **39-6** to remove ethyl ester and PMB under the conditions of LiOH and HCl/EtOAc, respectively. MS m/z: 376 [M+H]⁺.

Step7: Similar protocol as that for **T01,** using **39-7.** MS m/z: 459 [M+H]⁺. ¹H NMR (400 MHz, DMSO-d6) δ 9.56 (s, 1H), 9.49 (d, *J* = 8.0 Hz, 1H), 9.15 (s, 1H), 8.81 (d, *J* = 8.9 Hz, 1H), 8.68 (d, *J* = 6.7 Hz, 1H), 8.32 (s, 2H), 8.21 (d, *J* = 7.4 Hz, 1H), 7.89 (s, 1H), 7.62 (d, *J* = 5.4 Hz, 1H), 7.16 (t, *J* = 7.1 Hz, 1H), 6.48 (s, 1H), 4.41 - 4.17 (m, 1H), 3.51 (q, *J* = 7.7 Hz, 1H), 3.14 (s, 3H), 2.92 (d, *J* = 5.0 Hz, 3H), 2.02 (dt, *J* = 17.8, 9.4 Hz, 2H), 1.46 (dt, *J* = 21.9, 10.9 Hz, 1H).

### Example T40

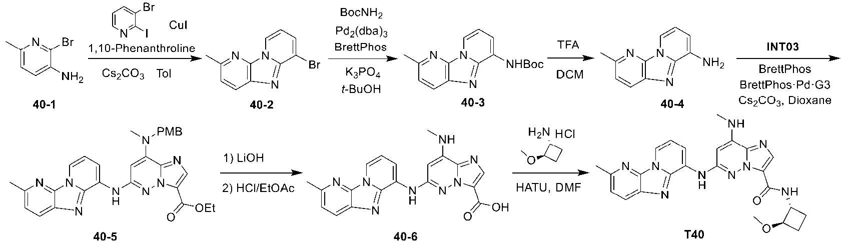

Step 1: Dissolve **40-1** (200 mg, 1.07 mmol) in toluene, add 3-bromo-4-iodopyridine (364 mg, 1.28 mmol), Cs₂CO₃ (1.05 g, 3.21 mmol), 1,10-phenanthroline (77 mg, 0.43 mmol), and replace nitrogen three times. Then add CuI (68 mg, 0.21 mmol), replace nitrogen three times, stir at 110 °C overnight. LCMS confirms complete reaction, allow the reaction mixture to cool to room temperature, filter and concentrate under reduced pressure, add DCM to dissolve, purify the crude product via silica gel chromatography (DCM:MeOH = 10:1) to obtain **40-2** (182 mg, yield 65%). MS m/z: 262 [M+H]⁺.

Step2: Similar protocol as that for **T16,** using **40-2.** MS m/z: 299 [M+H]⁺.

Step3: Similar protocol as that for **T16,** using **40-3.** MS m/z: 199 [M+H]⁺.

Step4: Similar protocol as that for **T16,** using **INT03** and **40-4.** MS m/z: 537 [M+H]⁺.

Step5 & Step6: Similar protocol as that for **T01,** using **40-5** to remove ethyl ester and PMB under the conditions of LiOH and HCl/EtOAc, respectively. MS m/z: 389 [M+H]⁺.

Step7: Similar protocol as that for **T01,** using **40-6.** MS m/z: 472 [M+H]⁺. ¹H NMR (400 MHz, DMSO-d6) δ 9.62 (s, 1H), 9.29 (bs, 1H), 9.28 (s, 1H), 8.94 (s, 1H), 8.67 (d, *J* = 4.4 Hz, 1H), 8.49 (dd, *J* = 22.2, 8.7 Hz, 2H), 8.18 (d, *J* = 8.6 Hz, 1H), 7.82 (s, 1H), 7.66 (d, *J* = 5.1 Hz, 1H), 7.52 (d, *J* = 8.6 Hz, 1H), 7.45 (dd, *J* = 8.5, 4.4 Hz, 1H), 6.02 (s, 1H), 3.99 (dd, *J* = 16.9, 8.3 Hz, 1H), 2.93 (d, *J* = 4.8 Hz, 3H), 2.86 (s, 3H), 2.68 (s, 3H), 2.62 (dt, *J* = 15.4, 7.6 Hz, 1H), 1.67 (p, *J* = 9.1, 8.7 Hz, 2H), 0.33 (p, *J* = 10.3 Hz, 1H).

### Example T41

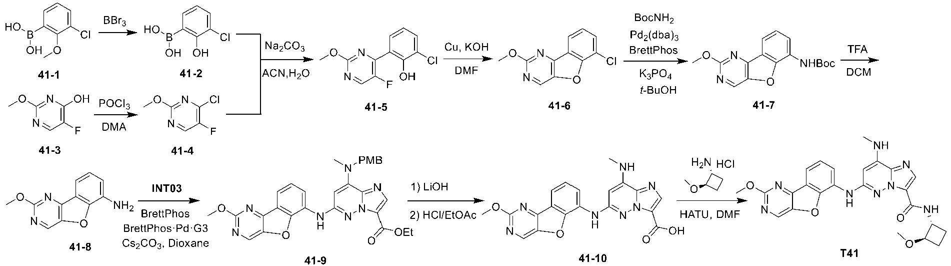

Step1: Dissolve **41-1** (500 mg, 2.69 mmol) in DCM (10 mL), then add BBr₃ (809 mg, 3.23 mmol). Stir the mixture at 25 °C for 3 hours. Quench the reaction by adding 2 mL of MeOH, and then concentrate the reaction solution under reduced pressure to obtain **41-2** (440 mg, yield 95%). MS m/z: 173 [M+H]⁺.

Step2: Dissolve **41-3** (3 g, 20.8 mmol) in DMA (10 mL), replace with nitrogen three times, cool down to 0 °C, add POCl₃ (10 mL) mixture, and stir at 25 °C for 3 h. Quench the reaction solution by adding it to ice-water, extract with EtOAc (50 mL × 3), wash with 2 N HCl, dry over anhydrous Na₂SO₄, and concentrate under reduced pressure to obtain **41-4** (1.3 g, yield 31%). MS m/z: 163 [M+H]⁺.

Step3: Dissolve **41-2** (440 mg, 2.55 mmol) in ACN (10 mL), add Na₂CO₃ (676 mg, 6.38 mmol), **41-4** (414 mg, 2.55 mmol) and water (5 mL). Replace with nitrogen three times, add Pd(PPh₃)₂Cl₂ (179 mg, 0.255 mmol) and replace with nitrogen three times, then stir the mixture at 75 °C for 5 h. Add 2 mL of water to quench the reaction, extract with EtOAc (15 mL × 3). Concentrate under reduced pressure, and purify the crude product via silica gel chromatography (PE:EtOAc = 50:1) to obtain **41-5** (420 mg, yield 65%). MS m/z: 255 [M+H]⁺.

Step4: Similar protocol as that for **T26,** using **41-5.** MS m/z: 235 [M+H]⁺.

Step5: Similar protocol as that for **T16,** using **41-6.** MS m/z: 316 [M+H]⁺.

Step6: Similar protocol as that for **T16,** using **41-7.** MS m/z: 216 [M+H]⁺.

Step7: Similar protocol as that for **T16,** using **INT03** and **41-8.** MS m/z: 554 [M+H]⁺.

Step8 & Step9: Similar protocol as that for **T01,** using **41-9** to remove ethyl ester and PMB under the conditions of LiOH and HCl/EtOAc, respectively. MS m/z: 406 [M+H]⁺.

Step 10: Similar protocol as that for **T01,** using **41-10.** MS m/z: 489 [M+H]⁺. ¹H NMR (400 MHz, DMSO-d6) δ 9.50 (s, 1H), 9.36 (bs, 1H), 8.58 (d, *J =* 9.0 Hz, 1H), 8.04 (d, *J =* 7.8 Hz, 1H), 7.93 (d, *J =* 7.7 Hz, 1H), 7.60 (dd, *J=* 10.7, 5.7 Hz, 2H), 6.01 (s, 1H), 4.03 (s, 3H), 3.98 (p, *J =* 8.3 Hz, 1H), 2.93 (d, *J =* 4.8 Hz, 3H), 2.88 (s, 3H), 2.62 (q, *J =* 7.7 Hz, 1H), 1.72 (t, *J=* 9.7 Hz, 2H), 0.33 (q, *J =* 9.9 Hz, 1H).

### Example T42

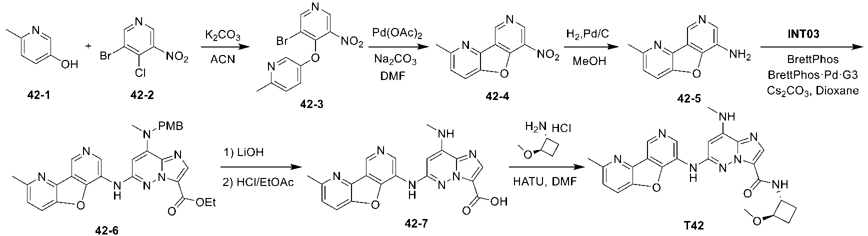

Step1: Dissolve **42-1** (460 mg, 4.21 mmol) in acetonitrile (5 mL), add K₂CO₃ (582 mg, 8.42 mmol) and **42-2** (1000 mg, 4.21 mmol), replace nitrogen three times, and allow the mixture to react at 50 °C for 2 hours. Concentrate the reaction solution under reduced pressure, and isolate and purify the crude product by prep-TLC (DCM:MeOH = 20:1) to obtain **42-3** (1171 mg, yield 90%). MS m/z: 310 [M+H]⁺.

Step2: Similar protocol as that for **T38,** using **42-3.** MS m/z: 230 [M+H]⁺.

Step3: Similar protocol as that for **T38,** using **42-4.** MS m/z: 200 [M+H]⁺.

Step4: Similar protocol as that for **T16,** using **INT03** and **42-5.** MS m/z: 538 [M+H]⁺.

Step5 & Step6: Similar protocol as that for **T01,** using **42-6** to remove ethyl ester and PMB under the conditions of LiOH and HCl/EtOAc, respectively. MS m/z: 390 [M+H]⁺.

Step7: Similar protocol as that for **T01,** using **42-7.** MS m/z: 473 [M+H]⁺. ¹H NMR (400 MHz, DMSO-d6) δ 9.62 (s, 1H), 9.28 (s, 1H), 8.94 (s, 1H), 8.67 (d, *J =* 4.4 Hz, 1H), 8.49 (dd, *J=* 22.2, 8.7 Hz, 2H), 8.18 (d, J = 8.6 Hz, 1H), 7.82 (s, 1H), 7.66 (d, *J* = 5.1 Hz, 1H), 7.52 (d, *J* = 8.6 Hz, 1H), 7.45 (dd, *J =* 8.5, 4.4 Hz, 1H), 6.02 (s, 1H), 3.99 (dd, *J =* 16.9, 8.3 Hz, 1H), 2.93 (d, *J =* 4.8 Hz, 3H), 2.86 (s, 3H), 2.68 (s, 3H), 2.62 (dt, *J =* 15.4, 7.6 Hz, 1H), 1.67 (p, *J =* 9.1*,* 8.7 Hz, 2H), 0.33 (p, *J =* 10.3 Hz, 1H).

### Example T43

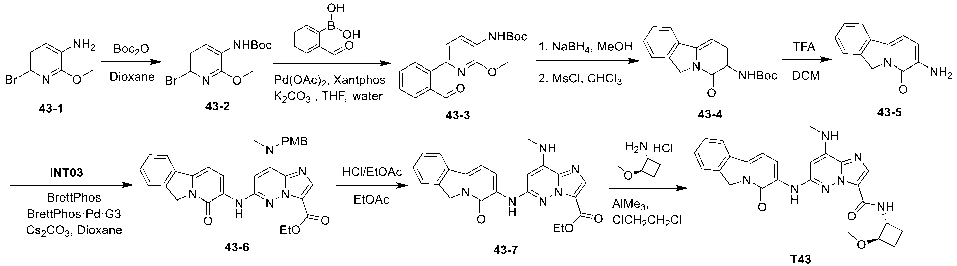

Step1: Dissolve **43-1** (1.0 g, 4.93 mmol) and Boc₂O (1.29 g, 5.91 mmol) in 1,4-dioxane (15 mL) and stir at 100 °C for 7 hours. Add additional Boc₂O (2.6 g, 11.93 mmol). After overnight reaction, TLC indicates the reaction is complete. Concentrate the reaction solution, add water, and extract with DCM. Concentrate the organic phase to obtain **43-2,** which is directly used for the next reaction step. MS m/z: 303 [M+H]⁺.

Step2: Dissolve **43-2** (0.50 g, 1.65 mmol), o-formylphenylboronic acid (0.247 g, 1.65 mmol), palladium acetate (0.011 g, 0.05 mmol), XantPhos (0.048 g, 0.09 mmol), and K₂CO₃ (0.456 g, 3.30 mmol) in THF (15 mL) and water (3 mL). Replace the atmosphere with nitrogen three times, and stir the mixture at 75 °C in an oil bath overnight. After concentrating the reaction solution, the crude product is purified using a prep-TLC (PE:EtOAc = 5:1) to obtain **43-3** (0.16 g, yield 30%). MS m/z: 329 [M+H]⁺.

Step3: Dissolve **43-3** (0.16 g, 0.61 mmol) in anhydrous MeOH (5 mL) and add NaBH₄ (0.038 g, 1.0 mmol). The mixture reacts at room temperature for 0.5 h. Quench the reaction with aqueous citric acid solution until neutral. After concentrating the reaction solution, extract it with DCM. After concentrating the organic phase, redissolve it with chloroform (5 mL), add di*iso*propylethylamine (0.2 mL), replace the atmosphere with nitrogen three times, and cool to 0 °C. Add methanesulfonyl chloride (0.1 mL). Stir the mixture at room temperature for 1 h, then heat it to 55 °C and react for half an hour. After concentrating the reaction solution, purify the crude product using a prep-TLC (DCM) to obtain **43-4** (0.15 g, yield 100%). MS m/z: 299 [M+H]⁺.

Step4: Similar protocol as that for **T16,** using **43-4.** MS m/z: 199 [M+H]⁺.

Step5: Similar protocol as that for **T16,** using **INT03** and **45-5.** MS m/z: 537 [M+H]⁺.

Step6: Similar protocol as that for **T01,** using **43-6** and HCl/EtOAc to remove PMB. MS m/z: 417 [M+H]⁺.

Step7: Dissolve (1R, 2R)-2-methoxycyclobutan-1-amine hydrochloride (13 mg, 95 µmol) in 1,2-dichloroethane (1.5 mL), and add 60 µL of a 1.6 M AlMe₃ solution in toluene. Stir the reaction mixture at room temperature for 1 hour. Add **43-7** (20 mg, 0.048 mmol) to the above reaction solution, and allow it to react at room temperature for 3 hours. Quench the reaction by adding 2 mL of water dropwise, and then extract with 2 mL of DCM:MeOH (10:1). Dry the organic phase and concentrate under reduced pressure. Purify the crude product using prep-TLC (DCM:MeOH = 20:1) to obtain **T43** (5 mg, yield 22%). MS m/z: 472 [M+H]⁺. ¹H NMR (400 MHz, DMSO-d6) δ 8.93 (d, *J =* 9.0 Hz, 1H), 8.67 (s, 1H), 8.21 (d*, J =* 7.7 Hz, 1H), 8.03 (d, *J =* 6.9 Hz, 1H), 7.86 (s, 1H), 7.70 (d, *J =* 6.9 Hz, 1H), 7.51 (td, *J =* 11.4, 9.8, 6.0 Hz, 3H), 7.06 (d, *J =* 7.6 Hz, 1H), 6.36 (s, 1H), 5.22 (s, 2H), 3.98 (p, *J =* 8.7 Hz, 1H), 3.16 (s, 3H), 2.88 (d, *J= 4.8* Hz, 3H), 2.67 (q, *J =* 7.7 Hz, 1H), 2.00 - 1.97 (m, 2H), 0.87- 0.84 (m, 1H).

### Example T44

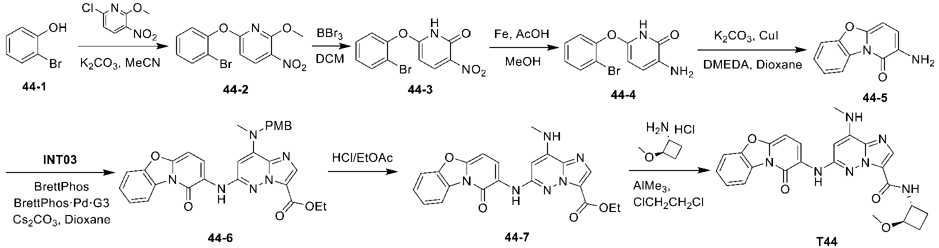

Step1: Similar protocol as that for **T42,** using **44-1.** MS m/z: 325 [M+H]⁺.

Step2: Similar protocol as that for **T22,** using **44-2.** MS m/z: 311 [M+H]⁺.

Step3: Dissolve **44-3** (190 mg, 0.61 mmol) in anhydrous MeOH (4 mL), add acetic acid (2 mL), replace with N₂ three times, add iron powder (170 mg, 3 mmol), stir the mixture at room temperature for 1 hour, filter with diatomaceous earth to obtain **44-4** (120 mg, yield 70%). HRMS m/z: 280.9916, 282.9895 [M+H]⁺.

Step4: Dissolve **44-4** (495 mg, 1.77 mmol), K₂CO₃ (489 mg, 3.54 mmol), and DMEDA (62.3 mg, 0.71 mmol) in 1,4-dioxane (15 mL). Replace the atmosphere with nitrogen three times, and then add cuprous iodide (67.3 mg, 0.35 mmol). Stir the mixture at 110 °C for 1 hour. Filter the reaction solution under reduced pressure, concentrate it in vacuo, and purify the crude product via silica gel column chromatography to obtain **44-5** (70 mg, yield 20%). HRMS m/z: 201.0657 [M+H]⁺.

Step5: Similar protocol as that for **T16,** using **44-5.** MS m/z: 539 [M+H]⁺.

Step6: Similar protocol as that for **T01,** using **44-6** and HCl/EtOAc to remove PMB. MS m/z: 419 [M+H]⁺.

Step7: Similar protocol as that for **T43,** using **44-7.** MS m/z: 474 [M+H]⁺. ¹H NMR (400 MHz, DMSO-d6) δ 8.96 (d, *J* = 9.2 Hz, 1H), 8.55 (s, 1H), 8.11 (d, *J* = 8.1 Hz, 1H), 8.08 (d, *J =* 8.3 Hz, 1H), 7.81 - 7.78 (m, 2H), 7.60 - 7.50 (m, 2H), 7.22 - 7.19 (m, 2H), 6.60 - 6.53 (m, 1H), 6. 01 (s, 1H), 4.22 (p, *J =* 8.7 Hz, 1H), 3.25 - 3.10 (m, 1H), 2.97 (s, 3H), 2.89 (d, *J =* 4.7 Hz, 3H), 2.08 - 2.00 (m, 2H), 0.86 - 0.83 (m, 1H).

### Example T45

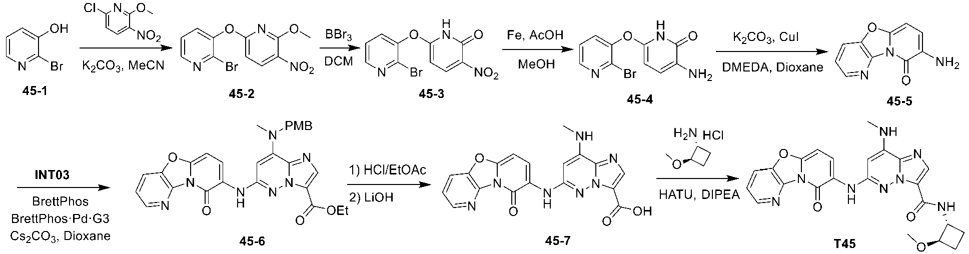

Step1: Similar protocol as that for **T42,** using **45-1.** MS m/z: 326 [M+H]⁺.

Step2: Similar protocol as that for **T22,** using **45-2.** MS m/z: 312 [M+H]⁺.

Step3: Similar protocol as that for **T44,** using **45-3.** HRMS m/z: 281.9894, 283.9874 [M+H]⁺.

Step4: Similar protocol as that for **T44,** using **45-4.** HRMS m/z: 202.0602 [M+H]⁺.

Step5: Similar protocol as that for **T16,** using **45-5.** HRMS m/z: 540.1978 [M+H]⁺.

Step6 & Step7: Similar protocol as that for **T01,** using **45-6** to remove ethyl ester and PMB under the conditions of LiOH and HCl/EtOAc, respectively. MS m/z: 392 [M+H]⁺.

Step8: Similar protocol as that for **T01,** using **45-7.** MS m/z: 475 [M+H]⁺. ¹H NMR (400 MHz, DMSO-d6) δ 8.96 (d, *J =* 9.2 Hz, 1H), 8.54 (s, 1H), 8.49 (s, 1H), 8.17 (d, *J =* 8.1 Hz, 1H), 8.08 (d, *J =* 8.3 Hz, 1H), 7.81 (s, 1H), 7.60 (s, 1H), 7.45 (s, 1H), 6.53 (d, *J =* 8.1 Hz, 1H), 6.10 (s, 1H), 4.22 (p, *J=* 8.7 Hz, 1H), 3.03 (s, 3H), 2.89 (d, *J =* 4.7 Hz, 3H), 2.60 - 2.50 (m, 1H), 2.00 (s, 2H), 0.86 - 0.83 (m, 1H).

### Example T46

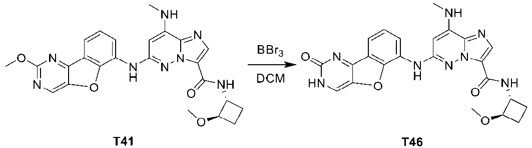

Step1: Dissolve **T41** (10 mg, 20.5 µmol) in DCM (1 mL), slowly add BBr₃ (20 mg, 82 µmol) dropwise under ice bath, and stir the mixture at room temperature for 1 hour. Adjust the pH of the reaction solution to 7 with NaHCO₃, and extract the aqueous phase with DCM. Combine the organic phases, dry over anhydrous Na₂SO₄, and concentrate under reduced pressure to obtain the crude product. Purify the crude product by prep-TLC (DCM:MeOH = 15:1) to obtain **T46** (7 mg, yield 72%). HRMS m/z: 475.1811 [M+H]⁺.

### Example T47

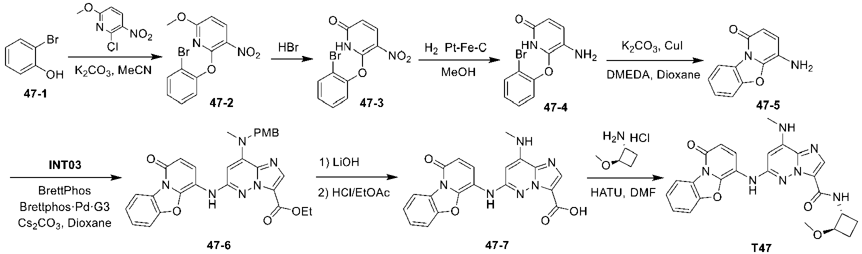

Step1: Similar protocol as that for **T42,** using **47-1.** MS m/z: 325.25, 327.25 [M+H]⁺.

Step2: Dissolve **47-2** (200 mg, 0.615 mmol) in HBr (33 wt% in AcOH) and let it react overnight at 50 °C. Quench the reaction by adding water, adjust the pH to 7 with sodium bicarbonate, and extract the aqueous phase with EtOAc. Combine the organic phases, wash with saturated saline solution, dry over anhydrous Na₂SO₄, and concentrate under reduced pressure to obtain **47-3** (135 mg, yield 71%). MS m/z: 311.25, 313.25 [M+H]⁺.

Step3: Dissolve **47-3** (130 mg, 0.42 mmol) in anhydrous MeOH (4 mL), add acetic acid (2 drops), perform nitrogen substitution three times, add Pt/Fe/C (13 mg), perform nitrogen substitution three times, and then perform hydrogen substitution three times. Stir the mixture at room temperature under a 1 atm hydrogen atmosphere for one hour. Filter and concentrate to obtain **47-4,** which is used for next step directly. HRMS m/z: 280.9905, 282.9883 [M+H]⁺.

Step4: Similar protocol as that for **T44,** using **47-4.** HRMS m/z: 201.0652 [M+H]⁺.

Step5: Similar protocol as that for **T16,** using **47-5.** MS m/z: 539.55 [M+H]⁺.

Step6 & Step7: Similar protocol as that for **T01,** using **47-6** to remove ethyl ester and PMB under the conditions of LiOH and HCl/EtOAc, respectively. MS m/z: 391 [M+H]⁺.

Step8: Similar protocol as that for **T01,** using **47-7.** HRMS m/z: 474.1874 [M+H]⁺.

### Example T48

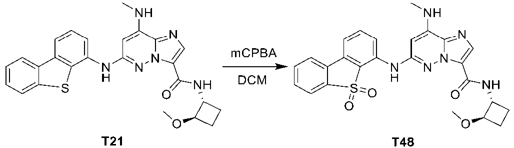

Step1: **T21** (5 mg, 10.6 µmol) was suspended in DCM (1 mL), and *m*CPBA (5.5 mg, 31.7 µmol) was added at room temperature. The mixture was stirred at room temperature for 16 hours. The crude reaction mixture was purified by prep-TLC (DCM:MeOH = 15:1) to obtain **T48** (2 mg, yield 37%). HRMS m/z: 505.1630 [M+H]⁺.

### Example T49

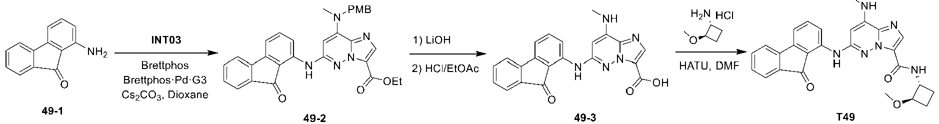

Step1: Similar protocol as that for **T16,** using **INT03** and **49-1.** HRMS m/z:534.2108 [M+H]⁺.

Step2 & Step3: Similar protocol as that for **T01,** using **49-2** to remove ethyl ester and PMB under the conditions of LiOH and HCl/EtOAc, respectively. MS m/z: 386 [M+H]⁺.

Step4: Similar protocol as that for **T01,** using **49-3.** MS m/z: 469 [M+H]⁺. ¹H NMR (600 MHz, DMSO-*d*₆) δ 9.33 (s, 1H), 8.75 (d, *J =* 7.8 Hz, 1H), 7.84 (s, 1H), 7.82 - 7.78 (m, 2H), 7.68- 7.64 (m, 1H), 7.58 - 7.53 (m, 3H), 7.42 (d, *J =* 7.8 Hz, 1H), 7.37 - 7.33 (m, 1H), 6.05 (s, 1H), 4.20 - 4.16 (m, 1H), 3.45 - 3.41 (m, 1H), 2.91 (d, *J =* 4.8 Hz, 3H), 2.86 (s, 3H), 1.98 - 1.81 (m, 2H), 1.12 - 1.03 (m, 1H).

### Example T50

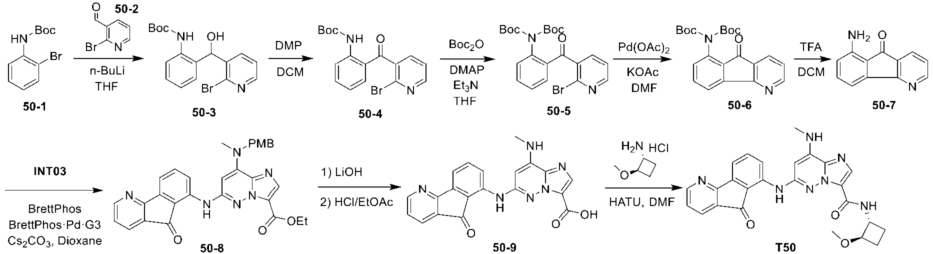

Step1: Dissolve **50-1** (1 g, 3.7 mmol) in THF (3 mL), cool to -78 °C under nitrogen protection, add *n-*BuLi (5.4 mL, 8.1 mmol), and stir for 0.5 h. Add **50-2** (350 mg, 2.25 mmol) at this temperature and allow the temperature to rise to room temperature. After the reaction is complete, add a saturated aqueous solution of ammonium chloride to quench the reaction. After slowly warming to room temperature, extract the mixture with EtOAc (30 mL × 3), combine the organic phases, wash with saturated saline solution, and dry over anhydrous Na₂SO₄. Filter and concentrate the organic phase under reduced pressure. Purify the crude product by prep-TLC (PE:EtOAc = 3:1) to obtain **50-3** (400 mg, yield 28%). MS m/z: 381.10 [M+H]⁺.

Step2: Dissolve **50-3** (450 mg, 1.18 mmol) in DCM (3 mL), cool to 0 °C under nitrogen protection, add Dess-Martin periodinane (1 g, 2.3 mmol), and gradually warm the mixture to room temperature while stirring for 0.5 h. After the reaction is complete, quench the reaction by adding a saturated Na₂SO₃ aqueous solution. Extract the mixture with EtOAc (30 mL × 3), combine the organic phases, wash with saturated saline solution, and dry over anhydrous Na₂SO₄. Filter and concentrate the organic phase under reduced pressure. Purify the crude product via prep-TLC (PE:EtOAc = 3:1) to obtain **50-4** (380 mg, yield 86%). MS m/z: 379.00 [M+H]⁺.

Step3: Dissolve **50-4** (300 mg, 0.8 mmol) in THF (5 mL), add triethylamine (304 mg, 3 mmol), Boc₂O (394 mg, 1.8 mmol), DMAP (88 mg, 0.72 mmol), and heat the mixture to 50 °C with stirring for 3 h. After the reaction is complete, quench the reaction by adding 10 mL of H₂O to the reaction solution. Extract the mixture with DCM (30 mL × 3), combine the organic phases, wash with saturated saline solution, and dry over anhydrous Na₂SO₄. Filter and concentrate the organic phase under reduced pressure. Purify the crude product by prep-TLC (PE:EtOAc = 3:1) to obtain **50-5** (300 mg, yield 79%). MS m/z: 479.05 [M+H]⁺.

Step4: Dissolve **50-5** (200 mg, 0.43 mmol) in DMF (3 mL), add potassium acetate (68 mg, 0.68 mmol) under nitrogen protection, replace the atmosphere with nitrogen three times, add palladium acetate (15 mg, 0.06 mmol), and heat the mixture to 85°C with stirring for 3 hours. After the reaction is complete, add 20 mL of H₂O to quench the reaction. Extract the mixture with EtOAc (30 mL × 3), combine the organic phases, wash with saturated saline solution, and dry over anhydrous Na₂SO₄. Filter and concentrate the organic phase under reduced pressure. Purify the crude product by prep-TLC (PE:EtOAc = 3:1) to obtain **50-6** (90 mg, yield 54%). MS m/z: 397.15 [M+H]⁺.

Step5: Similar protocol as that for **T16,** using **50-6.** MS m/z: 197.15 [M+H]⁺.

Step6: Similar protocol as that for **T16,** using **50-7.** MS m/z: 535.10 [M+H]⁺.

Step7 & Step8: Similar protocol as that for **T01,** using **50-8** to remove ethyl ester and PMB under the conditions of LiOH and HCl/EtOAc, respectively. MS m/z: 387 [M+H]⁺.

Step9: Similar protocol as that for **T01,** using **50-9.** MS m/z: 470 [M+H]⁺. ¹H NMR (400 MHz, DMSO-*d*₆) δ 9.33 (s, 1H), 8.70 - 8.60 (m, 2H), 7.98 -7.88 (m, 3H), 7.70 - 7.60 (m, 2H), 7.48 - 7.38 (m, 2H), 6.05 (s, 1H), 4.15 -4.10 (m, 1H), 3.02 (s, 3H), 2.87 (d, *J =* 4.8 Hz, 3H), 2.64 -2.60 (m, 1H), 1.88 - 1.84 (m, 2H), 0.77 - 0.74 (m, 1H).

### Example T51

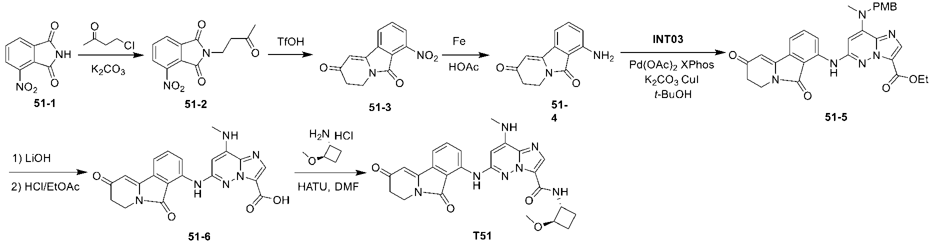

Step1: Dissolve 3-nitrophthalimide **51-1** (2.16 g, 11.3 mmol) in DMF (5 mL), add K₂CO₃ (1.43 g, 10 mmol), stir the reaction mixture at room temperature for 1 hour, and under nitrogen protection, add 4-chloro-2-butanone (1 g, 9.4 mmol) and sodium iodide (70.3 mg, 0.5 mmol). React the mixture at 40 °C. TLC indicates that the reaction is complete. The reaction mixture is concentrated by filtration under reduced pressure to obtain **51-2** (the crude product is directly used for the next step). MS m/z: 263.20 [M+H]⁺.

Step2: Add **51-2** (1.4 g, 5.3 mmol) into a 250 mL three-neck flask, using toluene (60 mL) as the solvent. Under nitrogen protection, add trifluoromethanesulfonic acid (1.6 g, 10.7 mmol). Stir the reaction mixture at 90 °C for 10 hours. The reaction mixture is concentrated by filtration under reduced pressure, and the crude product is purified by silica gel column chromatography (PE:EtOAc = 6:1) to obtain **51-3** (585 mg, 45%). MS m/z: 245.15 [M+H]⁺.

Step3: Similar protocol as that for **T44,** using **51-3.** MS m/z: 215.25 [M+H]⁺.

Step4: Add **51-4** (65 mg, 0.3 mmol), **INT03** (148 mg, 0.39 mmol), K₂CO₃ (105 mg, 0.76 mmol), and H₂O (1 mL) to *tert*-butanol. Under nitrogen protection, add XPhos (89.7 mg, 0.12 mmol), palladium acetate (13.6 mg, 0.06 mmol), and cuprous iodide (28.9 mg, 0.15 mmol). Stir the mixture at 110°C overnight. The reaction solution is concentrated by filtration under reduced pressure, and the crude product is purified by prep-TLC (PE:EtOAc=1:1) to obtain **51-5** (18 mg, 11%). MS m/z: 553.20 [M+H]⁺.

Step5 & Step6: Similar protocol as that for **T01,** using **51-5** to remove ethyl ester and PMB under the conditions of LiOH and HCl/EtOAc, respectively. MS m/z: 405 [M+H]⁺.

Step7: Similar protocol as that for **T01,** using **51-6.** MS m/z: 488 [M+H]⁺.

### Example T52

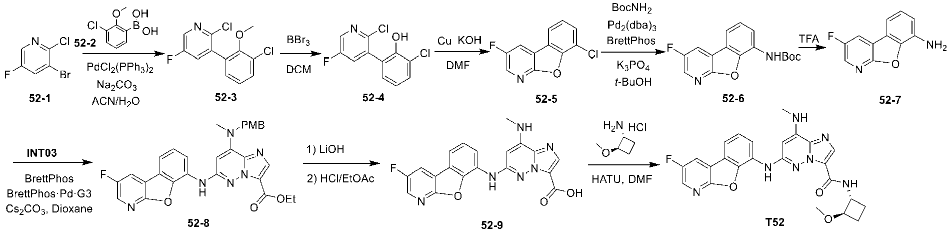

Step1: Dissolve **52-1** (300 mg, 1.43 mmol) in ACN (4 mL) and water (1 mL), add Na₂CO₃ (380 mg, 3.6 mmol), **52-2** (267 mg, 1.43 mmol), and replace with nitrogen three times. Add Pd(PPh₃)₂Cl₂ (106 mg, 0.15 mmol), heat the mixture to 75 °C and stir for 3 hours. After the reaction is complete, add 10 mL of H₂O to quench the reaction. Extract the mixture with EtOAc (30 mL × 3), combine the organic phases, wash with saturated saline solution, and dry over anhydrous Na₂SO₄. Filter and concentrate the organic phase under reduced pressure. Purify the crude product by prep-TLC (PE:EtOAc = 5:1) to obtain **52-3** (200 mg, yield 52%). MS m/z: 272.00 [M+H]⁺.

Step2: Similar protocol as that for **T22,** using **52-3.** MS m/z: 258 [M+H]⁺.

Step3: Similar protocol as that for **T26,** using **52-4.** MS m/z: 222 [M+H]⁺.

Step4: Similar protocol as that for **T16,** using **52-5.** MS m/z: 303.10 [M+H]⁺.

Step5: Similar protocol as that for **T16,** using **52-6.** MS m/z: 203.20 [M+H]⁺.

Step6: Similar protocol as that for **T16,** using **52-7.** MS m/z: 541.15 [M+H]⁺.

Step7 & Step8: Similar protocol as that for **T01,** using **52-8** to remove ethyl ester and PMB under the conditions of LiOH and HCl/EtOAc, respectively. MS m/z: 393 [M+H]⁺.

Step9: Similar protocol as that for **T01,** using **52-9.** MS m/z: 476 [M+H]⁺. ¹H NMR (400 MHz, DMSO-*d*₆) δ 9.34 (s, 1H), 8.71 (d, *J* = 7.8 Hz, 1H), 8.67 - 8.58 (m, 1H), 8.48 (s, 1H), 8.04 (d, *J* = 7.3 Hz, 1H), 7.79 - 7.75 (m, 2H), 7.59 - 5.53 (m, 2H), 5.99 (s, 1H), 3.98 (dd, *J=* 16.9, 8.3 Hz, 1H), 2.91 (d, *J =* 4.8 Hz, 3H), 2.86 (s, 3H), 2.63 - 2.59 (m, 1H), 1.79 - 1.58 (m, 2H), 0.34 - 0.30 (m, 1H).

### Example T53

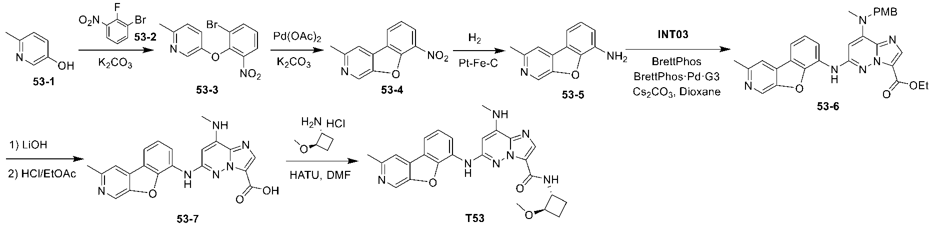

Step1: Similar protocol as that for **T42,** using **53-1.** MS m/z: 309, 311 [M+H]⁺.

Step2: Dissolve **53-3** (200 mg, 0.65 mmol) in DMF (3 mL), add K₂CO₃ (90 mg, 0.65 mmol) under nitrogen protection, replace nitrogen three times, add Pd(OAc)₂ (23 mg, 0.1 mmol), heat the mixture to 75 °C and stir for 3 hours. After the reaction is complete, add 10 mL of H₂O to quench the reaction. Extract the mixture with DCM (30 mL × 3), combine the organic phases, wash with saturated saline solution, and dry over anhydrous Na₂SO₄. Filter and concentrate the organic phase under reduced pressure. Purify the crude product by prep-TLC (PE:EtOAc = 2:1) to obtain **53-4** (34 mg, yield 23%). MS m/z: 229.15 [M+H]⁺.

Step3: Similar protocol as that for **T47,** using **53-4.** MS m/z: 199.40 [M+H]⁺.

Step4: Similar protocol as that for **T16,** using **53-5.** MS m/z: 537 [M+H]⁺.

Step5 & Step6: Similar protocol as that for **T01,** using **53-6** to remove ethyl ester and PMB under the conditions of LiOH and HCl/EtOAc, respectively. MS m/z: 389 [M+H]⁺.

Step7: Similar protocol as that for **T01,** using **53-7.** MS m/z: 472 [M+H]⁺. ¹H NMR (600 MHz, DMSO-*d*₆) δ 9.28 (s, 1H), 8.90 (s, 1H), 8.56 (s, 1H), 8.08 - 8.04 (m, 2H), 7.77 - 7.73 (m, 2H), 7.55 - 7.51 (m, 2H), 5.94 (s, 1H), 3.93 (dd, *J =* 16.9, 8.3 Hz, 1H), 2.89 (d, *J =* 4.8 Hz, 3H), 2.78 (s, 3H), 2.59 (s, 3H), 2.53 - 2.50 (m, 1H), 1.83 - 1.55 (m, 2H), 0.26 (p, *J =* 9.9 Hz, 1H).

### Example T54

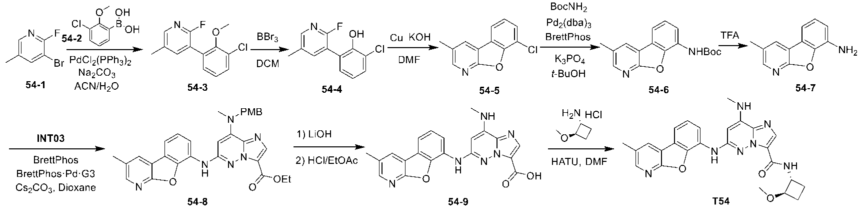

Step1: Similar protocol as that for **T52,** using **54-1.** MS m/z: 252.05 [M+H]⁺.

Step2: Similar protocol as that for **T22,** using **54-3.** MS m/z: 238.00 [M+H]⁺.

Step3: Similar protocol as that for **T26,** using **54-4.** MS m/z: 218.00 [M+H]⁺.

Step4: Similar protocol as that for **T16,** using **54-5.** MS m/z: 299.30 [M+H]⁺.

Step5: Similar protocol as that for **T16,** using **54-6.** MS m/z: 199.15 [M+H]⁺.

Step6: Similar protocol as that for **T16,** using **54-7.** MS m/z: 537.40 [M+H]⁺.

Step7 & Step8: Similar protocol as that for **T01,** using **54-8** to remove ethyl ester and PMB under the conditions of LiOH and HCl/EtOAc, respectively. MS m/z: 389 [M+H]⁺.

Step9: Similar protocol as that for T01, using **54-9.** MS m/z: 472 [M+H]⁺. ¹H NMR (400 MHz, DMSO-*d*₆) δ 9.28 (s, 1H), 8.64 (d, *J =* 7.8 Hz, 1H), 8.49 (s, 1H), 8.28 (s, 1H), 7.99 (d, *J =* 7.8 Hz, 1H), 7.78 (s, 1H), 7.68 (d, *J =* 7.8 Hz, 1H), 7.56 - 7.52 (m, 1H), 7.50 - 7.46 (m, 1H), 5.98 (s, 1H), 3.96 (dd, *J =* 16.9, 8.3 Hz, 1H), 2.91 (d, *J =* 4.8 Hz, 3H), 2.83 (s, 3H), 2.58 - 2.54 (m, 1H), 2.45 (s, 3H), 1.72 - 1.59 (m, 2H), 0.38 - 0.22 (m, 1H).

### Example T55

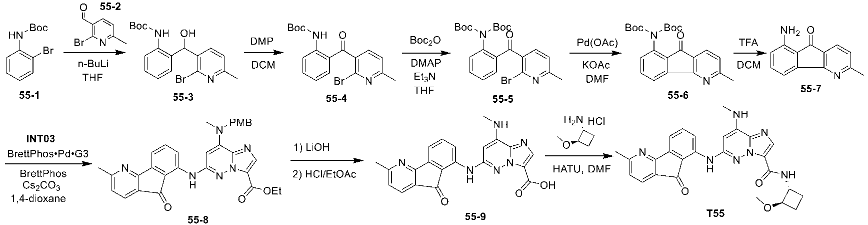

Step1: Similar protocol as that for **T50,** using **55-1.** MS m/z: 393 [M+H]⁺.

Step2: Similar protocol as that for **T50,** using **55-3.** MS m/z: 391 [M+H]⁺.

Step3: Similar protocol as that for **T50,** using **55-4.** MS m/z: 491 [M+H]⁺.

Step4: Similar protocol as that for **T50,** using **55-5.** MS m/z: 411.10 [M+H]⁺.

Step5: Similar protocol as that for **T16,** using **55-6.** MS m/z: 211.15 [M+H]⁺.

Step6: Similar protocol as that for **T16,** using **55-7.** MS m/z: 549.15 [M+H]⁺.

Step7 & Step8: Similar protocol as that for **T01,** using **55-8** to remove ethyl ester and PMB under the conditions of LiOH and HCl/EtOAc, respectively. MS m/z: 401 [M+H]⁺.

Step9: Similar protocol as that for **T01,** using **55-9.** MS m/z: 484 [M+H]⁺. ¹H NMR (400 MHz, DMSO-*d*₆) δ 9.32 (s, 1H), 8.72 (d, *J =* 7.8 Hz, 1H), 8.26 (s, 1H), 7.92 (d, *J =* 7.8 Hz, 1H), 7.83 (d, *J =* 7.8 Hz, 1H), 7.69 - 7.56 (m, 2H), 7.42 (d, *J* = 4.4 Hz, 1H), 7.21 (d, *J* = 7.4 Hz, 1H), 6.07 (s, 1H), 4.18 - 4.14 (m, 1H), 3.50 - 3.34 (m, 1H), 3.04 (s, 3H), 2.89 (d, *J =* 4.8 Hz, 3H), 2.54 (s, 3H), 1.92 - 1.88 (m, 2H), 1.12 - 1.08 (m, 1H).

### Example T56

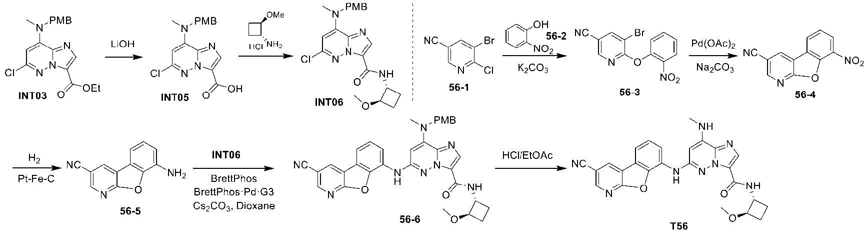

Step1: **INT03** (200 mg, 0.53 mmol) was dissolved in THF (6 mL) and MeOH (2 mL). LiOH (51 mg, 2.14 mmol) was added to H₂O (2 mL), and the mixture was stirred at room temperature. The reaction was monitored by TLC until complete, and the reaction solution was concentrated to remove MeOH and THF. The remaining phase was adjusted to pH 4-5 with 1 N HCl solution, and the aqueous phase was extracted with DCM (15 mL × 3). The organic phase was washed with saturated saline solution, dried over anhydrous Na₂SO₄, and concentrated under vacuum to obtain **INT05** (the crude product was directly used in the next step). MS m/z: 347.20 [M+H]⁺.

Step2: **INT05** (200 mg, 0.58 mmol) and 1-hydroxybenzotriazole (220 mg, 1.16 mmol) were dissolved in DMF (10 mL). Ten minutes later, N,N-diisopropylethylamine (112 mg, 1.73 mmol) was added. Half an hour later, a DMF solution of (1R,2R)-2-methoxycyclobutanamine hydrochloride (40 mg, 0.58 mmol) was added, and the mixture was stirred at room temperature. The reaction was monitored by TLC until complete, and the reaction solution was concentrated. The crude product was purified by prep-TLC to obtain **INT06** (196 mg, 79%). MS m/z: 430.25 [M+H]⁺.

Step3: Similar protocol as that for **T42,** using **56-2.** MS m/z: 320 [M+H]⁺.

Step4: Similar protocol as that for **T38,** using **56-3.** MS m/z: 240.15 [M+H]⁺.

Step5: Similar protocol as that for **T47,** using **56-4.** MS m/z: 210.25 [M+H]⁺.

Step6: Dissolve **56-5** (40 mg, 0.19 mmol) in dioxane (3 mL), add Cs₂CO₃ (66 mg, 0.2 mmol), Brettphos (12 mg, 0.02 mmol), **INT06** (90 mg, 0.2 mmol), and replace the atmosphere with nitrogen three times. Add Brettphos G3 Pd (10 mg, 0.01 mmol), heat the mixture to 75 °C, and stir for 3 hours. After the reaction is complete, add 10 mL of H₂O to quench the reaction. Extract the mixture with EtOAc (30 mL × 3), combine the organic phases, wash with saturated saline solution, and dry over anhydrous Na₂SO₄. Filter and concentrate the organic phase under reduced pressure. Purify the crude product by prep-TLC (DCM:MeOH = 20:1) to obtain **56-6** (55 mg, yield 48%). MS m/z: 603.25 [M+H]⁺.

Step7: Dissolve **56-6** (16 mg, 0.027 mmol) in 2M HCl/EtOAc (4 mL), and let the mixture stand at room temperature for 4.3 hours. After the reaction is complete, evaporate the reaction solution under reduced pressure and add 10 mL of EtOAc. Repeat the evaporation process three times. Purify the crude product via prep-TLC (DCM:MeOH = 17:1) to obtain **T56** (5.8 mg, yield 45%). MS m/z: 483 [M+H]⁺. ¹H NMR (400 MHz, DMSO-*d*₆) δ 9.43 (s, 1H), 9.30 (d, *J =* 4.4 Hz, 1H), 8.97 (d, *J =* 4.4 Hz, 1H), 8.65 (d, *J =* 7.8 Hz, 1H), 8.06 (d, *J =* 8.6 Hz, 1H), 7.87 (d, *J =* 8.6 Hz, 1H), 7.80 (s, 1H), 7.61 - 7.57 (m, 2H), 6.02 (s, 1H), 4.00 (dd, *J* = 16.9, 8.3 Hz, 1H), 2.91 (d, *J =* 4.8 Hz, 3H), 2.88 (s, 3H), 2.70 - 2.66 (m, 1H), 1.75 - 1.71 (m, 2H), 0.51 - 0.33 (m, 1H).

### Example T57

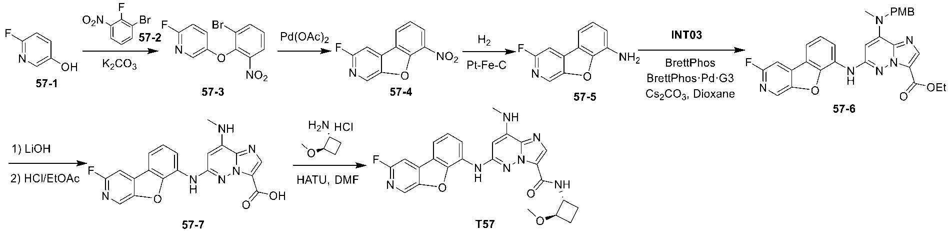

Step1: Similar protocol as that for **T42,** using **57-1.** MS m/z: 314.90 [M+H]⁺.

Step2: Similar protocol as that for **T53,** using **57-3.** MS m/z: 233.05 [M+H]⁺.

Step3: Similar protocol as that for **T47,** using **57-4.** MS m/z: 203 [M+H]⁺.

Step4: Similar protocol as that for **T16,** using **57-5.** MS m/z: 541.25 [M+H]⁺.

Step5 & Step6: Similar protocol as that for **T01,** using **57-6** to remove ethyl ester and PMB under the conditions of LiOH and HCl/EtOAc, respectively. MS m/z: 393 [M+H]⁺.

Step7: Similar protocol as that for **T01,** using **57-7.** MS m/z: 476 [M+H]⁺. ¹H NMR (400 MHz, DMSO-*d*₆) δ 9.35 (s, 1H), 8.71 (s, 1H), 8.60 - 8.56 (m, 1H), 8.13 - 8.10 (m, 2H), 8.08 (s, 1H), 7.80 - 7.77 (m, 1H), 7.77 (s, 1H), 7.58 -7.53 (m, 1H), 5.95 (s, 1H), 3.97 - 3.85 (m, 1H), 2.87 (s, 3H), 2.79 (d, *J =* 4.8 Hz, 3H), 2.55 - 2.51 (m, 1H), 1.60 - 1.56 (m, 2H), 0.26 - 0.20 (m, 1H).

### Example T58

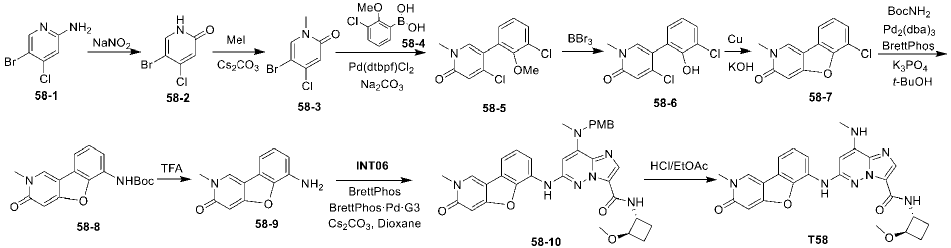

Step1: Dissolve **58-1** (200 mg, 0.97 mmol) in concentrated sulfuric acid (2 mL) and water (1 mL), add NaNO₂ (217 mg, 3.18 mmol), and stir the mixture at room temperature for 3 hours. After the reaction is complete, add 10 mL of ammonia water to the reaction solution to quench the reaction. Wash the mixture with water (10 mL × 3), filter under reduced pressure, retain the filter cake as the crude product, and purify it via prep-TLC (PE:EtOAc = 1:1) to obtain **58-2** (190 mg, yield 96%). MS m/z: 208 [M+H]⁺.

Step2: Dissolve **58-2** (190 mg, 0.91 mmol) in DMF (3 mL), add Cs₂CO₃ (163 mg, 0.5 mmol), cool to 0 °C, add iodomethane (71 mg, 0.5 mmol), and gradually heat the mixture to room temperature while stirring for 0.5 h. After the reaction is complete, quench the reaction by adding 10 mL of H₂O to the reaction mixture. Extract the mixture with EtOAc (30 mL × 3), combine the organic phases, wash with saturated saline solution, and dry over anhydrous Na₂SO₄. Filter and concentrate the organic phase under reduced pressure. Purify the crude product by prep-TLC (PE:EtOAc = 1:1) to obtain **58-3** (160 mg, yield 80%). MS m/z: 222 [M+H]⁺.

Step3: Dissolve **58-3** (200 mg, 0.7 mmol) in ACN (4 mL) and water (1 mL), add Na₂CO₃ (190 mg, 1.8 mmol), 58-4 (267 mg, 1.43 mmol), and replace the atmosphere with nitrogen three times. Add Pd(dtbpf)Cl₂ (66 mg, 0.1 mmol), heat the mixture to 75 °C, and stir for 3 hours. After the reaction is complete, add 10 mL of H₂O to quench the reaction. Extract the mixture with EtOAc (30 mL × 3), combine the organic phases, wash with saturated saline solution, and dry over anhydrous Na₂SO₄. Filter and concentrate the organic phase under reduced pressure. Purify the crude product by prep-TLC (PE:EtOAc = 1:1) to obtain **58-5** (100 mg, yield 40%). MS m/z: 285.95 [M+H]⁺.

Step4: Similar protocol as that for **T22,** using **58-5.** MS m/z: 270 [M+H]⁺.

Step5: Similar protocol as that for **T26,** using **58-6.** MS m/z: 234 [M+H]⁺.

Step6: Similar protocol as that for **T16,** using **58-7.** MS m/z: 315.10 [M+H]⁺.

Step7: Similar protocol as that for **T16,** using **58-8.** MS m/z: 215 [M+H]⁺.

Step8: Similar protocol as that for **T56,** using **58-9.** MS m/z: 608.15 [M+H]⁺.

Step9: Similar protocol as that for **T56,** using **58-10.** MS m/z: 488 [M+H]⁺. ¹H NMR (400 MHz, DMSO-*d*₆) δ 9.12 (s, 1H), 8.78 (s, 1H), 8.71 (d, *J =* 7.8 Hz, 1H), 7.77 (s, 1H), 7.67 (d, *J =* 7.8 Hz, 1H), 7.52 (s, 1H), 7.50 (s, 1H), 7.34 (d, *J =* 7.8 Hz, 1H), 6.40 (s, 1H), 5.90 (s, 1H), 4.06 - 4.00 (m, 1H), 3.54 (s, 3H), 2.91 (d, *J* = 4.8 Hz, 3H), 2.87 (s, 3H), 1.79 - 1.75 (m, 2H), 1.31 - 1.27 (m, 1H), 0.68 - 0.46 (m, 1H).

### Example T59

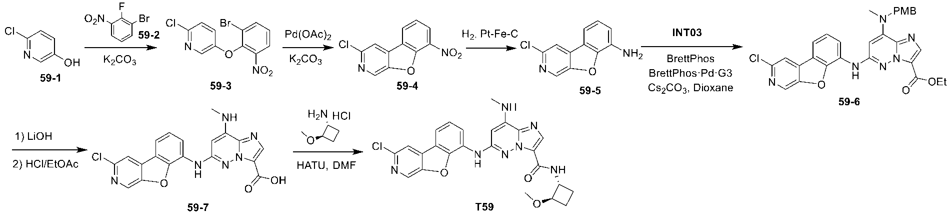

Step1: Similar protocol as that for **T42,** using **59-1.** MS m/z: 328.90, 330.90 [M+H]⁺.

Step2: Similar protocol as that for **T53,** using **59-3.** MS m/z: 249.35 [M+H]⁺.

Step3: Similar protocol as that for **T47,** using **59-4.** MS m/z: 219 [M+H]⁺.

Step4: Similar protocol as that for **T16,** using **59-5.** MS m/z: 557.10 [M+H]⁺.

Step5 & Step6: Similar protocol as that for **T01,** using **59-6** to remove ethyl ester and PMB under the conditions of LiOH and HCl/EtOAc, respectively. MS m/z: 409 [M+H]⁺.

Step7: Similar protocol as that for **T01,** using **59-7.** MS m/z: 492 [M+H]⁺. ¹H NMR (600 MHz, DMSO-*d*₆) δ 9.14 (s, 1H), 8.69 (s, 1H), 8.32 (d*, J =* 7.8 Hz, 1H), 8.24 (s, 1H), 8.06 (s, 1H), 7.93 (d, *J =* 7.8 Hz, 1H), 7.59 (d, *J* = 7.8 Hz, 1H), 7.55 (s, 1H), 7.35 - 7.31 (m, 1H), 5.73 (s, 1H), 3.72 - 3.66 (m, 1H), 2.68 (d, *J* = 4.8 Hz, 3H), 2.60 (s, 3H), 2.27 - 2.23 (m, 1H), 1.46 - 1.42 (m, 2H), 0.03 - 0.00 (m, 1H).

### Example T60

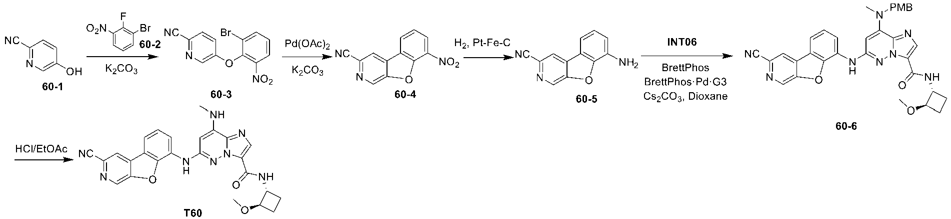

Step1: Similar protocol as that for **T42,** using **60-1.** MS m/z: 321.90 [M+H]⁺.

Step2: Similar protocol as that for **T53,** using **60-3.** MS m/z: 240.05 [M+H]⁺.

Step3: Similar protocol as that for **T47,** using **60-4.** MS m/z: 210.15 [M+H]⁺.

Step4: Similar protocol as that for **T56,** using **60-5.** MS m/z: 603.30 [M+H]⁺.

Step5: Similar protocol as that for **T56,** using **60-6.** MS m/z: 483 [M+H]⁺. ¹H NMR (400 MHz, DMSO-*d*₆) δ 9.42 (s, 1H), 9.24 (s, 1H), 9.04 (s, 1H), 8.52 (d, *J =* 7.8 Hz, 1H), 8.13 (d, *J =* 7.8 Hz, 1H), 7.88 (d, *J =* 7.8 Hz, 1H), 7.75 (s, 1H), 7.60 - 7.56 (m, 2H), 5.94 (s, 1H), 3.96 -3.90 (m, 1H), 2.87 (s, 3H), 2.80 (d, *J =* 4.8 Hz, 3H), 2.55 - 2.51 (m, 1H), 1.75 - 1.56 (m, 2H), 0.28 - 0.24 (m, 1H).

### Example T61

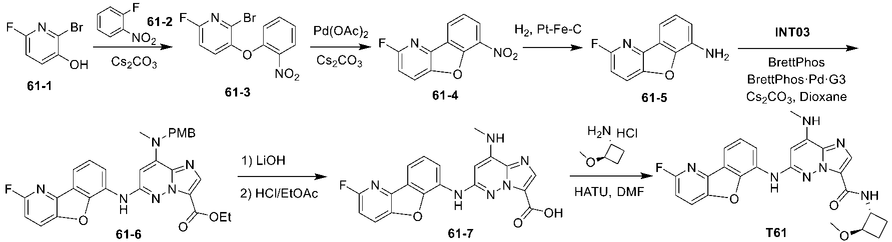

Step1: **61-1** (100 mg, 0.52 mmol) was dissolved in ACN (3 mL), followed by the addition of Cs₂CO₃ (172 mg, 0.52 mmol) and **61-2** (142 mg, 1.5 mmol). The mixture was heated to 75 °C and stirred for 6 hours. After the reaction was complete, 10 mL of H₂O was added to quench the reaction. The mixture was extracted with EtOAc (30 mL × 3), and the organic phases were combined, washed with saturated saline solution, and dried over anhydrous Na₂SO₄. The organic phases were filtered and concentrated under reduced pressure. The crude product was purified by prep-TLC (PE:EtOAc = 3:1) to obtain **61-3** (90 mg, yield 55%). MS m/z: 313.10, 315.10 [M+H]⁺.

Step2: **61-3** (90 mg, 0.3 mmol) was dissolved in DMF (3 mL), and Cs2CO3 (147 mg, 0.45 mmol) was added under nitrogen protection. The mixture was purged with nitrogen three times, followed by the addition of Pd(OAc)₂ (23 mg, 0.1 mmol). The mixture was heated to 75 °C and stirred for 3 hours. After the reaction was complete, 10 mL of H₂O was added to quench the reaction. The mixture was extracted with DCM (30 mL × 3), and the organic phases were combined, washed with saturated saline solution, and dried over anhydrous Na₂SO₄. The organic phases were filtered and concentrated under reduced pressure. The crude product was purified by prep-TLC (PE:EtOAc = 3:1) to obtain **61-4** (35 mg, yield 50%). MS m/z: 233.25 [M+H]⁺.

Step3: Similar protocol as that for **T47,** using **61-4.** MS m/z: 203.25 [M+H]⁺.

Step4: Similar protocol as that for **T16,** using **61-5.** MS m/z: 541.25 [M+H]⁺.

Step5 & Step6: Similar protocol as that for **T01,** using **61-6** to remove ethyl ester and PMB under the conditions of LiOH and HCl/EtOAc, respectively. MS m/z: 393 [M+H]⁺.

Step7: Similar protocol as that for **T01,** using **61-7.** MS m/z: 476 [M+H]⁺. ¹H NMR (400 MHz, DMSO-*d*₆) δ 9.34 (s, 1H), 8.55 (d, *J =* 7.8 Hz, 1H), 8.39 (t, *J =* 5.8 Hz, 1H), 7.97 (d, *J =* 7.8 Hz, 1H), 7.78 - 7.74 (m, 2H), 7.57 - 7.53 (m, 2H), 7.36 - 7.32 (m, 1H), 5.95 (s, 1H), 3.98 - 3.90 (m, 1H), 3.19 - 3.15 (m, 1H), 2.90 (d, *J =* 4.8 Hz, 3H), 2.82 (s, 3H), 1.65 - 1.61 (m, 2H), 0.23 - 0.21 (m, 1H).

### Example T62

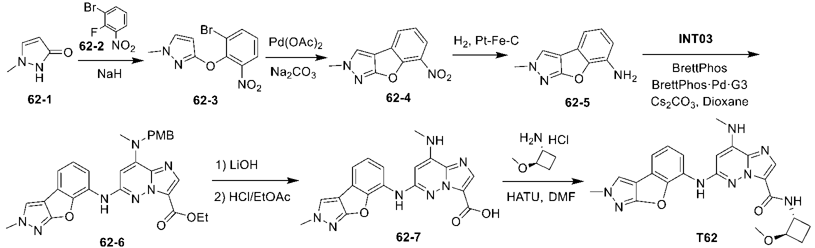

Step1: Similar protocol as that for **T38,** using **62-1.** MS m/z: 298.15 [M+H]⁺.

Step2: Similar protocol as that for **T38,** using **62-3.** MS m/z: 218.25 [M+H]⁺.

Step3: Similar protocol as that for **T47,** using **62-4.** MS m/z: 188.30 [M+H]⁺.

Step4: Similar protocol as that for **T16,** using **62-5.** MS m/z: 526.30 [M+H]⁺.

Step5 & Step6: Similar protocol as that for **T01,** using **62-6** to remove ethyl ester and PMB under the conditions of LiOH and HCl/EtOAc, respectively. MS m/z: 378 [M+H]⁺.

Step7: Similar protocol as that for **T01,** using **62-7.** MS m/z: 461 [M+H]⁺.

### Example T63

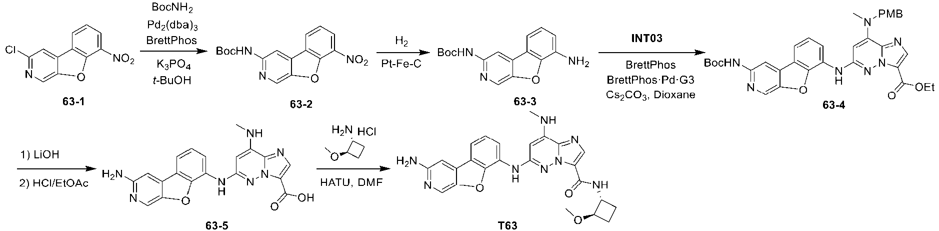

Step1: Similar protocol as that for **T16,** using **63-1** (same as **59-4).** MS m/z: 330.25 [M+H]⁺.

Step2: Similar protocol as that for **T47,** using **63-2.** MS m/z: 300.25 [M+H]⁺.

Step3: Similar protocol as that for **T16,** using **63-3.** MS m/z: 638.30 [M+H]⁺.

Step4 & Step5: Similar protocol as that for **T01,** using **63-4** to remove ethyl ester and PMB under the conditions of LiOH and HCl/EtOAc, respectively. MS m/z: 390 [M+H]⁺.

Step6: Similar protocol as that for **T01,** using **63-5.** MS m/z: 473 [M+H]⁺. ¹H NMR (600 MHz, DMSO-*d*₆) δ 9.22 (s, 1H), 8.58 (d, *J =* 7.8 Hz, 1H), 8.40 (s, 1H), 8.30 (s, 2H), 7.98 (d, *J =* 7.8 Hz, 1H), 7.74 (s, 1H), 7.70 (s, 1H), 7.50 (s, 1H), 7.40 (m, 1H), 7.18 - 7.12 (m, 1), 5.90 (s, 1H), 3.92 - 3.86 (m, 1H), 2.86 (d*, J =* 4.8 Hz, 3H), 2.82 (s, 3H), 2.60 - 2.50 (m, 1H), 1.96 - 1.90 (m, 2H), 0.30 - 0.25 (m, 1H).

### Example T64

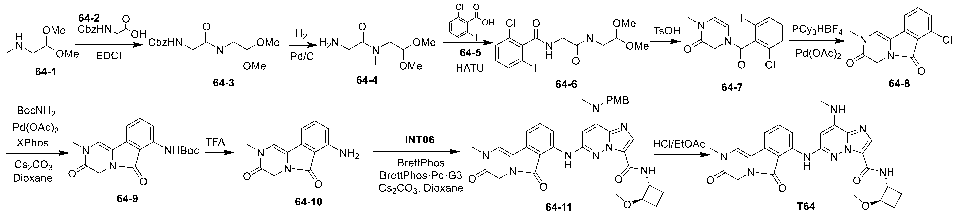

Step1: Dissolve **64-1** (500 mg, 4.2 mmol) and **64-2** (878 mg, 4.2 mmol) in DMF (10 mL). Add 1-hydroxybenzotriazole (62.5 mg, 0.46 mmol), EDCI (652 mg, 4.2 mmol), and N,N-di*is*opropylethylamine (81.4 mg, 0.63 mmol). Stir the mixture at room temperature. Monitor the reaction completion by TLC. Add water (10 mL) to the mixture. Extract the aqueous phase with DCM (15 mL × 3). Wash the organic phase with saturated saline solution, dry over anhydrous Na₂SO₄, and concentrate to obtain crude **64-3,** which is used for next step directly. MS m/z: 311.20 [M+H]⁺.

Step2: Dissolve crude **64-3** in MeOH, add 10% Pd/C, and stir the reaction mixture under a hydrogen atmosphere at room temperature for 2 hours. The reaction mixture is filtered under reduced pressure and concentrated to obtain **64-4,** which is used for next step directly. MS m/z: 177.30 [M+H]⁺.

Step3: Add **64-5** (500 mg, 1.77 mmol) and HATU (808 mg, 2.12 mmol) to DMF (5 mL), stir at room temperature for half an hour, add N,N-di*iso*propylethylamine (458 mg, 3.54 mmol) and **64-4** (312, 1.94 mmol), stir the mixture at room temperature overnight. Add water to the mixture, extract the aqueous phase with EtOAc (15 mL × 3), combine the organic phases, dry over anhydrous Na₂SO₄, concentrate, and purify the crude by prep-TLC to obtain **64-6** (673 mg, 86.4%). MS m/z: 440.90 [M+H]⁺.

Step4: Add **64-6** (311 mg, 0.71 mmol) and *p*-toluenesulfonic acid monohydrate (31 mg, 0.16 mmol) to toluene (10 mL). Stir the reaction mixture at 80 °C for 3 hours. Monitor the reaction completion by TLC. Cool the reaction mixture to room temperature and concentrate. Purify the crude product by prep-TLC to obtain **64-7** (266 mg, 82%). MS m/z: 376.90 [M+H]⁺.

Step5: Under nitrogen protection, **64-7** (170 mg, 0.45 mmol), Cs₂CO₃ (368 mg, 1.13 mmol), PCy₃HBF₄ (133 mg, 0.36 mmol), NaI (13.6 mg, 0.09 mmol), and palladium acetate (40.6 mg, 0.18 mmol) were added to toluene (5 mL), and the mixture was stirred at 110 °C overnight. The reaction solution was cooled to room temperature. It was filtered through diatomaceous earth, and the filtrate was concentrated. The crude product was purified by prep-TLC (PE:EtOAc = 2:1) to obtain **64-8** (99 mg, 88%). MS m/z: 249.15 [M+H]⁺.

Step6: Under nitrogen protection, **64-8** (99 mg, 0.4 mmol), Cs₂CO₃ (260 mg, 0.8 mmol), tert-butyl carbamate (93.5 mg, 0.8 mmol), XPhos (59 mg, 0.08 mmol), and palladium acetate (9 mg, 0.04 mmol) were added to dioxane (5 mL). The reaction mixture was stirred at 100 °C overnight. The reaction mixture was cooled to room temperature. It was filtered through diatomaceous earth, and the filtrate was concentrated. The crude product was purified by prep-TLC (PE:EtOAc = 1:1) to obtain **64-9** (103 mg, 78.6%). MS m/z: 330.35 [M+H]⁺.

Step7: Similar protocol as that for **T16,** using **64-9.** MS m/z: 230.35 [M+H]⁺.

Step8: Similar protocol as that for **T56,** using **64-10.** MS m/z: 623.55 [M+H]⁺.

Step9: Similar protocol as that for **T56,** using **64-11.** MS m/z: 503 [M+H]⁺. ¹H NMR (400 MHz, DMSO-*d*₆) δ 9.15 (s, 1H), 8.80 (d, *J* = 7.8 Hz, 1H), 8.27 (s, 1H), 7.89 (d, *J =* 7.8 Hz, 1H), 7.83 (s, 1H), 7.61 - 7.57 (m ,1H), 7.42 (d, *J =* 7.8 Hz, 1H), 7.17 (s, 1H), 5.96 (s, 1H), 4.43 (s, 2H), 4.26 - 4.20 (m ,1H), 3.16 (s, 3H), 3.10 (s, 3H), 2.88 (d, *J =* 4.8 Hz, 3H), 1.98 - 1.94 (m, 2H), 1.46 - 1.42 (m, 1H), 0.83 - 0.80 (m, 1H).

### Example T65

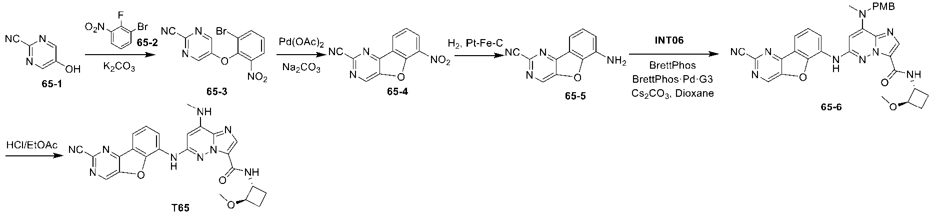

Step1: Similar protocol as that for **T42,** using **65-1.** HRMS m/z: 320.9599, 322.9576 [M+H]⁺.

Step2: Similar protocol as that for **T38,** using **65-3.** MS m/z: 241.25 [M+H]⁺.

Step3: Similar protocol as that for **T47,** using **65-4.** MS m/z: 211.25 [M+H]⁺.

Step4: Similar protocol as that for **T56,** using **65-5.** MS m/z: 604.50 [M+H]⁺.

Step5: Similar protocol as that for **T56,** using **65-6.** MS m/z: 484 [M+H]⁺. ¹H NMR (400 MHz, DMSO-*d*₆) δ 9.55 (s, 1H), 9.54 (s, 1H), 8.58 (d, *J =* 7.8 Hz, 1H), 8.15 (t, *J =* 6.5 Hz, 2H), 7.81 (s, 1H), 7.72 - 7.68 (m, 1H), 7.64 - 7.60 (m, 1H), 6.02 (s, 1H), 4.08 - 4.00 (m, 1H), 2.91 (s, 3H), 2.90 (d, *J =* 4.8 Hz, 3H), 2.88 - 2.83 (m, 1H), 1.78 - 1.74 (m, 2H), 0.52- 0.50 (m, 1H).

### Example T66

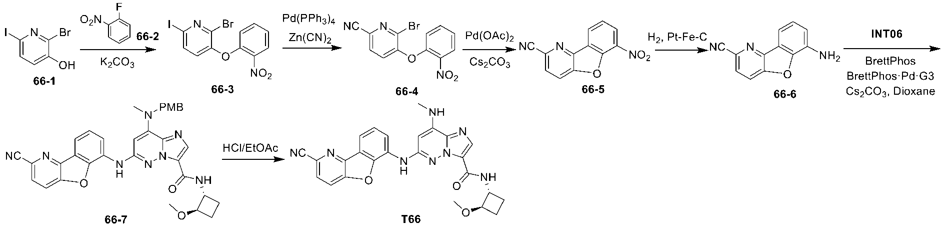

Step1: Similar protocol as that for **T42,** using **66-1.** MS m/z: 421[M+H]⁺.

Step2: Dissolve **66-3** (230 mg, 0.55 mmol) in DMF (3 mL), add zinc cyanide (89 mg, 0.76 mmol) under nitrogen protection, replace the atmosphere with nitrogen three times, add Pd(PPh₃)₄ (95 mg, 0.1 mmol), and heat the mixture to 75 °C with stirring for 3 hours. After the reaction is complete, add 10 mL of H₂O to quench the reaction. Extract the mixture with EtOAc (30 mL × 3), combine the organic phases, wash with saturated saline solution, and dry over anhydrous Na₂SO₄. Filter and concentrate the organic phase under reduced pressure. Purify the crude product by prep-TLC (PE:EtOAc = 2:1) to obtain **66-4** (130 mg, yield 74%). MS m/z: 320.20 [M+H]⁺.

Step3: Similar protocol as that for **T53,** using **66-4.** MS m/z: 240.05 [M+H]⁺.

Step4: Similar protocol as that for **T47,** using **66-5.** MS m/z: 210.15 [M+H]⁺.

Step5: Similar protocol as that for **T56,** using **66-6.** MS m/z: 603.55 [M+H]⁺.

Step6: Similar protocol as that for **T56,** using **66-7.** MS m/z: 483 [M+H]⁺.

### Example T67

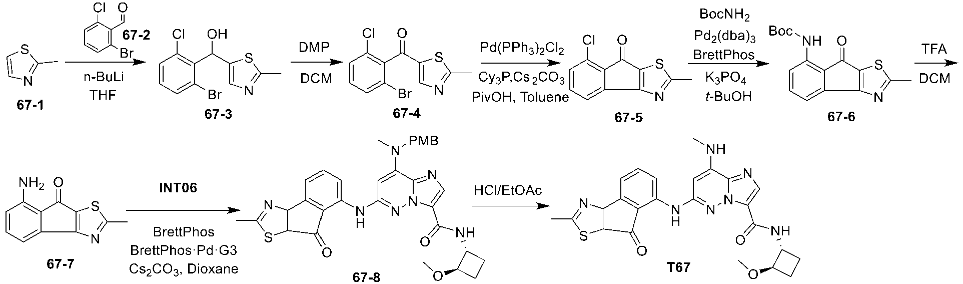

Step1: Similar protocol as that for **T50,** using **67-1.** MS m/z: 318.15 [M+H]⁺.

Step2: Similar protocol as that for **T50,** using **67-3.** MS m/z: 316 [M+H]⁺.

Step3: Dissolve **67-4** (150 mg, 0.47 mmol) in toluene (3 mL) in a reaction flask, add Cs₂CO₃ (232 mg, 0.71 mmol), pivalic acid (14 mg, 0.14 mmol), and tricyclohexylphosphine (14 mg, 0.05 mmol) at room temperature, replace the atmosphere with nitrogen three times, add Pd(PPh₃)₂Cl₂ (35 mg, 0.05 mmol), replace the atmosphere with nitrogen three times, heat the mixture to 80 °C and stir for 3 hours. After monitoring the complete consumption of the raw materials by TLC, add water to the reaction mixture and extract three times with EtOAc. Combine all the organic phases obtained, wash with saturated saline solution, dry over anhydrous Na₂SO₄, filter through diatomaceous earth, and concentrate the organic phases under reduced pressure. Purify the crude product by prep-TLC (PE:EtOAc = 1:1) to obtain **67-5** (57 mg, yield 52%). MS m/z: 236.30 [M+H]⁺.

Step4: Similar protocol as that for **T16,** using **67-5.** MS m/z: 317 [M+H]⁺.

Step5: Similar protocol as that for **T16,** using **67-6.** MS m/z: 217.30 [M+H]⁺.

Step6: Similar protocol as that for **T56,** using **67-7.** MS m/z: 612 [M+H]⁺.

Step7: Similar protocol as that for **T56,** using **67-8.** MS m/z: 492 [M+H]⁺.

### Example T68

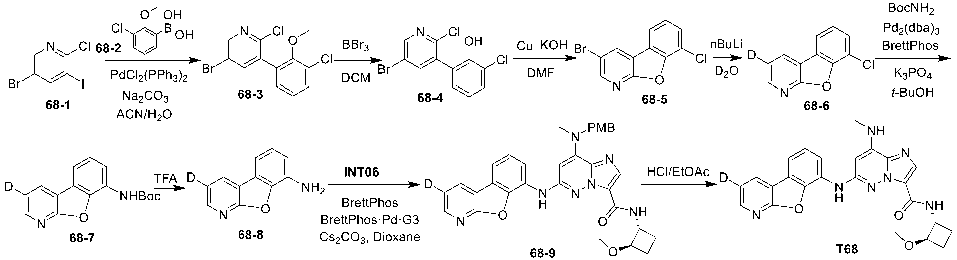

Step1: Similar protocol as that for **T52,** using **68-1.** MS m/z: 334.15 [M+H]⁺.

Step2: Similar protocol as that for **T22,** using **68-3.** MS m/z: 318 [M+H]⁺.

Step3: Similar protocol as that for **T26,** using **68-4.** MS m/z: 282 [M+H]⁺.

Step4: Add dry anhydrous THF to a Schlenk tube, cool it down to 0 °C, add isopropyl magnesium chloride (0.07 mL, 0.14 mmol), stir for 2 minutes, and then add n-butyllithium (0.14 mL, 0.2 mmol). Stir for 15 minutes, and then add **68-5** (75 mg, 0.27 mmol). Allow the reaction system to naturally warm up to room temperature and stir for 1 hour. Add D₂O (0.5 mL) to quench the reaction and stir for 15 minutes. Subsequently, add a saturated aqueous solution of ammonium chloride to the reaction system, and extract the mixture with EtOAc (30 mL × 3). Combine all organic phases, wash with saturated saline solution, and dry over anhydrous Na₂SO₄. Filter the obtained organic phase under reduced pressure, concentrate in vacuo, and purify the crude product by prep-TLC (PE:EtOAc = 3:1) to obtain **68-6** (45 mg, yield 81%). MS m/z: 205.30 [M+H]⁺.

Step5: Similar protocol as that for **T16,** using **68-6.** MS m/z: 286.35 [M+H]⁺.

Step6: Similar protocol as that for **T16,** using **68-7.** MS m/z: 186.35 [M+H]⁺.

Step7: Similar protocol as that for **T56,** using **68-8.** MS m/z: 579 [M+H]⁺.

Step8: Similar protocol as that for **T56,** using **68-9.** MS m/z: 459 [M+H]⁺.

**Biologic Example 1** Determination of Compound Cellular Proliferation Inhibitory Activity via CTG (CELLTITER-GLO) Luminescence Assay

Assay Principle: The Promega CellTiter-Glo^{™} luciferase kit is adopted for this assay. Luciferase is utilized as the detection reagent, and ATP is required to drive the luminescence reaction. Metabolically active cells generate ATP through respiration and other vital biological processes. An equal volume of CellTiter-Glo^{™} reagent is added to the cell culture medium, followed by luminescence intensity measurement. The luminescent signal is proportional to the ATP concentration in the system, and ATP level positively correlates with the number of viable cells. Accordingly, this method is applied to detect the inhibitory effect of test compounds on cell proliferation.

Experimental Procedures: Cell preparation Complete medium (RPMI 1640 + 10% FBS + 1% penicillin-streptomycin (P/S)) was prepared to revive Ba/F3-FL-TYK2-E957D cells (supplied by Precedo Pharmaceuticals Co.,Ltd). After approximately two passages, cells in logarithmic growth phase were harvested by centrifugation and counted, then resuspended to an appropriate concentration, the cell suspension was seeded into 96-well plates at 95 µL per well, with a seeding density of 2000 cells per well.

Test compounds were formulated into stock solutions with DMSO. Starting from a maximum concentration of 1 mM, serial 3-fold dilutions with DMSO were performed to generate 10 concentration gradients. The compound stock solutions were diluted 50-fold with culture medium, and 5 µL of the diluted compound solution was added to each well preloaded with 95 µL cell suspension.Minimum control wells (Min): Culture medium containing 0.1% DMSO without cells, Maximum control wells (Max): Mixture of DMSO and cell culture medium added to cells (final DMSO concentration: 0.1%). Cell incubation Plates were incubated for 72 h in a CO₂ incubator at 37 °C, 5% CO₂ and relative humidity above 90%. Luminescence detection 50 µL CellTiter-Glo reagent was added to each well to terminate the reaction. Plates were incubated at room temperature away from light for 30 min, gently shaken, and then measured on an Envision microplate reader to record the relative light unit (RLU) of each well.

The Inhibition Rate (Inh%) of the test compound was calculated in Excel based on the following formula: Inhibition Rate (Inh%) =[100-(RLUcpd-RLUMin)/(RLUMax-RLUMax)*100]%. Where RLUMax is the reading of the Control group on MD SpectraMax Paradigm, RLUcpd is the reading of the Treatment group on MD SpectraMax Paradigm, and RLUMin is the reading of the Blank group on MD SpectraMax Paradigm. Then, use GraphPad Prism (8) to generate the cell activity curve and calculate the relative maximum cell proliferation inhibition rate, the minimum cell proliferation inhibition rate, and IC50 value.

The data obtained by testing the representative molecules of the present invention using the test method described in Biological Example 1 are presented in Table 3 below.

**Table 3 Cell Proliferation Inhibition Test Data**

| Example | Ba/F3-TYK2-E957D IC50 nM | Example | Ba/F3-TYK2-E957D IC50 nM | Example | Ba/F3-TYK2-E957D IC50 nM |
|---|---|---|---|---|---|
| Deucravacitinib | 0.8 | **T23** | 0.18 | **T47** | 209.6 |
| Zasocitinib | 3.1 | **T24** | 1 | **T48** | 5.18 |
| **T01** | 330.9 | **T25** | 0.32 | **T49** | 0.3 |
| **T02** | 8.1 | **T26** | 0.32 | **T50** | 0.07 |
| **T03** | 3.5 | **T27** | 0.09 | **T51** | 4.07 |
| **T04** | 134.6 | **T28** | 2.1 | **T52** | 0.06 |
| **T05** | 15.3 | **T29** | 0.3 | **T53** | 0.05 |
| **T06** | 79 | **T30** | 0.07 | **T54** | 0.11 |
| **T07** | 106.8 | **T31** | 0.06 | **T55** | 0.39 |
| **T08** | 6.8 | **T32** | 0.45 | **T56** | 0.07 |
| **T09** | 50.1 | **T33** | 0.09 | **T57** | 0.12 |
| **T10** | 2.4 | **T35** | 0.22 | **T58** | 0.53 |
| **T11** | 81.8 | **T36** | 0.18 | **T59** | 0.22 |
| **T12** | 324.7 | **T37** | 0.56 | **T60** | 0.09 |
| **T13** | 31.8 | **T38** | 0.2 | **T61** | 0.27 |
| **T14** | 106.2 | **T39** | 0.4 | **T62** | 0.28 |
| **T16** | 65.9 | **T40** | 0.39 | **T63** | 1.52 |
| **T17** | 4 | **T41** | 0.24 | **T64** | 0.17 |
| **T18** | 11.7 | **T42** | 6.21 | **T65** | 0.24 |
| **T19** | 1.1 | **T43** | 87.19 | **T66** | 0.26 |
| **T20** | 106.7 | **T44** | 38.14 | **T67** | 0.12 |
| **T21** | 10.9 | **T45** | 74.79 | | |
| **T22** | 0.26 | **T46** | 20.65 | | |

### Biological Example2: TYK2 JH2 in vitro enzyme binding experiment

In this experiment, the inhibitory effect of compounds on TYK2 JH2 pseudokinase was tested using the method of Time-Resolved Fluorescence Resonance Energy Transfer (TR-FRET). In this experiment, TYK2 JH2 pseudokinase can bind simultaneously to a fluorescently labeled Tracer and a Tb antibody. The Tb antibody, as a fluorescent donor, produces fluorescence at a wavelength of 495 nm under excitation at a certain wavelength. The Tracer, as a fluorescent acceptor, can only receive the 495 nm fluorescence when it is sufficiently close to the Tb antibody, thereby generating fluorescence at a wavelength of 520 nm, which is the TR-FRET signal. When a compound is added to compete with the Tracer for binding to the pseudokinase JH2 region, the TR-FRET signal decreases due to reduced Tracer binding. The ratio of 520 nm/495 nm signals can reflect the inhibitory activity of the compound on the pseudokinase binding.

B2.1 The experimental procedure is as follows:
B2.1.1 Dissolve it in DMSO to a storage concentration of 10 mM.

B2.1.2 Prepare different concentration gradients in dilution plates that are 200 times the final concentration, and transfer them to 384-well plates.

B2.1.3 Use the Echo instrument to transfer 75nL from the 384-well plate to the 384 experiment plate. The positive control group and negative control group are replaced with the same volume of DMSO.

B2.1.4 Add 5 uL of TYK2 JH2 pseudokinase at a concentration three times the final concentration (with a final concentration of 0.5 nM) to a 384-well plate, respectively. The negative control group is replaced with the same volume of 1x experimental working solution, and then centrifuge at 1000 rpm for 30 seconds.

B2.1.5 Add 5 uL of Tb antibody at a concentration three times the final concentration (1x represents the final concentration) to each well of a 384-well plate, and centrifuge at 1000 rpm for 30 seconds.

B2.1.6 Add 5 uL of Tracer, which is 3 times the final concentration (with 1 nM being the final concentration), to each well of a 384-well plate, and centrifuge at 1000 rpm for 30 seconds.

B2.1.7 Incubate at room temperature for 60 minutes, and incubate overnight at 4 degrees Celsius.

B2.1.8 Read the ratio of 520 nm/495 nm fluorescence signals using the Envision microplate reader (PerkinElmer).

B2.2 Data Analysis: The software XLfit, developed by IDBS and integrated into the Microsoft Excel environment, was used for test data processing and analysis. First, the average response signals of the positive control group and the negative control group were calculated. Then, the response inhibition rate of each well was determined based on the formula: "Single-well inhibition rate % = ((Average of positive control group - Single-well signal value) / (Average of positive control group - Average of negative control group)) × 100". Subsequently, the concentration and response data were imported into XLfit software. Utilizing the Dose Response One Site 205 model, a four-parameter inhibition model was employed, and a four-parameter inhibition rate-concentration curve was fitted to calculate the IC50 value of the compound.

The data obtained by testing the representative molecules of the present invention using the test method described in Biological Example 2 are presented in Table 4 below. In Table 4, "A" indicates an IC₅₀ value less than 10 nM; "B" indicates an IC₅₀ value greater than or equal to 10 nM and less than 100 nM; and "C" indicates an IC₅₀ value greater than or equal to 100 nM and less than 1000 nM.

**Table 4 TYK2 JH2 Enzymology Test Data**

| Example | TYK2 JH2 IC₅₀ nM | Example | TYK2 JH2 IC₅₀ nM |
|---|---|---|---|
| Deucravacitinib | A | **T33** | A |
| Zasocitinib | A | **T35** | A |
| **T22** | A | **T36** | A |
| **T23** | A | **T38** | A |
| **T27** | A | **T41** | A |
| **T30** | A | **T44** | A |
| **T31** | A | | |

Although specific embodiments of the present invention have been described above, those skilled in the art should understand that these are merely illustrative examples. Various alterations or modifications can be made to these embodiments without departing from the principles and essence of the invention. Therefore, the scope of protection of the present invention is defined by the appended claims.

## Claims

1. A compound represented by Formula (I), a pharmaceutically acceptable salt thereof, a solvate thereof, or a solvate of the pharmaceutically acceptable salt thereof: 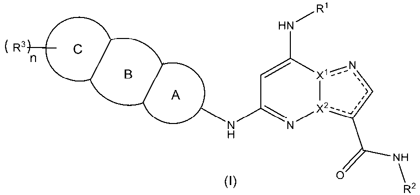 wherein, 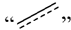 the bond represents a single bond or a double bond;
X¹ is C or N;
X² is C or N;
R¹ is H, C₁-C₆ alkyl, or C₁-C₆ alkyl substituted with one or more deuterium atoms;
R² is C₁-C₆ alkyl, C₁-C₆ alkoxy, C₃-C₁₂ cycloalkyl, 3-12 membered heterocycloalkyl, C₁-C₆ alkyl substituted with one or more R²⁻¹, C₁-C₆ alkoxy substituted with one or more R²⁻², C₃-C₁₂ cycloalkyl substituted with one or more R²⁻³, or 3-12 membered heterocycloalkyl substituted with one or more R²⁻⁴;
R²⁻¹ and R²⁻² are each independently halogen, OH, C₃-C₁₂ cycloalkyl, or 3-12 membered heterocycloalkyl; Or, two adjacent R²⁻¹ groups, together with the carbon atom to which they are attached, together form a C₃-C₇ monocyclic cycloalkyl, a C₅-C₁₂ fused cycloalkyl, a C₅-C₁₂ bridged cycloalkyl, a C₅-C₁₂ spiro cycloalkyl, a 3-7 membered monocyclic heterocycloalkyl, a 5-12 membered fused heterocycloalkyl, a 5-12 membered bridged heterocycloalkyl, or a 5-12 membered spiro heterocycloalkyl;
Or, two R²⁻¹ groups attached to the same carbon atom, together with the carbon atom to which they are attached, together form a C₃-C₇ monocyclic cycloalkyl, a C₅-C₁₂ fused cycloalkyl, a C₅-C₁₂ bridged cycloalkyl, a C₅-C₁₂ spiro cycloalkyl, a 3-7 membered monocyclic heterocycloalkyl, a 5-12 membered fused heterocycloalkyl, a 5-12 membered bridged heterocycloalkyl, or a 5-12 membered spiro heterocycloalkyl;
Or, two adjacent R²⁻² groups, together with the carbon atom to which they are attached, together form a C₃-C₇ monocyclic cycloalkyl, a C₅-C₁₂ fused cycloalkyl, a C₅-C₁₂ bridged cycloalkyl, a C₅-C₁₂ spiro cycloalkyl, a 3-7 membered monocyclic heterocycloalkyl, a 5-12 membered fused heterocycloalkyl, a 5-12 membered bridged heterocycloalkyl, or a 5-12 membered spiro heterocycloalkyl;
Or, two R²⁻² groups attached to the same carbon atom, together with the carbon atom to which they are attached, together form a C₃-C₇ monocyclic cycloalkyl, a C₅-C₁₂ fused cycloalkyl, a C₅-C₁₂ bridged cycloalkyl, a C₅-C₁₂ spiro cycloalkyl, a 3-7 membered monocyclic heterocycloalkyl, a 5-12 membered fused heterocycloalkyl, a 5-12 membered bridged heterocycloalkyl, or a 5-12 membered spiro heterocycloalkyl; R²⁻³ and R²⁻⁴ are each independently halogen, OH, C₁-C₆ alkyl, or C₁-C₆ alkoxy;
Or, two adjacent R²⁻³ groups, together with the carbon atom to which they are attached, together form a C₃-C₇ cycloalkyl or a 3-7 membered heterocycloalkyl;
Or, two R²⁻³ groups attached to the same carbon atom, together with the carbon atom to which they are attached, together form a C₃-C₇ cycloalkyl or a 3-7 membered heterocycloalkyl;
Or, two adjacent R²⁻⁴ groups, together with the carbon atom to which they are attached, together form a C₃-C₇ cycloalkyl or a 3-7 membered heterocycloalkyl;
Or, two R²⁻⁵ groups attached to the same carbon atom, together with the carbon atom to which they are attached, together form a C₃-C₇ cycloalkyl or a 3-7 membered heterocycloalkyl.
Ring A, Ring B and Ring C are each independently a benzene ring, a 5- to 6-membered heteroaryl, a C₅-C₆ cycloalkene or a 5- to 6-membered heterocycloalkene;
Ring A and Ring B share a bond, and said bond is a single bond or a double bond;
Ring B and Ring C share a bond, and said bond is a single bond or a double bond;
R³ is a substituent on Ring A, Ring B or Ring C;
Each R³ is independently halogen, CN, C₁-C₆ alkyl, C₁-C₆ alkoxy, C₃-C₁₂ cycloalkyl, 3-12 membered heterocycloalkyl, C₁-C₆ alkyl substituted with one or more R³⁻¹, or C₁-C₆ alkoxy substituted with one or more R³⁻²;
R³⁻¹ and R³⁻² are each independently halogen;
n is 0, 1, 2 or 3;
In the "3-12 membered heterocycloalkyl", "3-12 membered heterocycloalkyl substituted with one or more R²⁻⁴", "5-6 membered heterocycloalkene", "3-8 membered heterocycloalkyl", "3-7 membered monocyclic heterocycloalkyl", "5-12 membered fused heterocycloalkyl", "5-12 membered bridged heterocycloalkyl", "5-12 membered spiro heterocycloalkyl" and "3-7 membered heterocycloalkyl", the types of heteroatoms or heteroatomic groups are each independently selected from one or more of N, O, S, C(=O), S(=O) and S(=O)₂, and the number of heteroatoms or heteroatomic groups is each independently one or more;
In the "5-6 membered heteroaryl", the types of heteroatoms are each independently selected from one or more of N, O and S, and the number of heteroatoms is each independently one or more.

2. The compound of claim 1 represented by Formula (I), or a pharmaceutically acceptable salt, solvate, or solvate of said pharmaceutically acceptable salt thereof, **characterized in that** it satisfies one or more of the following conditions:
(1) In the "3-12 membered heterocycloalkyl", "3-12 membered heterocycloalkyl substituted with one or more R²⁻⁴", "5-6 membered heterocycloalkene", "3-8 membered heterocycloalkyl", "3-7 membered monocyclic heterocycloalkyl", "5-12 membered fused heterocycloalkyl", "5-12 membered bridged heterocycloalkyl", "5-12 membered spiro heterocycloalkyl" and "3-7 membered heterocycloalkyl", the types of heteroatoms or heteroatomic groups are each independently selected from 1, 2 or 3 of N, O, S, C(=O), S(=O) and S(=O)₂, and the number of heteroatoms or heteroatomic groups is each independently 1, 2 or 3;
(2) In the "5-6 membered heteroaryl", the types of heteroatoms are each independently selected from 1, 2 or 3 of N, O and S, and the number of heteroatoms is each independently 1, 2 or 3.

3. The compound of claim 1 or claim 2 represented by Formula (I), or a pharmaceutically acceptable salt, solvate, or solvate of said pharmaceutically acceptable salt thereof, **characterized in that** it satisfies one or more of the following conditions:
(1) Each "C₁-C₆ alkyl" is independently methyl, ethyl, n-propyl, isopropyl, n-butyl, isobutyl, sec-butyl, tert-butyl, n-pentyl, -CH₂CH₂CH₂CH₂CH₃, -CH(CH₃)CH₂CH₂CH₃, -CH₂CH(CH₃)CH₂CH₃, - CH₂CH₂CH(CH₃)₂, -CH(C₂H₅)CH₂CH₃, -C(CH₃)₂CH₂CH₃, -CH(CH₃)CH(CH₃)₂ or -CH₂C(CH₃)₃, for example, methyl, isopropyl, isobutyl or -CH₂C(CH₃)₃;
(2) Each "C₁-C₆ alkoxy" is independently methoxy, ethoxy, n-propoxy, isopropoxy, n-butoxy, isobutoxy, sec-butoxy or tert-butoxy, for example, methoxy;
(3) Each "C₃-C₁₂ cycloalkyl" is independently a C₃-C₆ cycloalkyl, for example, cyclopropyl, cyclobutyl, cyclopentyl, cyclohexyl, spiro[2.2]pentyl or spiro[2.3]hexyl, more particularly 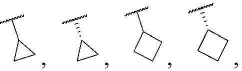 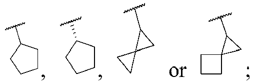
(4) Each "3-12 membered heterocycloalkyl" is independently a 3-6 membered heterocycloalkyl in which the heteroatoms are selected from one or more of N, O and S, and the number of heteroatoms is 1 or 2, for example, aziridinyl, oxiranyl, azetidinyl, oxetanyl, tetrahydrofuranyl, pyrrolidinyl, morpholinyl or piperidinyl, further for example 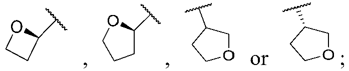
(5) Each "halogen" is independently F, Cl, Br or I, for example, F;
(6) Each "5-6 membered heteroaryl" is a 5-6 membered heteroaryl in which the heteroatoms are selected from one or more of N, O and S, and the number of heteroatoms is 1 or 2, for example, furan ring, thiophene ring, pyrrole ring, pyrazole ring, imidazole ring, pyridine ring, pyrazine ring, pyridazine ring or pyrimidine ring;
(7) Each "5-6 membered heterocycloalkene" is 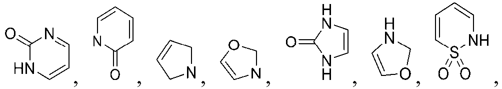 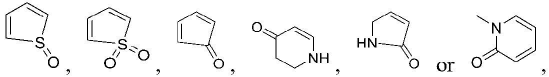 for example

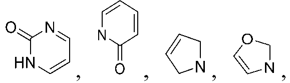

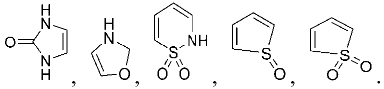

4. The compound of claim 1 represented by Formula (1), or a pharmaceutically acceptable salt, solvate, or solvate of said pharmaceutically acceptable salt thereof, **characterized in that** it satisfies one or more of the following conditions:
(1) 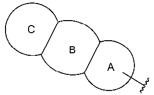 is 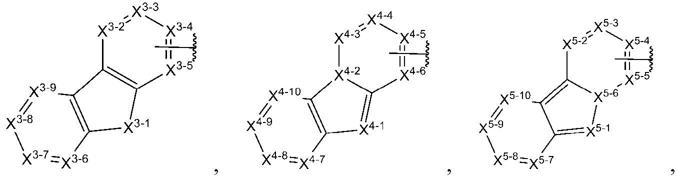 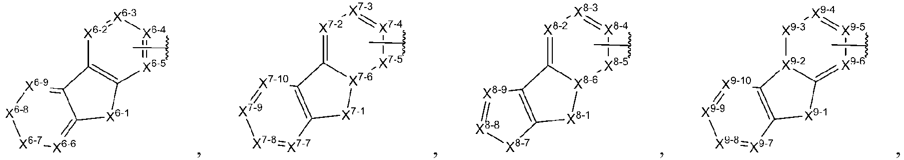 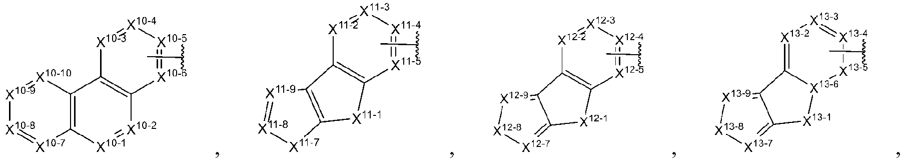 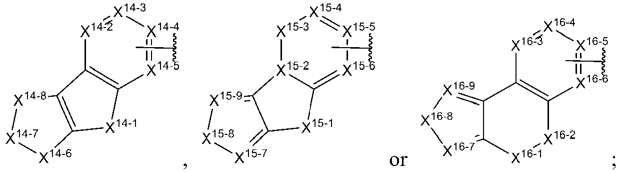 wherein,
X³⁻¹ is CH₂, NH, O, S, C(=O), S(=O) or S(=O)₂; X³⁻² , X³⁻³, X³⁻⁴, X³⁻⁵, X³⁻⁶, X³⁻⁷, X³⁻⁸ and X³⁻⁹ are each independently CH or N;
X⁴⁻¹, X⁴⁻², X⁴⁻³, X⁴⁻⁴, X⁴⁻⁵, X⁴⁻⁶, X⁴⁻⁷, X⁴⁻⁸, X⁴⁻⁹ and X⁴⁻¹⁰ are each independently CH or N;
X⁵⁻¹, X⁵⁻², X⁵⁻³, X⁵⁻⁴, X⁵⁻⁵, X⁵⁻⁶, X⁵⁻⁷, X⁵⁻⁸, X⁵⁻⁹ and X⁵⁻¹⁰ are each independently CH or N;
X⁶⁻¹, X⁶⁻⁷ and X⁶⁻⁸ are each independently CH₂, NH, O, S, C(=O), S(=O) or S(=O)₂, X⁶⁻², X⁶⁻³, X⁶⁻⁴, X⁶⁻⁵, X⁶⁻⁶ and X⁶⁻⁹ are each independently CH or N;
X⁷⁻¹ and X⁷⁻⁵ are each independently CH₂, NH, O, S, C(=O), S(=O) or S(=O)₂, X⁷⁻², X⁷⁻³, X⁷⁻⁴, X⁷⁻⁶, X⁷⁻⁷, X⁷⁻⁸, X⁷⁻⁹ and X⁷⁻¹⁰ are each independently CH or N;
X⁸⁻¹, X⁸⁻⁵ and X⁸⁻⁷ are each independently CH₂, NH, O, S, C(=O), S(=O) or S(=O)₂, X⁸⁻², X⁸⁻³, X⁸⁻⁴, X⁸⁻⁶, X⁸⁻⁸ and X⁸⁻⁹ are each independently CH or N;
X⁹⁻¹ and X⁹⁻³ are each independently CH₂, NH, O, S, C(=O), S(=O) or S(=O)₂, X⁹⁻², X⁹⁻⁴, X⁹⁻⁵, X⁹⁻⁶, X⁹⁻⁷, X⁹⁻⁸, X⁹⁻⁹ and X⁹⁻¹⁰ are each independently CH or N;
X¹⁰⁻¹, X¹⁰⁻² X¹⁰⁻³, X¹⁰⁻⁴, X¹⁰⁻⁵, X¹⁰⁻⁶, X¹⁰⁻⁷, X¹⁰⁻⁸, X¹⁰⁻⁹ and X¹⁰⁻¹⁰ are each independently CH or N; X¹¹⁻¹ and X¹¹⁻⁷ are each independently CH₂, NH, O, S, C(=O), S(=O) or S(=O)₂, X¹¹⁻², X¹¹⁻³, X¹¹⁻⁴, X¹¹⁻⁵, X¹¹⁻⁸ and X¹¹⁻⁹ are each independently CH or N;
X¹²⁻¹ and X¹²⁻⁸ are each independently CH₂, NH, O, S, C(=O), S(=O) or S(=O)₂, X¹²⁻², X¹²⁻³, X¹²⁻⁴, X¹²⁻⁵, X¹²⁻⁷ and X¹²⁻⁹ are each independently CH or N;
X¹³⁻¹, X¹³⁻⁵ and X¹³⁻⁸ are each independently CH₂, NH, O, S, C(=O), S(=O) or S(=O)₂, X¹³⁻², X¹³⁻³, X¹³⁻⁴, X¹³⁻⁶, X¹³⁻⁷ and X¹³⁻⁹ are each independently CH or N;
X¹⁴⁻¹, X¹⁴⁻⁶, X¹⁴⁻⁷ and X¹⁴⁻⁸ are each independently CH₂, N^{H}, O, S, C(=O), S(=O) or S(=O)₂, X¹⁴⁻², X¹⁴⁻³, X¹⁴⁻⁴ and X¹⁴⁻⁵ are each independently CH or N;
X¹⁵⁻¹, X¹⁵⁻³ and X¹⁵⁻⁸ are each independently CH₂, NH, O, S, C(=O), S(=O) or S(=O)₂, X¹⁵⁻², X¹⁵⁻⁴, X¹⁵⁻⁵ X¹⁵⁻⁶, X¹⁵⁻⁷ and X¹⁵⁻⁹ are each independently CH or N;
X¹⁶⁻¹, X¹⁶⁻² and X¹⁶⁻⁸ are each independently CH₂, NH, O, S, C(=O), S(=O) or S(=O)₂, X¹⁶⁻³, X¹⁶⁻⁴, X¹⁶⁻⁵, X¹⁶⁻⁶, X¹⁶⁻⁷ and X¹⁶⁻⁹ are each independently CH or N.
(2) Each R³ is independently F, CN, C₁-C₃ alkyl, C₁-C₃ alkoxy, C₃-C₆ cycloalkyl, or C₁-C₃ alkyl substituted with one or more F atoms;
(3) n is 0, 1 or 2;
(4) R¹ is C₁-C₃ alkyl;
(5) R² is C₁-C₅ alkyl, C₃-C₆ cycloalkyl, 3-6 membered heterocycloalkyl, C₁-C₅ alkyl substituted with one or more R²⁻¹, C₃-C₆ cycloalkyl substituted with one or more R²⁻³, or 3-6 membered heterocycloalkyl substituted with one or more R²-⁴;
R²⁻¹ is independently OH or 3-6 membered heterocycloalkyl;
R²⁻³ is independently F, OH or OCH₃;
R²⁻⁴ is independently OH or OCH₃.

5. The compound of claim 1 represented by Formula (I), or a pharmaceutically acceptable salt, solvate, or solvate of said pharmaceutically acceptable salt thereof, **characterized in that** it satisfies one or more of the following conditions:
(1) 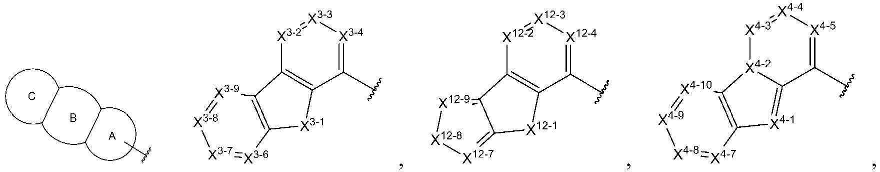 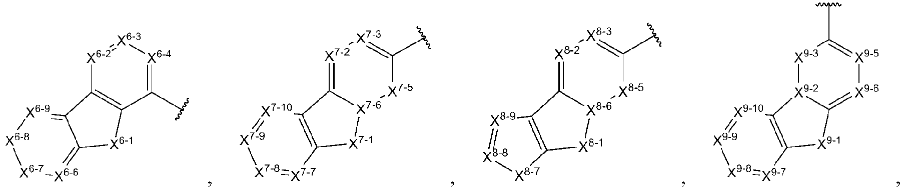 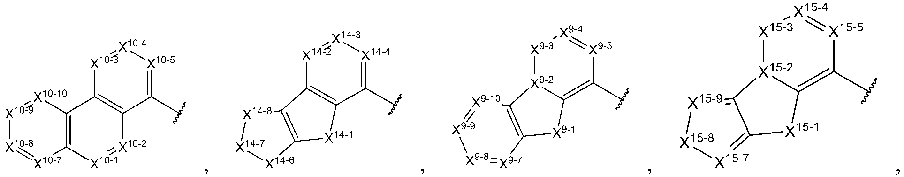 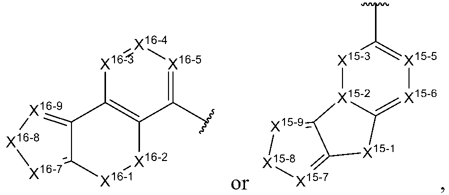 wherein,
X³⁻¹ is CH₂, NH, O, S, C(=O), S(=O) or S(=O)₂, X³⁻², X³⁻³, X³⁻⁴, X³⁻⁶, X³⁻⁷, X³⁻⁸ and X³⁻⁹ are each independently CH or N;
X⁴⁻¹, X⁴⁻², X⁴⁻³, X⁴⁻⁴, X⁴⁻⁵, X⁴⁻⁷, X⁴⁻⁸, X⁴⁻⁹ and X⁴⁻¹⁰ are each independently CH or N;
X⁶⁻¹, X⁶⁻⁷ and X⁶⁻⁸ are each independently CH₂, NH, O, S, C(=O), S(=O) or S(=O)₂, X⁶⁻², X⁶⁻³, X⁶⁻⁴, X⁶⁻⁶ and X⁶⁻⁹ are each independently CH or N;
X⁷⁻¹ and X⁷⁻⁵ are each independently CH₂, NH, O, S, C(=O), S(=O) or S(=O)₂, X⁷⁻², X⁷⁻³, X⁷⁻⁶, X⁷⁻⁷, X⁷⁻⁸, X⁷⁻⁹ and X⁷⁻¹⁰ are each independently CH or N;
X⁸⁻¹, X⁸⁻⁵ and X⁸⁻⁷ are each independently CH₂, NH, O, S, C(=O), S(=O) or S(=O)₂, X⁸⁻², X⁸⁻³, X⁸⁻⁶, X⁸⁻⁸ and X⁸⁻⁹ are each independently CH or N;
X⁹⁻¹ and X⁹⁻³ are each independently CH₂, NH, O, S, C(=O), S(=O) or S(=O)₂, X⁹⁻², X⁹⁻⁴, X⁹⁻⁵, X⁹⁻⁶, X⁹⁻⁷, X⁹⁻⁸, X⁹⁻⁹ and X⁹⁻¹⁰ are each independently CH or N;
X¹⁰⁻¹, X¹⁰⁻², X¹⁰⁻³, X¹⁰⁻⁴, X¹⁰⁻⁵, X¹⁰⁻⁷, X¹⁰⁻⁸, X¹⁰⁻⁹ and X¹⁰⁻¹⁰ are each independently CH or N;
X¹²⁻¹ and X¹²⁻⁸ are each independently CH₂, NH, O, S, C(=O), S(=O) or S(=O)₂, X¹²⁻², X¹²⁻³, X¹²⁻⁴, X¹²⁻⁷ and X¹²⁻⁹ are each independently CH or N;
X¹⁴⁻¹, X¹⁴⁻⁶, X¹⁴⁻⁷ and X¹⁴⁻⁸ are each independently CH₂, NH, O, S, C(=O), S(=O) or S(=O)₂, X¹⁴⁻², X¹⁴⁻³ and X¹⁴⁻⁴ are each independently CH or N;
X¹⁵⁻¹, X¹⁵⁻³ and X¹⁵⁻⁸ are each independently CH₂, NH, O, S, C(=O), S(=O) or S(=O)₂, X¹⁵⁻², X¹⁵⁻⁴, X¹⁵⁻⁵, X¹⁵⁻⁶, X¹⁵⁻⁷ and X¹⁵⁻⁹ are each independently CH or N;
X¹⁶⁻² and X¹⁶⁻⁸ are each independently CH₂, NH, O, S, C(=O), S(=O) or S(=O)₂, X¹⁶⁻³, X¹⁶⁻⁴, X¹⁶⁻⁵, X¹⁶⁻⁷ and X¹⁶⁻⁹ are each independently CH or N;
Better, 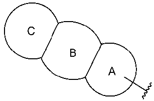
for any of the following cases:
Case 1: is 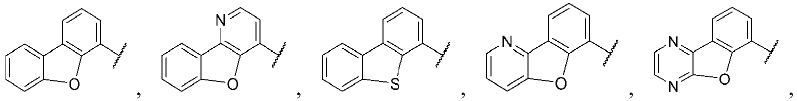 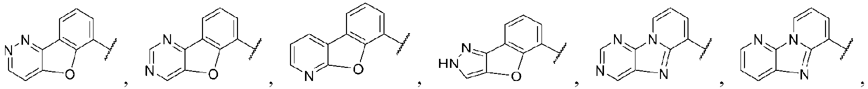 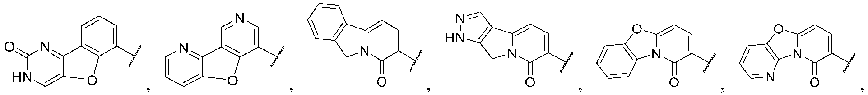 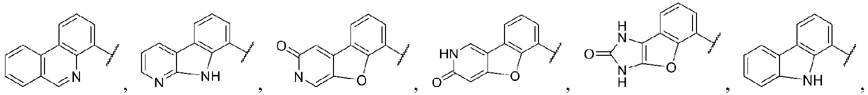 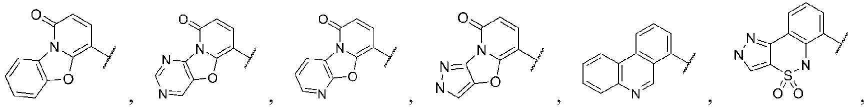 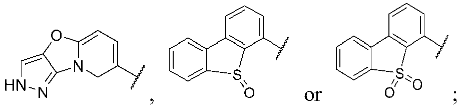
Case 2: is 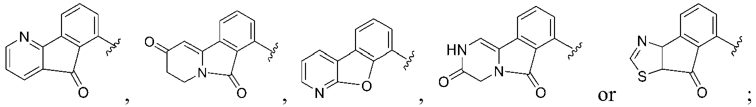
(2) R³ is F, CN, CH₃, CF₃, OCH₃ or 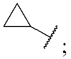
(3) R¹is a methyl group;
(4) R² is

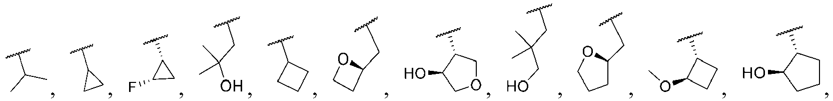

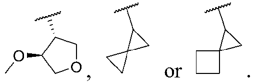

6. The compound of claim 1 represented by Formula (I), or a pharmaceutically acceptable salt, solvate, or solvate of said pharmaceutically acceptable salt thereof, **characterized in that** 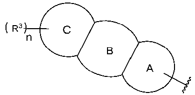
for any of the following cases:
Case 1: is 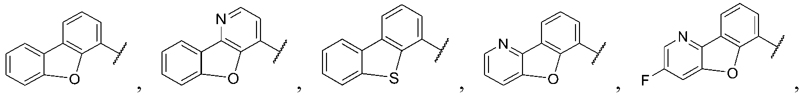 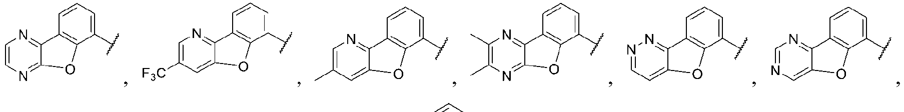 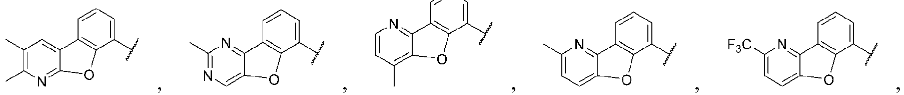 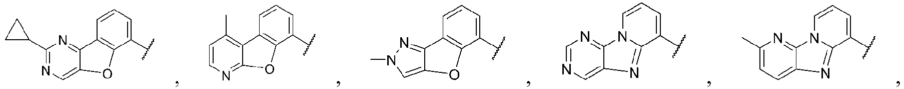 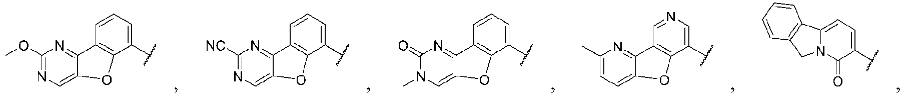 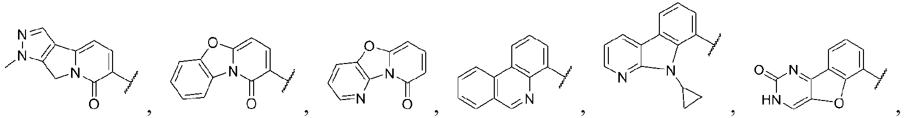 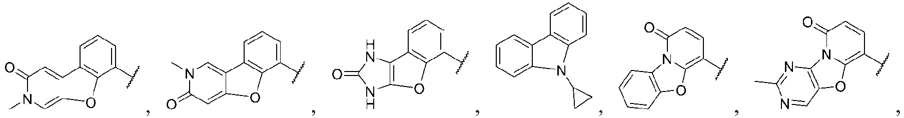 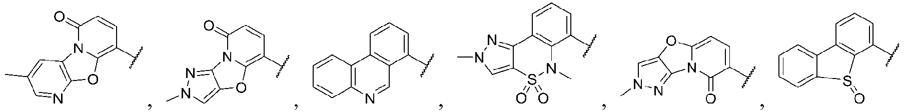
or 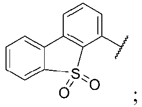
Case 2: is

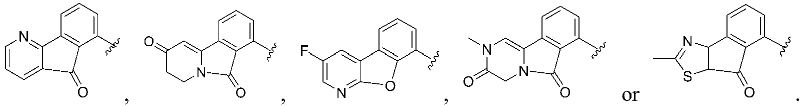

7. The compound of any one of claims 1 to 6 represented by Formula (I), or a pharmaceutically acceptable salt, solvate, or solvate of said pharmaceutically acceptable salt thereof, **characterized in that** the compound represented by Formula (I) is a compound represented by Formula (I-1), (I-2), (I-3) or (I-4): 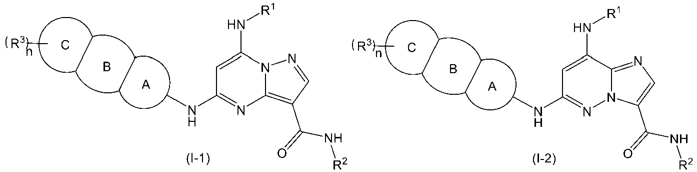 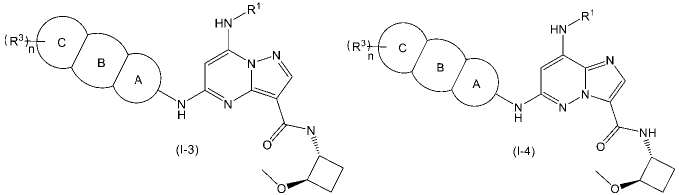 wherein, the definitions of R¹, R², R³, n, Ring A, Ring B and Ring C are the same as those defined in any one of claims 1 to 6.

8. The compound of any one of claims 1 to 6 represented by Formula (I), or a pharmaceutically acceptable salt, solvate, or solvate of said pharmaceutically acceptable salt thereof, **characterized in that** the compound represented by Formula (I) is any one of the following compounds:

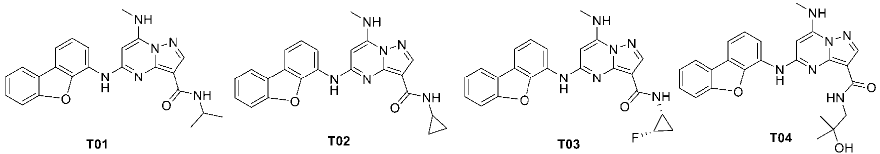

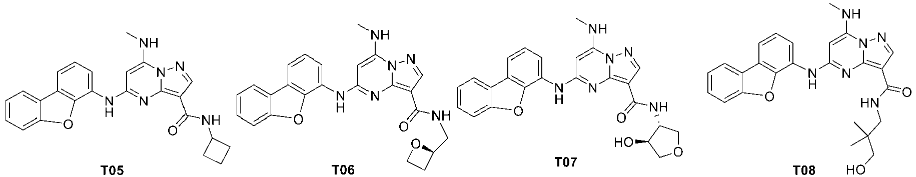

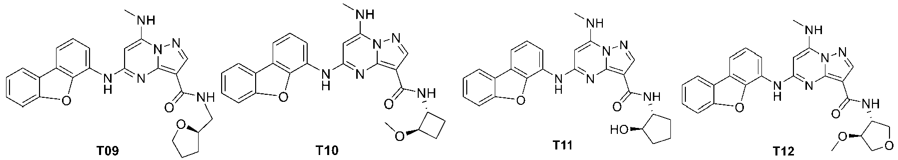

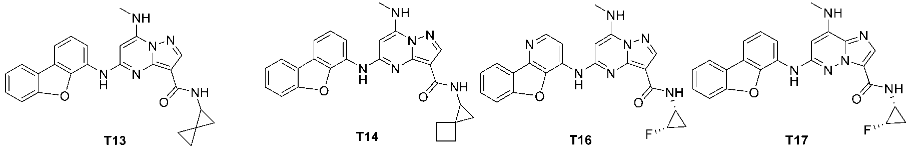

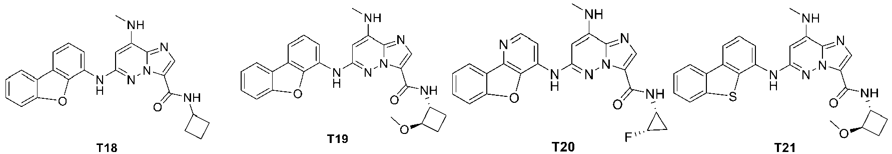

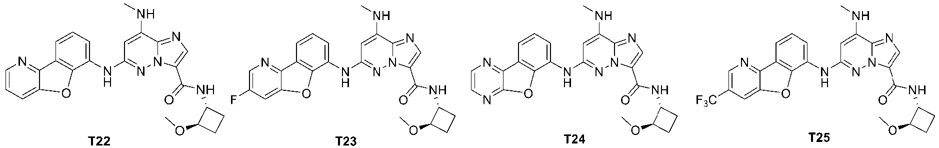

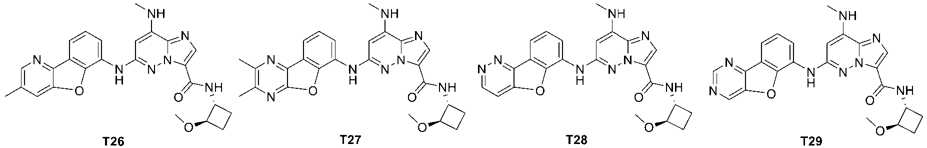

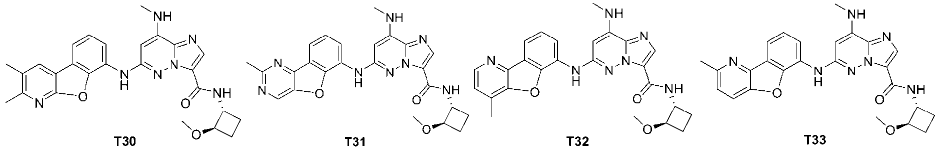

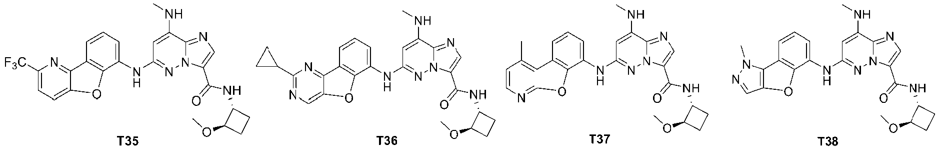

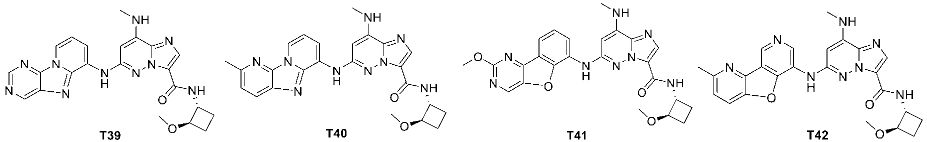

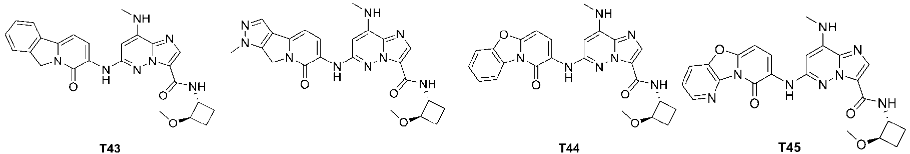

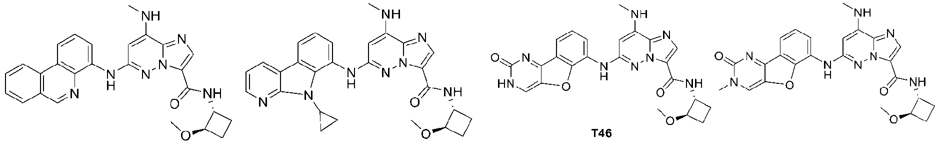

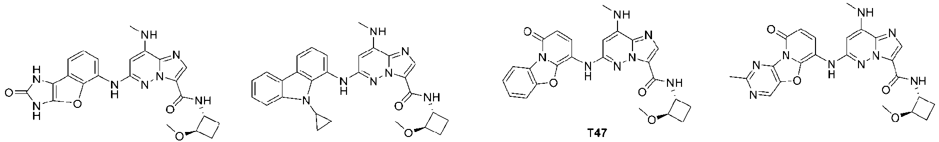

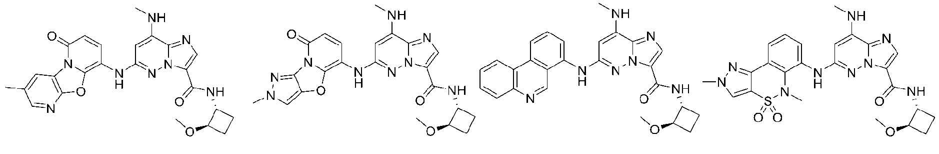

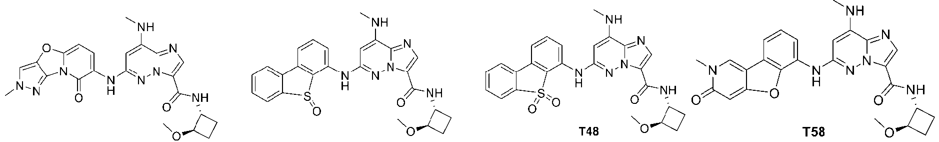

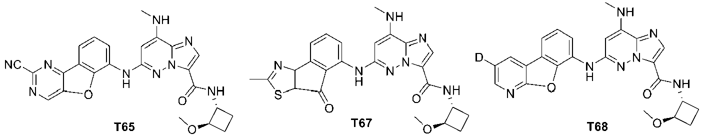

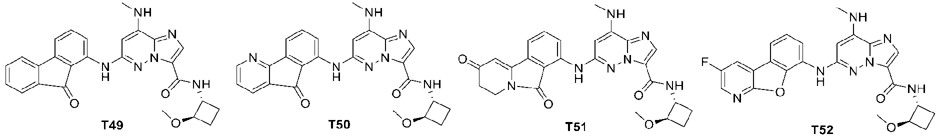

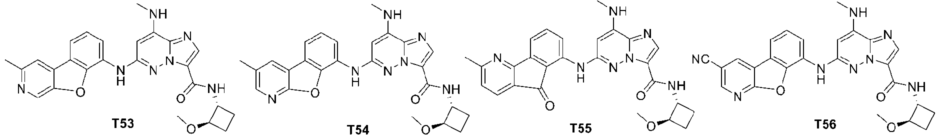

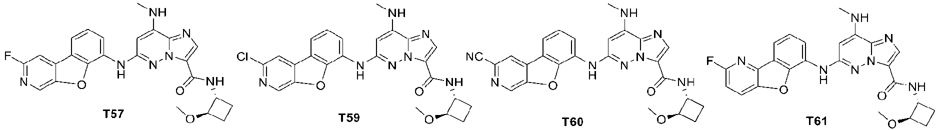

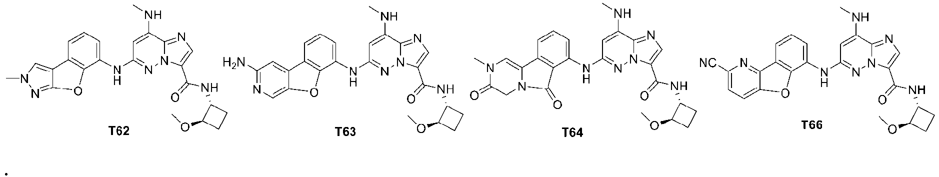

9. A pharmaceutical composition, comprising the compound of any one of claims 1 to 8 represented by Formula (I), or a pharmaceutically acceptable salt, solvate, or solvate of said pharmaceutically acceptable salt thereof, together with pharmaceutically acceptable excipients.

10. Use of the compound of any one of claims 1 to 8 represented by Formula (I), or a pharmaceutically acceptable salt, solvate, or solvate of said pharmaceutically acceptable salt thereof, or the pharmaceutical composition of claim 9, in the preparation of a TYK2 inhibitor; preferably, the TYK2 is TYK2-E957D.

11. Use of the compound of any one of claims 1 to 8 represented by Formula (I), or a pharmaceutically acceptable salt, solvate, or solvate of said pharmaceutically acceptable salt thereof, or the pharmaceutical composition of claim 9, in the manufacture of a medicament for preventing and/or treating TYK2-related diseases; preferably, the TYK2-related diseases are diseases associated with TYK2-E957D and/or TYK2-JH2; more preferably, the TYK2-related diseases are autoimmune diseases, for example, psoriasis, psoriatic arthritis, inflammatory bowel disease, lupus erythematosus or allergic dermatitis.

12. Use of the compound of any one of claims 1 to 8 represented by Formula (I), or a pharmaceutically acceptable salt, solvate, or solvate of said pharmaceutically acceptable salt thereof, or the pharmaceutical composition of claim 9, in the manufacture of a medicament for preventing and/or treating autoimmune diseases, for example, psoriasis, psoriatic arthritis, inflammatory bowel disease, lupus erythematosus or allergic dermatitis.
